# EUROPEAN PATENT APPLICATION

(11) **EP 4 497 758 A1**
(43) Date of publication of application: **29.01.2025**
(21) Application number: 24183812.7
(22) Date of filing: 21.06.2024
(51) Int. Cl.: C07K 16/42, A61K 39/00, A61P 37/00

(54) **NEW USES OF OMALIZUMAB**

(30) Priority: 28.07.2023 EP 23188544; 22.04.2024 EP 24171754
(71) Applicant: Novartis AG, 4056 Basel (CH); GENENTECH, INC., South San Francisco, CA 94080-4990 (US)
(72) Inventor: Chaikou, Maria, 4056 Basel (CH); Rigollet, Mathieu Paul-Léon, 4056 Basel (CH)
(74) Representative: Carpmaels & Ransford LLP

(57) **Abstract**

150mg/mL omalizumab formulation for use in a method of treatment of one or more of: allergic asthma; chronic rhinosinusitis with nasal polyps; chronic spontaneous urticaria; nasal polyps; food allergy; moderate to severe persistent asthma in patients with a positive skin test or *in vitro* reactivity to a perennial aeroallergen and symptoms inadequately controlled with inhaled corticosteroids, wherein the omalizumab is administered subcutaneously by a syringe.

## Description

This application claims the benefit of European patent application 23188544.3 filed July 28th 2023 and 24171754.5 filed April 22nd 2024, the complete contents of which are incorporated herein by reference for all purposes.

The present disclosure relates to methods of treating a patient with one or more of: allergic asthma; food allergy; chronic rhinosinusitis with nasal polyps; chronic spontaneous urticaria; nasal polyps; moderate to severe persistent asthma in patients with a positive skin test or in vitro reactivity to a perennial aeroallergen and symptoms that are inadequately controlled with inhaled corticosteroids, the method comprising administering 150mg/mL omalizumab formulation to the patient, wherein the omalizumab formulation is administered subcutaneously by a syringe. The present disclosure also relates to 150mg/mL omalizumab formulation for use according to the methods described herein. The present disclosure also relates to syringes comprising a reservoir filled with 150mg/mL omalizumab formulation and configured to perform the methods described herein. The present disclosure also relates to the use of 150mg/mL omalizumab formulation for the manufacture of a medicament for any of the methods of treatment disclosed herein.

### BACKGROUND OF THE INVENTION

Omalizumab is a recombinant DNA-derived humanized IgG1k monoclonal antibody that selectively binds to human immunoglobulin E (IgE) and is used to treat allergic asthma, chronic hives and nasal polyps, IgE-mediated food allergy, and chronic spontaneous urticaria.

When omalizumab first entered the market in 2003, it was available only as a dry lyophilized powder (XOLAIR^{®} lyophilized powder). To administer XOLAIR^{®} to a patient, it was necessary to reconstitute the powder with sterile water and then inject the resulting solution. The process for reconstituting XOLAIR^{®} lyophilized powder comprises introducing 0.9 or 1.4mL water for injection to a vial of 75 or 150mg lyophilized powder, respectively, and swirling the vial for 5 to 10 seconds approximately every 5 minutes over the course of around 15 to 20 minutes to allow the powder to dissolve completely. As this process is lengthy and complex, XOLAIR^{®} lyophilized powder can be administered only by a healthcare professional, limiting ease of use and patient experience. As described in US2002/0045571, this reconstituted formulation is about 80 fold more viscous than water at 25 °C.

After initial authorization omalizumab (XOLAIR^{®}) became available as a 75mg/0.5mL and 150mg/1mL solution in a single-dose prefilled syringe for subcutaneous delivery, the syringe having a needle of gauge 26. This eliminated the need for reconstitution immediately before administration, greatly improving ease of use, patient experience and, ultimately, patient compliance and outcomes. Furthermore, XOLAIR^{®} was approved for self-injection, further improving ease of use.

Subcutaneous injection of 150mg/mL omalizumab formulation offers advantages (Shi et al. Subcutaneous Injection Performance in Yucatan Miniature Pigs with and without Human Hyaluronidase and Auto-injector Tolerability in Humans. AAPS PharmSciTech. 2021 Jan; 22(1): 39). Firstly, it provides the possibility of self-administration, limiting the need for patients to visit healthcare providers to receive treatment. Secondly, subcutaneous injections are typically less painful than other injections such as intravenous and intramuscular injections. As a result, subcutaneous injection is often the preferred route of delivery for patients and healthcare professionals alike.

However, there are still difficulties associated with current dosage regimes and modes of administration of omalizumab. Firstly, omalizumab is usually administered in a dosage of 150mg to 375mg subcutaneously every 2 to 4 weeks but only 15% to 20% of patients can receive their XOLAIR^{®} treatment with one single injection using the currently available 75mg/0.5mL and 150mg/1mL doses. Secondly, omalizumab formulations are highly viscous and so subcutaneous injection using the currently available prefilled syringes requires high injection forces and long injection times. As a result, patients and caregivers can lack the ability to complete injections of omalizumab with confidence. Thirdly, omalizumab injections can cause injection site reactions - indeed, in patients with asthma, injection site reactions of any severity occur at a rate of 45% and severe injection site reactions occur at a rate of 12%. This problem is exacerbated by the requirement to administer each injection at a new injection site and that 80% to 85% of patients must administer the contents of multiple syringes to achieve their prescribed dose. Each of these difficulties negatively impact patient experience and convenience and so negatively impact patient adherence to omalizumab treatment. In chronic illnesses such as those treated by omalizumab, non-adherence is estimated at around 50% (Baryakova et al. 'Overcoming barriers to patient adherence: the case for developing innovative drug delivery systems'. Nature Reviews Drug Discovery. March 2023; volume 22, pages 387-409) . Therefore, there is a need to address the above difficulties to improve ease of use and patient experience and so improve patient adherence and outcomes.

### SUMMARY OF THE INVENTION

Currently, omalizumab is provided for use in treating of one or more of: allergic asthma; chronic rhinosinusitis with nasal polyps; chronic spontaneous urticaria; nasal polyps; moderate to severe persistent asthma in patients with a positive skin test or in vitro reactivity to a perennial aeroallergen and symptoms that are inadequately controlled with inhaled corticosteroids. The antibody is subcutaneously administered as a 150mg/mL omalizumab formulation to the patient, by a syringe having a reservoir filled with 0.5mL or 1mL of 150mg/mL omalizumab formulation.

It is an object of the invention to provide new uses of omalizumab, new syringes, and new omalizumab administration/dosage regimens which can overcome the above obstacles to improve ease of use, patient experience, patient adherence and outcomes using omalizumab. In particular, it is an object of the invention to provide new uses which reduce the number of individual injections required per treatment, reduce the time and injection forces required to deliver the omalizumab formulation subcutaneously and to reduce the risk of injection site reactions. It is also an object of the invention to achieve these improvements without loss of antibody stability and purity, and while achieving bioequivalence with the known syringes.

However, there are significant difficulties in providing a mode of administration of omalizumab which achieves these objects because omalizumab is unusually viscous in solution, particularly at the high concentration which is required for a 150mg/mL dosage. These difficulties are described in more detail below.

The XOLAIR^{®} formulation is 150mg/mL antibody with 42.1mg/mL L-arginine hydrochloride, 1.37mg/mL L-histidine, 2.34mg/mL L-histidine hydrochloride monohydrate, 0.4mg/mL polysorbate 20 as an aqueous solution (in sterile water for injection (USP)). Omalizumab XOLAIR^{®} 75mg/0.5mL, 150mg/1mL and 300mg/2mL all have the same formulation, with the high 150mg/mL antibody concentration. The viscosity of omalizumab XOLAIR^{®} is 12 to 14 mPa·s at room temperature. In one experiment, viscosity measured at a shear rate of 200s⁻¹ was: 24.7 ± 0.5 mPa·s at of 5.0 ± 0.2 °C, 15.3 ± 0.5 mPa·s at 15.0 ± 0.2 °C, 11.4 ± 0.5 mPa·s at 25.0 ± 0.2 °C and 7.2 ± 1.2 mPa·s at 40.0 ± 0.2 °C.

The high viscosity of omalizumab formulations presents several difficulties in achieving the object of the invention.

In general, the higher the viscosity a fluid has, the more force and time are required to inject it using a syringe. Patients can struggle to apply high injection forces to syringes and so can fail to push syringe stoppers to their terminal positions within the syringe to administer a complete dose. Furthermore, high injection forces increase the risk of syringe breakage.

Long injection times greatly reduce patient experience and compliance. In general, it is accepted that patients can comfortably hold a syringe in place for a maximum of 20 seconds. Therefore, when using syringes which inject drugs in a time approaching 20 seconds, there is an increased risk of incomplete dosage because patients prematurely remove the syringe from the injection site due to discomfort.

These issues of high injection forces and long injection times are also exacerbated when administering larger volumes of omalizumab (>1mL) because the higher the volume of fluid that must be injected, the more force and time are required to inject it. In addition, higher dosing volumes also result in higher pressures at the injection site, leading to higher chance of reaction at the injection site and associated injection errors.

Lubricants can be included within a syringe (*e.g.* siliconisation) to reduce injection force and times, but they can cause protein aggregation, reducing the purity and effectiveness of the omalizumab solution. In addition, the protein aggregation can cause blockages, thus requiring more force to inject the solution, reducing ease of use. Finally, it is necessary to bear in mind that regulatory and international standards must be met when making any improvements to currently available omalizumab prefilled syringes.

Studies have been conducted to investigate the impact of viscosity, volume and flow rate on patient experience of subcutaneous injection, see: 1) Rini et al. Enabling faster subcutaneous delivery of larger volume, high viscosity fluids. Expert Opinion on Drug Delivery. 2022, Vol. 19, No. 9, 1165-1176, 2) Roberts et al. Novel cannula design improves large volume auto-injection rates for high viscosity solutions. Drug Delivery, 29:1 43-51 and 3) Berteau et al. Evaluation of the impact of viscosity, injection volume, and injection flow rate on subcutaneous injection tolerance. Medical Devices: Evidence and Research 2015: 8 473-484 . However, some of these studies have been conducted on non-biologic solutions and so the applicability of the findings to uses of omalizumab is limited. Berteau et al. (2015) and Rini et al. (2022) make clear that it is difficult to accurately predict the behaviour of any biologic when it is administered subcutaneously because the unique characteristics of each drug will have an impact on its behaviour. These unique characteristics include particle size, shape, settling/resuspension characteristics and aggregation/agglomeration potential and viscoelastic flow behaviour. Berteau et al. (2015) concludes that each drug delivery device for high-volume, high-speed subcutaneous injection must be *specifically* designed for each drug in order to optimise ease of use, patient experience, patient adherence and outcomes.

The inventors have overcome the difficulties set out above to provide new uses of omalizumab and methods of treatment which address the various issues at hand. According to an aspect of the invention, there is provided X mL of 150mg/mL omalizumab formulation for use in a method of treatment of one or more of: allergic asthma; food allergy; chronic rhinosinusitis with nasal polyps; chronic spontaneous urticaria; nasal polyps; moderate to severe persistent asthma in patients with a positive skin test or *in vitro* reactivity to a perennial aeroallergen and symptoms that are inadequately controlled with inhaled corticosteroids, wherein X is 2 and wherein the X mL 150mg/mL omalizumab formulation is administered subcutaneously by a syringe, the syringe comprising:
a reservoir filled with X mL of 150mg/mL omalizumab formulation, wherein the XmL 150mg/mL omalizumab formulation has 6000 or fewer particles of diameter ≥ 10µm and/or 600 or fewer particles of diameter ≥ 25µm; and the syringe further comprising:
a stopper, and
a needle having a gauge of 27; and
the syringe being configured to expel the omalizumab formulation contained in the reservoir through the needle when a force is applied to the stopper.

Providing a syringe containing 2mL of 150mg/mL omalizumab formulation reduces the number of individual injections required per treatment for patients who are prescribed a dose larger than 150mg omalizumab. Reduced dosing frequency improves patient adherence and experience (Baryakova et al. 2023). In addition, a lower number of injections leads to reduced risk of injection site reaction, further improving patient adherence and experience. In this way, the present invention improves ease of use, patient experience, patient adherence and outcomes using omalizumab.

As will be demonstrated below, providing a syringe containing a needle of gauge 27 reduces the time required to deliver the omalizumab as compared to the currently available syringes. In this way, the present invention reduces the injection time required to administer the dose. In addition, since the needle gauge is larger than in the prior art, the needle of the present invention has a smaller outer diameter, reducing pain during needle insertion. Hence, the present invention improves ease of use, patient experience, patient adherence and outcomes using omalizumab.

In some embodiments of the invention, the needle has one of:
a) a minimum internal diameter of 0.277mm;
b) a minimum internal diameter of 0.191mm;
c) a minimum internal diameter of 0.184mm; and
d) a minimum internal diameter of 0.241mm.

Larger minimum internal diameters further reduce the injection time required to administer omalizumab.

According to a second aspect of the invention, there is provided X mL of 150mg/mL omalizumab formulation for use in a method of treatment of one or more of: allergic asthma; food allergy; chronic rhinosinusitis with nasal polyps; chronic spontaneous urticaria; nasal polyps; moderate to severe persistent asthma in patients with a positive skin test or *in vitro* reactivity to a perennial aeroallergen and symptoms that are inadequately controlled with inhaled corticosteroids, , wherein X is 2, 1 or 0.5 and wherein the X mL 150mg/mL omalizumab formulation is administered subcutaneously by a syringe, the syringe comprising:
a reservoir filled with X mL of 150mg/mL omalizumab formulation, wherein the XmL 150mg/mL omalizumab formulation has 6000 or fewer particles of diameter ≥ 10µm and/or 600 or fewer particles of diameter ≥ 25µm; and the syringe further comprising:
a stopper, and
a needle; and
the syringe being configured to expel the omalizumab formulation contained in the reservoir through the needle when a force is applied to the stopper, wherein one of the following options is fulfilled when a constant force of 10N is applied to the stopper,
   a) when X is 2, the syringe is configured to expel the omalizumab formulation contained in the reservoir through the needle in less than 16 seconds;
   b) when X is 1, the syringe is configured to expel the omalizumab formulation contained in the reservoir through the needle in less than 7.5 seconds; and
   c) when X is 0.5, the syringe is configured to expel the omalizumab formulation contained in the reservoir through the needle in less than 4 seconds.

Systems for measuring injection time of syringes and methods of setting up said systems are well known in the art.

The invention reduces the injection forces required, improving ease of use and making it more likely that a complete dose will be administered. Hence, the present invention improves ease of use, patient experience, patient adherence and outcomes using omalizumab.

In some embodiments of the invention wherein X is 2, the syringe is configured to expel the omalizumab formulation contained in the reservoir through the needle in one or more of:
a) less than 8.5 seconds when a constant force of 17N is applied to the stopper; and
b) less than 5 seconds when a constant force of 30N is applied to the stopper.

In some embodiments of the invention wherein X is 2, the syringe is configured to expel X mL of the omalizumab formulation contained in the reservoir through the needle in one or more of:
a) about 16 seconds to about 14 seconds when a constant force of 10N is applied to the stopper;
b) about 8.5 seconds to about 6 seconds when a constant force of 17N is applied to the stopper; and
c) about 5 seconds to about 4 seconds when a constant force of 30N is applied to the stopper.

In some embodiments of the invention wherein X is 1, the syringe is to expel X mL of 150mg/ml omalizumab formulation through the needle in one or more of:
a) less than 3 seconds when a constant force of 17N is applied to the stopper; and
b) less than 2 seconds when a constant force of 30N is applied to the stopper.

In some embodiments of the invention wherein X is 1, the syringe is configured to expel X mL of 150mg/ml omalizumab formulation through the needle in one or more of:
a) less than 7.5 seconds to about 4 seconds when a constant force of 10N is applied to the stopper;
b) less than 3.5 seconds to about 2 seconds when a constant force of 17N is applied to the stopper; and
c) less than 2 seconds to about 1 second when a constant force of 30N is applied to the stopper.

In some embodiments of the invention wherein X is 1, the syringe is configured to expel X mL of 150mg/ml omalizumab formulation through the needle in less than 5 seconds, less than 4.75 seconds, less than 4.5 seconds, less than 4.25 seconds, or in about 4 seconds when a constant force of 10N is applied to the stopper.

In some embodiments of the invention wherein X is 1, the syringe is configured to expel X mL of 150mg/ml omalizumab formulation through the needle in less than 5 seconds to about 4 seconds, less than 4.75 seconds to about 4 seconds, less than 4.5 seconds to about 4 seconds, less than 4.25 seconds to about 4 seconds, or in about 4 seconds when a constant force of 10N is applied to the stopper.

In some embodiments of the invention wherein X is 0.5, the syringe is to expel X mL of 150mg/ml omalizumab formulation through the needle in one or more of:
a) less than 2 seconds when a constant force of 17N is applied to the stopper; and
b) less than 1 second when a constant force of 30N is applied to the stopper.

In some embodiments of the invention wherein X is 0.5, the syringe is to expel X mL of 150mg/ml omalizumab formulation through the needle in one or more of:
a) less than 4 seconds to about 2 seconds when a constant force of 10N is applied to the stopper;
b) less than 2 seconds to about 1 second when a constant force of 17N is applied to the stopper; and
c) less than 1 second to about 0.5 seconds when a constant force of 30N is applied to the stopper.

In some embodiments of the invention, the syringe contains:
a) at least 76% main omalizumab charge variant measured by ion exchange chromatography after being filled with 150mg/mL omalizumab solution stored at 5°C ± 3°C; and/or
b) at least 77% main omalizumab charge variant measured by ion exchange chromatography after being filled with 150mg/mL omalizumab solution and then stored at 5°C ± 3°C for 2.5 months.

In some embodiments of the invention, the percentage of main omalizumab charge variant in the syringe measured by ion exchange chromatography reduces by 2% or less after the syringe is stored at 5°C ± 3°C for 2.5 months (e.g.measured from filling).

In some embodiments of the invention, the proportion of the first peak of the syringe measured by hydrophobic interaction chromatography is at least:
a) 62% after filling with 150mg/mL omalizumab solution stored at 5°C ± 3°C; and/or
b) 62% after being filled with 150mg/mL omalizumab solution and then stored at 5°C ± 3°C for 2.5 months.

In some embodiments of the invention, the proportion of the first peak of the syringe measured by hydrophobic interaction chromatography reduces by 0.4% or less after the syringe is stored at 5°C ± 3°C for 2.5 months.

In some embodiments of the invention, the syringe contains at least 0.4 mg/mL polysorbate 20 after filling with 150mg/mL omalizumab solution stored at 5°C ± 3°C.

In some embodiments of the invention, the syringe contains at least 69% main omalizumab charge variant measured by ion exchange chromatography after being filled with 150mg/mL omalizumab solution and then stored at 25°C ± 3°C for 1.5 months.

In some embodiments of the invention, the proportion of the first peak of the syringe measured by hydrophobic interaction chromatography is at least:
a) 56% for the syringe after being filled with 150mg/mL omalizumab solution and then stored at 25°C ± 3°C for 1.5 months; and/or
b) 51% for the syringe after being filled with 150mg/mL omalizumab solution and then stored at 25°C ± 3°C for 2.5 months.

In some embodiments of the invention, the syringe contains:
a) at least 0.4mg/mL polysorbate 20 after being filled with 150mg/mL omalizumab solution and then stored at 25°C ± 3°C for 1.5 months; and/or
b) at least 0.3mg/mL polysorbate 20 after being filled with 150mg/mL omalizumab solution and then stored at 25°C ± 3°C for 6 months.

Percentage of main omalizumab charge variant, hydrophobic interaction chromatography peak, and polysorbate 20 content are measures of omalizumab purity and stability. As will be demonstrated below, the present invention preserves the purity and stability of omalizumab similarly to current uses.

In some embodiments of the invention, the needle has one of: gauge 23, gauge 24, gauge 25, gauge 26, gauge 27, gauge 28, gauge 29, gauge 30, gauge 31, and gauge 32.

In some embodiments of the invention, the needle has one of:
a) gauge 23 defining a minimum internal diameter of 0.370mm,
b) gauge 23 defining a minimum internal diameter of 0.317mm,
c) gauge 24 defining a minimum internal diameter of 0.343mm,
d) gauge 24 defining a minimum internal diameter of 0.280mm,
e) gauge 25 defining a minimum internal diameter of 0.292mm,
f) gauge 25 defining a minimum internal diameter of 0.232mm,
g) gauge 26 defining a minimum internal diameter of 0.292mm,
h) gauge 26 defining a minimum internal diameter of 0.232mm,
i) gauge 27 defining a minimum internal diameter of 0.277mm;
j) gauge 27 defining a minimum internal diameter of 0.191 mm;
k) gauge 27 defining a minimum internal diameter of 0.184mm;
l) gauge 27 defining a minimum internal diameter of 0.241 mm;
m) gauge 28 defining a minimum internal diameter of 0.133mm,
n) gauge 28 defining a minimum internal diameter of 0.190mm,
o) gauge 29 defining a minimum internal diameter of 0.265mm,
p) gauge 29 defining a minimum internal diameter of 0.240mm,
q) gauge 29 defining a minimum internal diameter of 0.190mm,
r) gauge 29 defining a minimum internal diameter of 0.133mm,
s) gauge 30 defining a minimum internal diameter of 0.240mm,
t) gauge 30 defining a minimum internal diameter of 0.190mm,
u) gauge 30 defining a minimum internal diameter of 0.165mm,
v) gauge 30 defining a minimum internal diameter of 0.133mm,
w) gauge 31 defining a minimum internal diameter of 0.176mm,
x) gauge 31 defining a minimum internal diameter of 0.146mm,
y) gauge 31 defining a minimum internal diameter of 0.125mm,
z) gauge 31 defining a minimum internal diameter of 0.114mm,
aa) gauge 32 defining a minimum internal diameter of 0.146mm,
bb) gauge 32 defining a minimum internal diameter of 0.125mm,
cc) gauge 32 defining a minimum internal diameter of 0.105mm, and
dd) gauge 32 defining a minimum internal diameter of 0.089mm.

In some embodiments of the invention, the needle has a gauge of gauge 25 to gauge 29, preferably gauge 26 to gauge 28, most preferably gauge 27.

In some embodiments when the needle has a gauge of 25, the needle has one of:
a) a minimum internal diameter of 0.292mm, and
b) a minimum internal diameter of 0.232mm.

In some embodiments when the needle has a gauge of 26, the needle has one of:
a) a minimum internal diameter of 0.292mm; and
b) a minimum internal diameter of 0.232mm.

In some embodiments when the needle has a gauge of 27, the needle has one of:
a) a minimum internal diameter of 0.277mm;
b) a minimum internal diameter of 0.191 mm;
c) a minimum internal diameter of 0.184mm; and
d) a minimum internal diameter of 0.241 mm.

In some embodiments when the needle has a gauge of 28, the needle has one of:
a) a minimum internal diameter of 0.133mm; and
b) a minimum internal diameter of 0.190mm.

In some embodiments when the needle has a gauge of 29, the needle has one of:
a) a minimum internal diameter of 0.265mm;
b) a minimum internal diameter of 0.240mm;
c) a minimum internal diameter of 0.190mm; and
d) a minimum internal diameter of 0.133mm;
preferably wherein the needle has a minimum internal diameter of 0.240mm or 0.265mm.

In another preferred embodiment of the invention, the needle has a gauge of 29. In another preferred embodiment of the invention, the needle has a gauge of 29 and a minimum internal diameter of 0.240mm. In another preferred embodiment of the invention, the needle has a gauge of 29 and a minimum internal diameter of 0.265mm.

In some embodiments of the invention, the stopper has a fluoro-resin on the product contacting side or an ethylene tetrafluoroethylene (ETFE) barrier film lamination. The lamination of the stopper reduces friction, reducing injection forces, reducing injection time and making it more likely that a complete dose will be administered. In this way, the present invention improves ease of use, patient experience, patient adherence and outcomes using omalizumab. In addition, the lamination helps to prevent contamination between the material of the stopper and omalizumab, preserving the stability and purity of the omalizumab.

In some embodiments of the invention, the stopper has a B2-40 UV cured lubrication coating or is lubricated with 1000cSt silicone oil. The coating of the stopper reduces friction, reducing injection forces and reducing injection time and making it more likely that a complete dose will be administered. In this way, the present invention improves ease of use, patient experience, patient adherence and outcomes using omalizumab. In addition, the coating of the stopper helps to prevent contamination between the material of the stopper and omalizumab, preserving the stability and purity of the omalizumab.

In some embodiments of the invention, the syringe has an internal coating of between 0.3-1.0 mg (e.g. 0.4 ± 0.2mg or 0.7 ± 0.2mg) of polydimethylsiloxane. The internal coating of the syringe reduces friction, reducing injection forces, reducing injection time and making it more likely that a complete dose will be administered. In this way, the present invention improves ease of use, patient experience, patient adherence and outcomes using omalizumab. In addition, the internal coating of the syringe helps to preserve the stability and purity of the omalizumab.

In some embodiments of the invention, the needle has 3 bevels on its distal surface or 5 bevels on its distal surface. The bevelled distal surface reduces needle insertion pain, improving patient experience and so patient adherence and outcomes.

In some embodiments of the invention, the apparent viscosity of the 150mg/mL omalizumab formulation at a shear rate of 200s⁻¹ is: 24.7 ± 0.5 mPa·s at 5.0 ± 0.2 °C, 15.3 ± 0.5 mPa·s at 15.0 ± 0.2 °C, 11.4 ± 0.5 mPa·s at 25.0 ± 0.2 °C, or 7.2 ± 1.2 mPa·s at 40.0 ± 0.2 °C.

In some embodiments of the invention, the syringe has a latex-free needle shield. The latex-free needle shield reduces risk of injection site reaction, improving patient experience and so patient adherence and outcomes.

In some embodiments of the invention, the syringe has a round flange at a proximal end of the reservoir. In some embodiments of the invention the round flange has a maximum diameter of 11 ± 0.25mm or 14.7 ± 0.25mm. The round flange helps the syringe to better withstand high injection forces, reducing the risk of syringe breakage and improving patient experience. This is particularly advantageous when the syringe of the present invention is used in combination with an autoinjector which holds syringe by the flange.

In some embodiments of the invention, when X is 1 or 0.5, the syringe has a length of 54.0 ± 0.5mm and an internal diameter of 6.35mm ± 0.05mm.

In some embodiments of the invention, when X is 1 or 0.5, when the stopper is not inserted into the reservoir, it has one of:
a) a maximum external diameter of 6.67mm to 7.10mm and/or a length of 7.85 ± 0.4 mm; and
b) a maximum external diameter of 6.70 ± 0.15mm and/or a length of 7.85 ± 0.4 mm.

In some embodiments of the invention when X is 1 or 0.5, the stopper has at least one circumferential rib of outer diameter 6.60 ± 0.15 mm. The circumferential ribs help to reduce contact between the stopper and the syringe, minimising friction and so reducing injection force and time. In this way, the present invention improves patient experience, adherence and outcomes.

In some embodiments of the invention when X is 2, the syringe has a length of 54.0 ± 0.5mm and an internal diameter of 8.65mm ± 0.05mm.

In some embodiments of the invention when X is 2, the stopper has a maximum external diameter 9.05 ± 0.15mm and/or length 7.70 ± 0.4 mm when not inserted into the reservoir.

In some embodiments of the invention, when X is 2, the stopper has at least one circumferential rib of outer diameter 9.00 ± 0.15 mm. The ribs help to reduce contact between the stopper and the syringe, minimising friction and so reducing injection force and time. In this way, the present invention improves patient experience, adherence and outcomes.

In some embodiments of the invention, a break loose force configured to move the stopper of the present invention from an initial stationary point to a velocity of 190mm/min is one or more of:
a) about 2N to about 4N after storage at 5°C ± 3 °C for 2.5 months after filling;
b) about 2N to about 4N after storage at 5°C ± 3 °C for 6 months after filling.

In some embodiments of the invention, a break loose force configured to move the stopper of the present invention from an initial stationary point to a velocity of 190mm/min is one or more of:
a) about 2N to about 3.5N at 25°C ± 3 °C after filling;
b) about 2N to about 4.5N after storage at 25°C ± 3 °C for 1.5 months after filling;
c) about 2.5N to about 5N after storage at 25°C ± 3 °C for 2.5 months after filling; and/or
d) about 2.5N to about 5.5N after storage at 25°C ± 3 °C for 6 months after filling.

In some embodiments of the invention, a break loose force configured to move the stopper of the present invention from an initial stationary point to a velocity of 190mm/min is one or more of:
a) about 3N to about 4.5N after storage at 40°C ± 3 °C for 1.5 months after filling; and
b) about 3N to about 5N after storage at 40°C ± 3 °C for 2.5 months after filling.

The syringes of the present invention require a lower break loose force than the prior art syringes, reducing injection force and time. In addition, the break loose forces of the syringes of the present invention are less variable than in prior art syringes. It has been shown that reliability of syringes is an important factor in reducing injection errors (Menzella et al. Self-administration of omalizumab: why not? A literature review and expert opinion. Expert Opinion on Biological Therapy; Vol. 21, 2021 - Issue 4, pgs 499-507). In this way, the present invention reduces risk of injection errors, improving patient experience, patient adherence and outcomes.

Needle phobia reduces patient adherence (Baryakova et al.). The needle shield device of the present invention covers the needle to improve the experience of patients with needle phobia, improving patient adherence and outcomes. In some embodiments of the invention, the syringe is provided in needle shield device comprising a needle sleeve and a plunger; and wherein
a manual force can be applied to the plunger to expel the 150mg/mL omalizumab formulation and unlatch needle sleeve so that the needle sleeve can cover the needle after injection.

In some embodiments of the invention, the syringe is manufactured by a method comprising:
aseptic filling of the reservoir with the 150mg/mL omalizumab formulation;
and stoppering the reservoir with the stopper.

In some embodiments of the invention, the aseptic filling is performed by a peristaltic pump or stainless steel piston pump.

In some embodiments of the invention, prior to the aseptic filling, the reservoir and needle are sterilised by ethylene oxide gas treatment, electron beam (e-beam) and/or steam sterilisation by autoclave.

While the claims herein relate to 150mg/mL omalizumab formulation for use in a method of treatment, wherein the 150mg/mL omalizumab formulation is administered subcutaneously by a syringe, it will be understood that the syringes of the present invention can be provided alone, or in combination with an autoinjector and that the present invention also encompasses new methods of treatment and omalizumab dosing regimens using the syringe of the present invention. Accordingly, additional clauses covering the present invention are set out below.

The invention has been described in relation to the treatment of: allergic asthma; food allergy; chronic rhinosinusitis with nasal polyps; chronic spontaneous urticaria; nasal polyps; moderate to severe persistent asthma in patients with a positive skin test or in vitro reactivity to a perennial aeroallergen and symptoms that are inadequately controlled with inhaled corticosteroids. More particularly, it can be used for one or more of:
- Moderate to severe persistent asthma in adults and pediatric patients 6 years of age and older with a positive skin test or in vitro reactivity to a perennial aeroallergen and symptoms that are inadequately controlled with inhaled corticosteroids;
- Chronic rhinosinusitis with nasal polyps (CRSwNP) in adult patients 18 years of age and older with inadequate response to nasal corticosteroids, as add-on maintenance treatment;
- IgE-mediated food allergy in adult and pediatric patients aged 1 year and older for the reduction of allergic reactions (Type I), including anaphylaxis, that may occur with accidental exposure to one or more foods (and can be used in conjunction with food allergen avoidance); and/or
- chronic spontaneous urticaria (CSU) in adults and adolescents 12 years of age and older who remain symptomatic despite H1 antihistamine treatment.

### THE DRAWINGS

Figure 1 is a schematic diagram of a syringe for subcutaneous delivery according to the prior art.
Figure 2 shows dosing regimens currently used to administer 150mg/mL omalizumab formulation.
Figure 3 is a schematic diagram of a syringe for subcutaneous delivery according to an embodiment of the invention.
Figure 4 is a schematic diagram of a syringe for subcutaneous delivery according to an embodiment of the invention.
Figure 5 is a schematic diagram of a syringe for subcutaneous delivery according to an embodiment of the invention.
Figure 6 is a schematic diagram of a syringe for subcutaneous delivery according to an embodiment of the invention.
Figure 7 shows experimental results of injection time vs constant force for the prior art syringe and syringes of the present invention.
Figure 8 shows experimental results of break loose force vs storage duration and temperature for the prior art syringe and syringes of the present invention.
Figure 9 shows the percentage of the main omalizumab charge variant measured by ion exchange chromatography for the prior art syringe and syringes of the present invention.
Figure 10 shows the proportion of the first HIC (Hydrophobic Interaction Chromatography) peak for the prior art syringe and syringes of the present invention
Figure 11 shows the proportion of the second HIC peak for the prior art syringe and syringes present invention.
Figure 12 shows the proportion of the third HIC peak for the prior art syringe and syringes of the present invention.
Figure 13 shows the polysorbate 20 (PS20) content of the prior art syringe and syringes of the present invention.
Figures 14A and 14B show schematic diagrams of a prefilled syringe with a needle shield device
Figure 15 is a schematic diagram of a prefilled syringe within an autoinjector.
Figure 16 shows the measured time to expel the 150mg/mL omalizumab formulation out of the reservoir and through the needle using an autoinjector when combined with syringes of the invention.
Figure 17 shows new dosing regimens of 150mg/mL omalizumab formulation in accordance with the present invention.

### DETAILED DESCRIPTION

### INTRODUCTION

The present invention provides new uses of omalizumab, new syringes and new administration regimens which improve ease of use, patient experience, patient adherence and outcomes using omalizumab. In particular, the present invention provides new uses of omalizumab, new syringes and new administration regimens which reduce the number of individual injections required per treatment, reduce the time and injection forces required to deliver the omalizumab formulation subcutaneously and reduce the risk of injection site reactions.

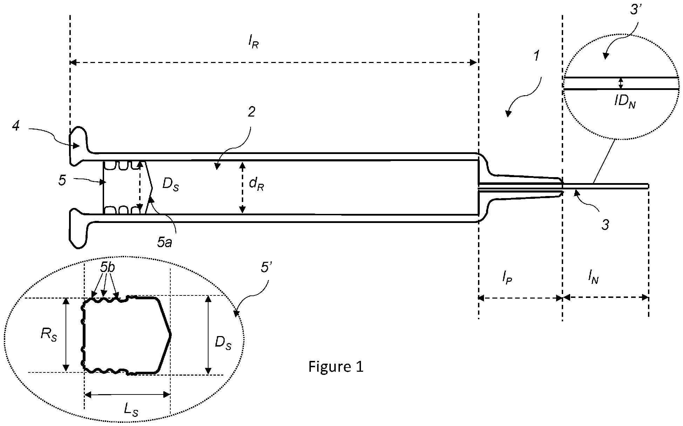

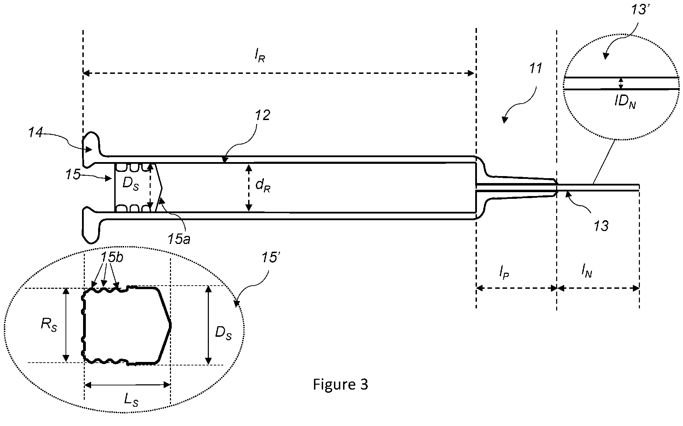

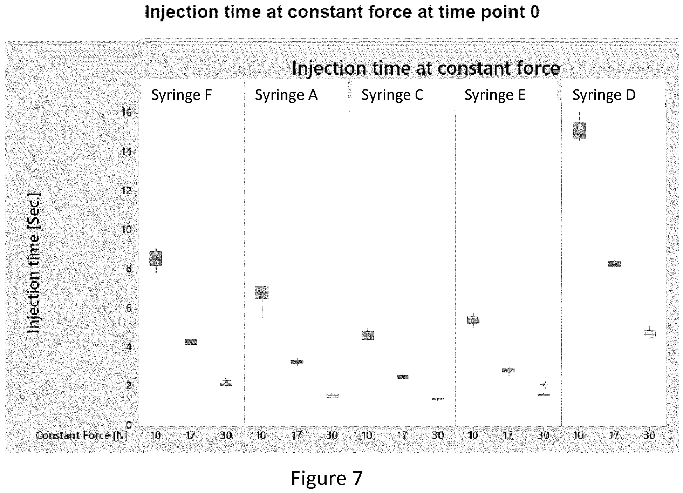

Below is a detailed description of the present invention and its advantages and improvements over the current uses of omalizumab, current syringes and current administration regimens. The detailed description of the present invention is structured as follows. Firstly, the syringe currently used to administer omalizumab subcutaneously is described with reference to Figure 1. The method of manufacturing the syringe of Figure 1 is also described. Secondly, seven embodiments of the syringes of the present invention, along with methods of manufacture, are described with reference to Figures 3 to 6. Thirdly, experimental results of tests conducted on the prior art syringes and five embodiments of syringes of the present invention are described with reference to Figures 7 to 13.

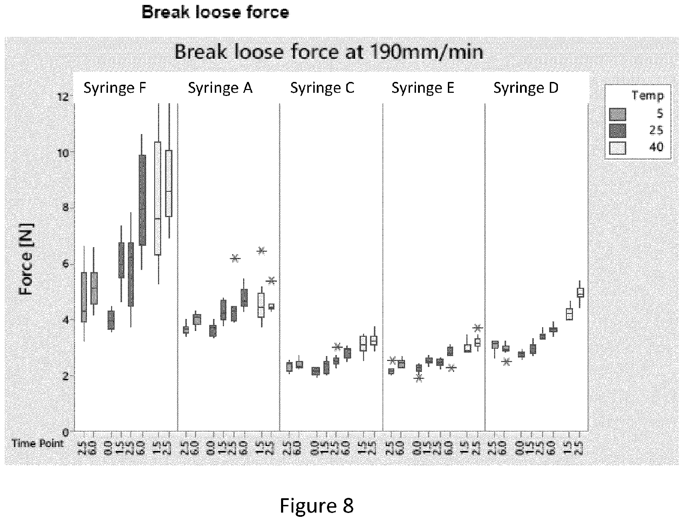

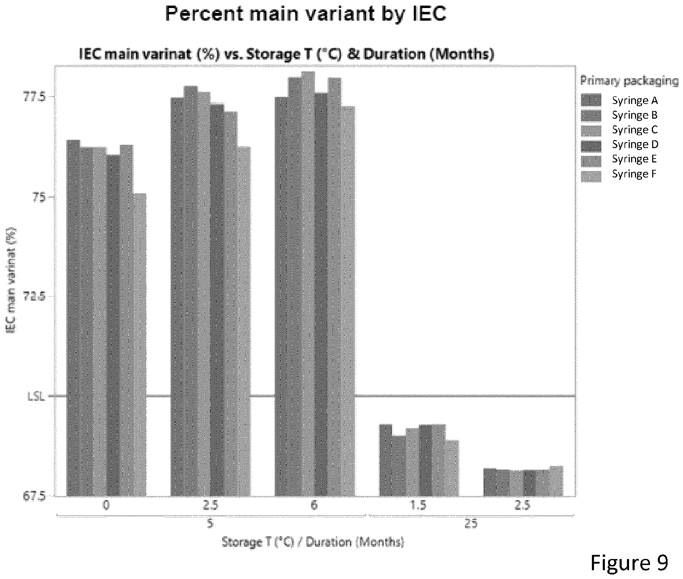

As will be explained in detail below, the experimental results of tests conducted on the prior art syringe and five embodiments of syringes of the invention demonstrate the following points.
i) The syringes of the present invention expel omalizumab in less time than prior art syringes when forces are applied to the stopper see Figure 7 and related description).
ii) Multiple characteristics of the syringes of the present invention contribute to the reduced injection time referenced in point (i), including but not limited to: needle gauge, needle minimum internal diameter, stopper lamination, stopper coating, number of bevels on the needle distal surface, syringe flange, stopper maximum internal diameter and diameter of outer rib on the stopper (see Figure 7 and related description).
iii) The syringes of the present invention require a lower break loose force than the prior art syringe (see Figure 8 and related description).
iv) The syringes of the present invention preserve the purity and stability of omalizumab and thus improve the bioavailability of omalizumab (see Figures 9 to 13 and related description).

As a result, the experimental results demonstrate that the inventors have provided syringes which reduce the time and injection forces required to deliver the omalizumab formulation subcutaneously and reduce the risk of injection site reactions.

In particular, according to one aspect of the present invention, there is provided X mL of 150mg/mL omalizumab formulation for use in a method of treatment of one or more of: allergic asthma; food allergy; chronic rhinosinusitis with nasal polyps; chronic spontaneous urticaria; nasal polyps; moderate to severe persistent asthma in patients with a positive skin test or in vitro reactivity to a perennial aeroallergen and symptoms that are inadequately controlled with inhaled corticosteroids, wherein X is 2, 1 or 0.5 and wherein the X mL 150mg/mL omalizumab formulation is administered subcutaneously by a syringe, the syringe comprising:
a reservoir filled with X mL of 150mg/mL omalizumab formulation, wherein the X mL 150mg/mL omalizumab formulation has 6000 or fewer particles of diameter ≥ 10µm and/or 600 or fewer particles of diameter ≥ 25µm; and the syringe further comprising:
a stopper, and
a needle; and
the syringe being configured to expel the omalizumab formulation contained in the reservoir through the needle when a force is applied to the stopper, wherein one of the following options is fulfilled when a constant force of 10N is applied to the stopper,
   a) when X is 2, the syringe is configured to expel the omalizumab formulation contained in the reservoir through the needle in less than 16 seconds;
   b) when X is 1, the syringe is configured to expel the omalizumab formulation contained in the reservoir through the needle in less than 7.5 seconds; and
   c) when X is 0.5, the syringe is configured to expel the omalizumab formulation contained in the reservoir through the needle in less than 4 seconds.

As indicated in point (ii) above, multiple characteristics of the syringes of the present invention contribute to the reduced injection time achieved in the one aspect of the invention. However, as demonstrated in Figures 7 and 8 and the accompanying description, a needle gauge of 27 is particularly advantageous in achieving the reduced injection time. Therefore, according to an aspect of the invention, the present invention provides X mL of 150mg/mL omalizumab formulation for use in a method of treatment of one or more of: allergic asthma; food allergy; chronic rhinosinusitis with nasal polyps; chronic spontaneous urticaria; nasal polyps; moderate to severe persistent asthma in patients with a positive skin test or in vitro reactivity to a perennial aeroallergen and symptoms that are inadequately controlled with inhaled corticosteroids, wherein X is 2, wherein the omalizumab formulation is administered subcutaneously by a syringe, the syringe comprising:
a reservoir filled with X mL of 150mg/mL omalizumab formulation, wherein the X mL 150mg/mL omalizumab formulation has 6000 or fewer particles of diameter ≥ 10µm and/or 600 or fewer particles of diameter ≥ 25µm; and the syringe further comprising:
a stopper, and
a needle having a gauge of 27; and
the syringe being configured to expel the omalizumab formulation contained in the reservoir through the needle when a force is applied to the stopper.

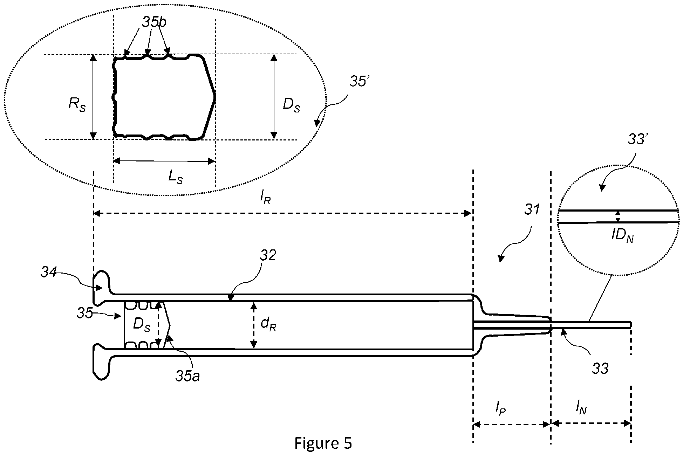

As will be demonstrated with respect to Figures 7 and 8, for syringes which administers 0.5mL or 1mL omalizumab, the syringe of the present invention which administers omalizumab fastest is the syringe described with reference to Figure 5. Accordingly, one preferred embodiment of the invention is a syringe according to the first or second aspects above, wherein one or more of the following is provided.
a) The syringe is configured to expel 1mL 150mg/mL omalizumab formulation in one or more of: 4.64 ± 0.22 seconds when a constant force of 10N is applied to the stopper, 2.54 ± 0.11 seconds when constant force of 17N is applied to the stopper, and 1.39 ± 0.04 seconds when a constant force of 30N is applied to the stopper.
b) The syringe has an internal coating of 0.4 ± 0.2mg of polydimethylsiloxane (PDMS).
c) The syringe has a needle of gauge 27 defining a minimum internal diameter *ID_{N}* of 0.277mm.
d) The stopper, when it is not inserted into the syringe reservoir, has a maximum external diameter *Dₛ*6.70 ± 0.15mm and length *Lₛ* 7.85 ± 0.4 mm.
e) The stopper has a conically shaped distal surface and at least one circumferential rib of outer diameter *R_{S}* 6.60 ± 0.15 mm.
f) The stopper is formed from bromobutyl elastomer and has an ethylene tetrafluoroethylene (ETFE) barrier film lamination.
g) The stopper has a B2-40 UV cured lubrication coating.

Finally, since the syringes of the present invention achieve reduced injection time, reduced break loose force as compared to syringes of the prior art, while maintaining comparable purity, stability and bioavailability of omalizumab, the syringes of the invention can be used to administer larger volumes of omalizumab than prior art syringes, i.e. larger than 0.5mL or 1mL 150mg/mL omalizumab.

### TERMINOLOGY USED IN THE DETAILED DESCRIPTION

The term 'distal' refers to a location that is relatively closer to a site of injection, and the term `proximal' refers to a location that is relatively further away from the injection site. The term 'about' in relation to a numerical value *x* is optional and means *x* ± 5%.

### Fill volume

It is necessary to fill syringes in order to account for residue left in the syringe and ensure that the recommended dose is expelled from the syringe. Accordingly, in this application, where syringes are referred to as being filled with 0.5mL of 150mg/mL omalizumab formulation, this should be understood as 0.520mL ± 5%. Where syringes are referred to as being filled with 1mL of 150mg/mL omalizumab formulation, this should be understood as 1.020mL ± 2.5%. Where syringes are referred to as being filled with 2mL of 150mg/mL omalizumab formulation in this application, this should be understood as 2.040mL ± 2.75%.

### Extractable volume

When 150mg/mL omalizumab formulation is expelled from a syringe, some residue will be left behind. In this application, where syringes are referred to as expelling 0.5mL, this should be understood as 0.5mL ± 5%. Where syringes are referred to as expelling 1mL, this should be understood as 1ml ± 5%. Where syringes are referred to as expelling 2mL, this should be understood as 2ml ± 5%.

### Omalizumab formulation

Containers with a nominal content of less than 100mL of 150mg/mL omalizumab formulation (150mg/mL antibody with 42.1mg/mL L-arginine hydrochloride, 1.37mg/mL L-histidine, 2.34mg/mL L-histidine hydrochloride monohydrate, 0.4mg/mL polysorbate 20 as an aqueous solution (in sterile water for injection (USP)) have 6000 or fewer particles of diameter ≥ 10µm and/or 600 or fewer particles of diameter ≥ 25µm. The number of particles of diameter ≥ 10µm and/or 25µm can be measured by a light obscuration particle count test or a microscopic particle count test as set out in United States Pharmacopeia, Chapter 788

Omalizumab formulations used according to the invention can include a histidine buffer, arginine hydrochloride, and a polysorbate (such as polysorbate 20). They can have a pH in the range of 5.5-6.5. One useful formulation for 150mg/mL omalizumab has 42.1mg/mL arginine hydrochloride, 1.37mg/mL histidine, 2.34mg/mL histidine hydrochloride monohydrate, and 0.4mg/mL polysorbate 20.

### CURRENT USES OF OMALIZUMAB

### Current syringe (also referred to as the prior art syringe)

Figure 1 shows a schematic diagram of a syringe 1 currently used to provide 150mg/mL omalizumab (XOLAIR^{®}) solution. The syringe has reservoir 2 length *I_{R}* 54 ± 0.5mm and an internal diameter *d_{R}* of 6.35 ± 0.1mm filled with 0.5mL or 1 mL 150mg/mL omalizumab (XOLAIR^{®}) solution. The length *I_{R}* of the syringe is the maximum internal length of the syringe reservoir. The syringe is formed from borosilicate glass and the inner surface of the syringe has an internal coating of ≤ 1.0 mg of polydimethylsiloxane (PDMS) with a kinematic viscosity of about 1000cSt. A needle 3 has a length *l_{N}* of 12.7 ± 0.2mm extending outside of the syringe, a gauge *G_{N}* of 26 defining a minimum internal diameter *ID_{N}* of 0.232mm (inset 3' shows close up view of internal diameter *ID_{N}*) and 3 bevels on its distal surface. The needle 3 has an internal coating of polydimethylsiloxane (PDMS) with a kinematic viscosity of about 12500cSt. The needle 3 is staked meaning that it is inserted into the syringe and secured in place with adhesive; the length of the fluid path *I_{P}* between the distal end of the reservoir and the proximal end of the part of the needle 3 extending from the syringe is about 8mm. At a proximal end of the reservoir is a flange 4 in the form of a cut flange.

The syringe also comprises a stopper 5 which, when it is not inserted into the syringe reservoir 2, has a maximum external diameter *D_{S}* of6.67mm to 7.10mm and length *L_{S}* of 7.85 ± 0.4 mm. The stopper has a conically shaped distal surface 5a and at least three circumeferential ribs 5b of outer diameter *R_{S}* 6.60 ± 0.1 mm. A close up of the stopper is shown inset, labelled 5'. The stopper is formed from bromobutyl elastomer and has a fluoro-resin on the product contacting side. The stopper is lubricated with DC360 Medical Fluid 1000cSt silicone oil.

Before use, the needle is enclosed by a rubber needle shield (not shown) to maintain needle sterility. The rubber needle shield is formed from styrene butadiene rubber which is made with epoxypyrene, derived from natural rubber latex. The rubber needle shield is enclosed by an outer rigid polypropylene cap.

The syringe 1 is a BD Hypak^{™} 1mL glass prefillable syringe with staked needle fitted with a stopper 5 which is a BD (West) 4023/50 FluroTec^{®} stopper. The BD Hypak^{™} 1mL glass prefillable syringe with staked needle is manufactured by Becton Dickinson. The elastomeric formulation of the bromobutyl elastomer is 4023/50 and the fluoro-resin is a FluroTec^{®} coating. The stopper 5 is manufactured by West Pharmaceutical Services and supplied by Becton Dickinson. The rubber needle shield is a FM27/0 rubber component and is enclosed by outer rigid polypropylene cap; the rubber needle shield is manufactured by AptarGroup.

### Method of manufacturing the prior art syringe

The method of manufacturing the prior art syringe shown in and described with reference to Figure 1 is set out below.

The first step is thawing and homogenizing frozen pre-formulated omalizumab 15% drug substance. Thawing can be achieved by circulating heat transfer fluid through a jacketed vessel containing the omalizumab 15% drug substance. Homogenization can be achieved by recirculating the thawed omalizumab 15% drug substance through a recirculation loop of the jacketed vessel.

The second step is filtering and mixing the omalizumab 15% drug substance. Filtering can be achieved by passing the omalizumab 15% drug substance through a 0.2µm rated membrane filter made from polyvinylidene fluoride. Mixing can be achieved with a magnetically coupled stirrer within a jacketed storage vessel holding the omalizumab 15% drug substance. Optionally, the omalizumab 15% drug substance can be pooled and refrozen during this second step.

The third step is in-line sterile filtration of the omalizumab 15% drug substance. In-line filtration can be achieved by passing the omalizumab 15% drug substance through a 0.2µm rated membrane filter made from polyvinylidene fluoride.

The fourth step is aseptic filling of a reservoir 2 with staked needle 3 as shown in and described with reference to Figure 1. The reservoir 2 is filled with 1mL or 0.5mL omalizumab formulation by a stainless steel piston pump. Dosing accuracy can be monitored off-line by determination of extractable volume. Prior to aseptic filling, the following processes are carried out:
a) the reservoir 2 and staked needle 3 are washed with water for injection (WFI) and the internal coating of ≤ 1.0 mg of polydimethylsiloxane (PDMS) with a kinematic viscosity of about 1000cSt is sprayed onto the reservoir body with a diving nozzle;
b) the rubber needle shield and outer rigid polypropylene cap are assembled with the reservoir 2 and staked needle 3 so that they enclose the staked needle 3; and
c) the assembled reservoir 2, staked needle 3, rubber needle shield and outer rigid polypropylene cap undergo steam sterilization by autoclave.

The fifth step is syringe stoppering i.e. the placement of the stopper 5 into the reservoir 2. The stopper 5 can be placed into the appropriate position in the reservoir 2 by stopper placement tube technology. Prior to syringe stoppering, the stopper 5 is steam sterilized by autoclave.

### Current dosing regimens of omalizumab

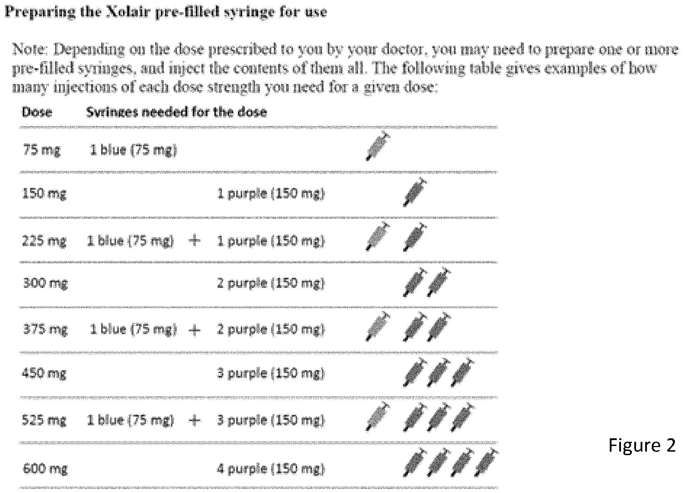

Figure 2 shows dosing regimens currently used to administer 150mg/mL omalizumab formulation. 150mg/mL omalizumab formulation is administered in doses of 75mg to 600mg by subcutaneous injection every 2 or 4 weeks. 150mg/mL omalizumab formulation is currently provided in syringes containing 0.5mL or 1mL 150mg/mL omalizumab formulation. Therefore, as shown in Figure 2, if a patient requires a dose of 225mg omalizumab, the dose must be administered using one syringe containing 1mL 150mg/mL omalizumab formulation and one syringe containing 0.5mL 150mg/mL omalizumab formulation. If a patient requires a dose of 300mg omalizumab, the dose must be administered using two syringes, each containing 1mL 150mg/mL omalizumab formulation. If a patient requires a dose of 375mg omalizumab, the dose must be administered using three syringes-two syringes each containing 1mL 150mg/mL omalizumab formulation and one syringe containing 0.5mL 150mg/mL omalizumab formulation. If a patient requires a dose of 450mg omalizumab, the dose must be administered using three syringes each containing 1mL 150mg/mL omalizumab formulation. If a patient requires a dose of 525mg omalizumab, the dose must be administered using four syringes - three syringes each containing 1mL 150mg/mL omalizumab formulation and one syringe containing 0.5mL 150mg/mL omalizumab formulation. If a patient requires a dose of 600mg omalizumab, the dose must be administered using four syringes, each containing 1mL 150mg/mL omalizumab formulation.

### Difficulties with current uses of omalizumab

The difficulties with current uses of omalizumab were set out in detail above. To summarise, the syringe 1 currently used to provide 150mg/mL omalizumab formulation is unsuitable for use with volumes greater than 1mL. Due to the high viscosity of the 150mg/mL omalizumab solution, high injection forces are required to inject more than 1mL of 150mg/mL omalizumab formulation in an injection time which is acceptable for patients. As a result, patients can struggle to apply the necessary injection forces and so can fail to push stopper 5 to its terminal position within the syringe 1 to administer a complete dose. Furthermore, even when the syringe 1 is used to provide 0.5mL or 1mL 150mg/mL omalizumab formulation, it would be advantageous to reduce the injection forces and time required, and any risk of injection site reaction.

### NEW USES OF OMALZIUMAB

Figures 3 to 6 show syringes which are part of the invention.

### First and second embodiments of the invention:

Figure 3 shows a syringe 11 according to the invention. The syringe has a reservoir 12 of length *I_{R}* of 54.0 ± 0.5mm and an internal diameter *d_{R}* of 6.35mm ± 0.05mm filled with 0.5mL (in a first embodiment) or 1 mL (in a second embodiment) 150mg/mL omalizumab (XOLAIR^{®}) solution. The syringe 11 is formed from borosilicate glass and the inner surface of the syringe has an internal coating of 0.4 ± 0.2mg of polydimethylsiloxane (PDMS). The syringe has a round flange 14 of maximum diameter 11 ± 0.25mm. The syringe comprises a staked needle 13 having a length *I_{N}* 12.7mm extending outside of the syringe and a gauge 27 defining a minimum internal diameter *ID_{N}* of 0.277mm (inset 13' shows close up view of internal diameter *ID_{N}*). The needle 13 is staked meaning that it is inserted into the syringe and secured in place with adhesive; the length of the fluid path *I_{P}* between the distal end of the reservoir and the proximal end of the part of the needle 13 extending from the syringe is about 8mm. The needle has 5 bevels on its distal surface. Before use, the needle 13 is enclosed by an elastomer needle shield to maintain needle sterility (not shown). The elastomer needle shield can comprise an outer rigid polypropylene cap. The needle shield is latex free.

The syringe also comprises a stopper 15 which, when it is not inserted into the syringe reservoir 12, has a maximum external diameter *Dₛ* 6.67mm to 7.10mm and length *L_{S}* 7.85 ± 0.4 mm. The stopper has a conically shaped distal surface 15a and at least one circumferential rib 15b of outer diameter 6.60 ± 0.1 mm. A close up of the stopper is shown inset, labelled 15'. The stopper is formed from bromobutyl elastomer and has a fluoro-resin on the product contacting side. The stopper is lubricated with DC360 Medical Fluid 1000cSt silicone oil.

The syringe 11 is a BD Neopak^{™} 1mL long glass prefillable syringe with staked needle fitted with a stopper 15 which is a BD (West) 4023/50 FluroTec^{®} stopper. The BD Neopak^{™} 1mL long glass prefillable syringe with staked needle is manufactured by Becton Dickinson. The elastomeric formulation of the bromobutyl elastomer is 4023/50 and the fluoro-resin is a FluroTec^{®} coating. The stopper 15 is manufactured by West Pharmaceutical Services and supplied by Becton Dickinson.

### Third and fourth embodiments of the invention:

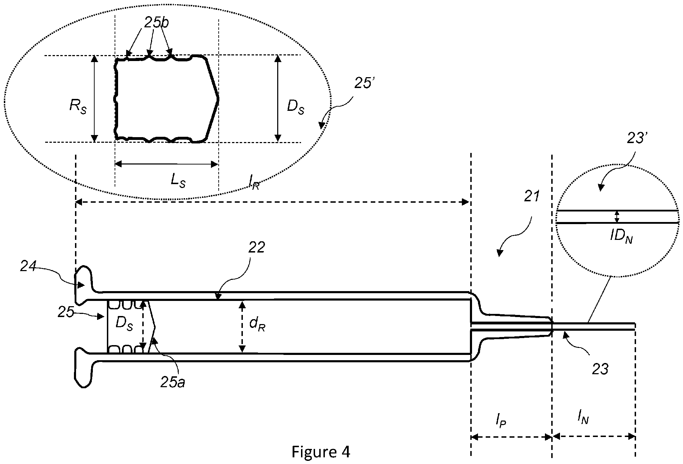

Figure 4 shows a syringe 21 according to the invention. The syringe 21 has a reservoir 22 of length *I_{R}* of 54 ± 0.5mm and an internal diameter *d_{R}* of 6.35mm ± 0.05mm filled with 0.5mL (in a third embodiment) or 1 mL (in a fourth embodiment) 150mg/mL omalizumab (XOLAIR^{®}) solution. The syringe is formed from borosilicate glass and the inner surface of the syringe has an internal coating of silicone oil. The syringe has a round flange 24 of maximum diameter 11 ± 0.25mm. The syringe comprises a needle having a length *I_{N}* 12.7mm extending outside of the syringe and a gauge 27 defining a minimum internal diameter *ID_{N}* of 0.191mm (inset 23' shows close up view of internal diameter *ID_{N}*). The needle 23 is staked meaning that it is inserted into the syringe and secured in place with adhesive; the length of the fluid path *I_{P}* between the distal end of the reservoir and the proximal end of the part of the needle 23 extending from the syringe is about 8mm. Before use, the needle 23 is enclosed by an elastomer needle shield to maintain needle sterility (not shown). The elastomer needle shield can comprise an outer rigid polypropylene cap. The needle shield is latex free.

The syringe also comprises a stopper 25 which, when it is not inserted into the syringe reservoir 22, has a maximum external diameter *D_{S}* 6.70 ± 0.15mm and length *L_{S}* 7.85 ± 0.4 mm. The stopper has a conically shaped distal surface 25a and at least one circumferential rib 25b of outer diameter Rs 6.60 ± 0.15 mm. A close up of the stopper is shown inset, labelled 25'. The stopper is formed from bromobutyl elastomer and has an ethylene tetrafluoroethylene (ETFE) barrier film lamination. The stopper has a B2-40 UV cured lubrication coating; this is a coating in which PDMS is sprayed onto the stopper, heated and UV cured to the surface of the stopper.

The syringe 21 is a Nexa^{®} 1mL long glass prefillable syringe with staked needle fitted with a stopper 25 which is a West NovaPure^{®} 1mL long stopper. The Nexa^{®} 1mL glass prefillable syringe with staked needle is manufactured by Ompi, a Stevanato Group Brand. The elastomeric formulation of the bromobutyl elastomer is 4023/50 and the ethylene tetrafluoroethylene (ETFE) barrier film lamination is a FluroTec^{®} coating. The stopper 25 is manufactured and supplied by West Pharmaceutical Services.

### Fifth and sixth embodiments of the invention:

Figure 5 shows a syringe 31 according the invention. The syringe has a reservoir 32 of length *I_{R}* of 54.0 ± 0.5mm and an internal diameter *d_{R}* of 6.35mm ± 0.05mm filled with 0.5mL (in a fifth embodiment) or 1 mL (in a sixth embodiment) 150mg/mL omalizumab (XOLAIR^{®}) solution. The syringe 31 is formed from borosilicate glass and the inner surface of the syringe has an internal coating of 0.4 ± 0.2mg of polydimethylsiloxane (PDMS). The syringe has a round flange 34 of maximum diameter 11 ± 0.25mm. The syringe comprises a needle 33 having a length *I_{N}* 12.7mm extending outside of the syringe and a gauge 27 defining a minimum internal diameter *ID_{N}* of 0.277mm (inset 33' shows close up view of internal diameter *ID_{N}*). The needle 33 is staked meaning that it is inserted into the syringe and secured in place with adhesive; the length of the fluid path *I_{P}* between the distal end of the reservoir and the proximal end of the part of the needle 33 extending from the syringe is about 8mm. The needle has 5 bevels on its distal surface. Before use, the needle 33 is enclosed by an elastomer needle shield to maintain needle sterility (not shown). The elastomer needle shield can comprise an outer rigid polypropylene cap. The needle shield is latex free.

The syringe also comprises a stopper 35 which, when it is not inserted into the syringe reservoir 32, has a maximum external diameter *D_{S}* 6.70 ± 0.15mm and length *L_{S}* 7.85 ± 0.4 mm. The stopper has a conically shaped distal surface 35a and at least one circumferential rib 35b of outer diameter Rs 6.60 ± 0.15 mm. A close up of the stopper is shown inset, labelled 35'. The stopper is formed from bromobutyl elastomer and has an ethylene tetrafluoroethylene (ETFE) barrier film lamination. The stopper has a B2-40 UV cured lubrication coating; this is a coating in which PDMS is sprayed onto the stopper, heated and UV cured to the surface of the stopper.

The syringe 31 is a BD Neopak^{™} 1mL long glass prefillable syringe with staked needle fitted with a stopper 35 which is a West NovaPure^{®} 1mL long stopper. The BD Neopak^{™} 1mL long glass prefillable syringe with staked needle is manufactured by Becton Dickinson. The elastomeric formulation of the bromobutyl elastomer is 4023/50 and the ethylene tetrafluoroethylene (ETFE) barrier film lamination is a FluroTec^{®} coating. The stopper 35 is manufactured and supplied by West Pharmaceutical Services.

### Seventh embodiment of the invention:

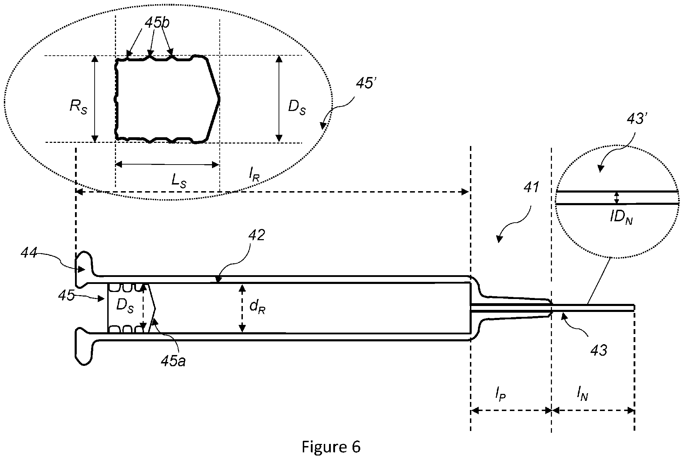

Figure 6 shows a syringe 41 according to a seventh embodiment of the present invention. The syringe has a reservoir 42 of length *I_{R}* of 54.5 ± 0.4mm and an internal diameter *d_{R}* of 8.65mm ± 0.1mm filled with 2mL 150mg/mL omalizumab (XOLAIR^{®}) solution. The syringe is formed from borosilicate glass and the inner surface of the syringe has an internal coating of 0.7 ± 0.2mg polydimethylsiloxane (PDMS). The syringe has a round flange 44 of maximum diameter 14.7 ± 0.25mm. The syringe comprises a needle having a length *I_{N}* 12.7mm extending outside of the syringe and a gauge 27 defining a minimum internal diameter *ID_{N}* of 0.277mm (inset 43' shows close up view of internal diameter *ID_{N}*). The needle 43 is staked meaning that it is inserted into the syringe and secured in place with adhesive; the length of the fluid path *I_{P}* between the distal end of the reservoir and the proximal end of the part of the needle 43 extending from the syringe is about 8mm. The needle has 5 bevels on its distal surface. Before use, the needle 43 is enclosed by a thermoplastic elastomer needle shield to maintain needle sterility. The needle shield can comprise an outer rigid polypropylene cap. The needle shield is latex free.

The syringe also comprises a stopper 45 which, when it is not inserted into the syringe, has a maximum external diameter *D_{S}* 9.05 ± 0.15mm and length *L_{S}* 7.70 ± 0.4 mm. The stopper has a conically shaped distal surface 45a, and comprises at least one circumferential rib 45b of outer diameter *R_{S}* 9.00 ± 0.15 mm. A close up of the stopper is shown inset, labelled 45'. The stopper is formed from bromobutyl elastomer and has an ethylene tetrafluoroethylene barrier film lamination. The stopper has a B2-40 UV cured lubrication coating; this is a coating in which PDMS is sprayed onto the stopper, heated and UV cured to the surface of the stopper.

The syringe 41 is a BD Neopak^{™} 2.25mL glass prefillable syringe with staked needle fitted with a stopper 45 which is a West NovaPure^{®} 1-3mL stopper. The BD Neopak^{™} 2.25mL glass prefillable syringe with staked needle is manufactured by Becton Dickinson. The elastomeric formulation of the bromobutyl elastomer is 4023/50 and the ethylene tetrafluoroethylene (ETFE) barrier film lamination is a FluroTec^{®} coating. The stopper 45 is manufactured and supplied by West Pharmaceutical Services.

According to the invention, each of the syringes of Figures 3 to 6 can be used to administer 150mg/mL omalizumab formulation subcutaneously.

### Methods of manufacturing the syringes according to the present invention

A method of manufacturing the syringes according the present invention is set out below.

The first step is thawing and homogenizing frozen pre-formulated omalizumab 15% drug substance. Thawing can be achieved by circulating heat transfer fluid through a jacketed vessel containing the omalizumab 15% drug substance. Homogenization can be achieved by recirculating the thawed omalizumab 15% drug substance through a recirculation loop of the jacketed vessel.

The second step is filtering and mixing the omalizumab 15% drug substance. Filtering can be achieved by passing the omalizumab 15% drug substance through a 0.2µm rated membrane filter made from polyvinylidene fluoride. Mixing can be achieved with a magnetically coupled stirrer within a jacketed storage vessel holding the omalizumab 15% drug substance. Optionally, the omalizumab 15% drug substance can be pooled and refrozen during this second step.

The third step is in-line sterile filtration of the omalizumab 15% drug substance. In-line filtration can be achieved by passing the omalizumab 15% drug substance through a 0.2µm rated membrane filter made from polyvinylidene fluoride.

The fourth step is aseptic filling of a reservoir 12, 22, 32, 42 with staked needle 13, 23, 33, 43 as shown in and described with reference to any one of Figures 3 to 6. The reservoir 12, 22, 32, 42 is filled with 0.5mL, 1mL or 2mL omalizumab formulation by a peristaltic pump. Dosing accuracy can be monitored by automated weighing of syringes before and after filing.

Aseptic filling by peristaltic pump has advantages over aseptic filling by stainless steel piston pumps. Firstly, peristaltic pumps exert less stress on protein than stainless steel piston pumps. Secondly, unlike stainless steel piston pumps, peristaltic pumps do not require cleaning when used for single-use dispensing. In this way, the peristaltic pump reduces the chance of cross-contamination. Thirdly, peristaltic pumps are faster to set up and calibrate than stainless steel piston pumps.

Prior to aseptic filling, the following processes are carried out:
a) the reservoir 12, 22, 32, 42 and staked needle 13, 23, 33, 43 are washed with water for injection (WFI) and the internal coating of 0.4 ± 0.2mg of polydimethylsiloxane (PDMS) with a kinematic viscosity of about 1000cSt is sprayed onto the reservoir body with a diving nozzle;
b) the elastomer needle shield and outer rigid polypropylene cap are assembled with the reservoir 12, 22, 32, 42 and staked needle 3 so that they enclose the staked needle 13, 23, 33, 43; and
c) the assembled reservoir 12, 22, 32, 42, staked needle 13, 23, 33, 43, elastomer needle shield and outer rigid polypropylene cap are sterilised by ethylene oxide gas treatment.

The fifth step is syringe stoppering i.e. the placement of the stopper 15, 25, 35, 45 into the reservoir 12, 22, 32, 42. The stopper 15, 25, 35, 45 can be placed into the appropriate position in the reservoir 12, 22, 32, 42 by stopper placement tube technology. Prior to syringe stoppering, the stopper 15, 25, 35, 45 is sterilized. This sterilisation can be achieved by steam sterilisation by autoclave, electron beam (e-beam) and/or ethylene oxide gas treatment.

Accordingly, the present invention provides X mL of 150mg/mL omalizumab formulation for use in a method of treatment of one or more of: allergic asthma; food allergy; chronic rhinosinusitis with nasal polyps; chronic spontaneous urticaria; nasal polyps; moderate to severe persistent asthma in patients with a positive skin test or *in vitro* reactivity to a perennial aeroallergen and symptoms that are inadequately controlled with inhaled corticosteroids, wherein X is 2, 1 or 0.5 and wherein the X mL 150mg/mL omalizumab formulation is administered subcutaneously by a syringe according to the present invention, wherein the syringe is manufactured by a method comprising:
aseptic filling of the reservoir with the 150mg/mL omalizumab formulation;
and stoppering the reservoir with the stopper.

In a further embodiment, the present invention provides XmL of 150mg/mL omalizumab formulation, wherein the aseptic filling is performed by a peristaltic pump or stainless steel piston pump.

In a further embodiment, the present invention provides XmL of 150mg/mL omalizumab formulation wherein, prior to the aseptic filling, the reservoir and needle are sterilised by ethylene oxide gas treatment, electron beam (e-beam) and/or steam sterilisation by autoclave.

### New dosing regimens of omalizumab

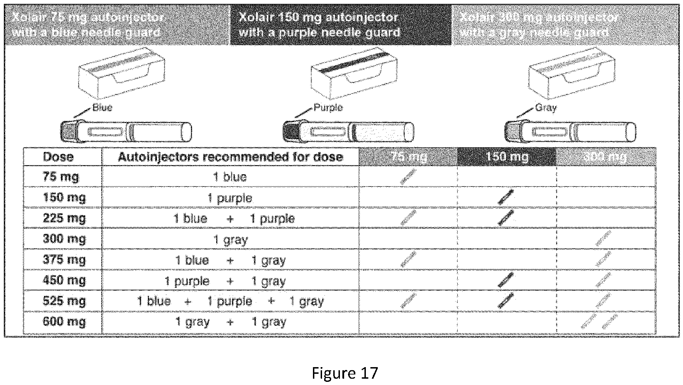

Figure 17 shows new dosing regimens of the present invention. Figure 17 depicts dosing regimens which include the use of an autoinjector fitted with a syringe of the present invention filled with 0.5mL, 1mL or 2mL 150mg/mL omalizumab formulation. However, the use of an autoinjector is optional and the dosing regimens depicted can also be performed using syringes of the invention when not fitted into a device and/or when fitted into a needle shield device.

Advantageously, the syringes of the present invention configured to administer 2mL of 150mg/mL omalziumab reduce the number of injections required as compared to current dosing regimens shown in Figure 2. For example, as shown in Figures 17, if a patient requires a dose of 300mg omalizumab, the dose can be administered using only one syringe of the invention containing 2mL 150mg/mL omalizumab formulation. If a patient requires a dose of 375mg omalizumab, the dose can be administered using two syringes - one syringe of the invention filled with 2mL 150mg/mL omalizumab formulation and one syringe filled with 0.5mL 150mg/mL omalizumab formulation. If a patient requires a dose of 450mg, the dose can be administered using two syringes - one syringe of the invention filled with 2mL 150mg/mL omalizumab formulation and one syringe filled with 1mL 150mg/mL omalizumab formulation. If a patient requires a dose of 525mg, the dose can be administered using three syringes - one syringe of the invention filled with 2mL 150mg/mL omalizumab formulation, one syringe filled with 0.5mL 150mg/mL omalizumab formulation and one syringe filled with 1mL 150mg/mL omalizumab formulation. If a patient requires a dose of 600mg, the dose can be administered using two syringes of the invention filled with 2mL 150mg/mL omalizumab formulation. The syringes filled with 0.5ml 150mg/mL omalizumab formulation and 1mL 150mg/mL omalizumab formulation can be syringes of the present invention or the current syringes.

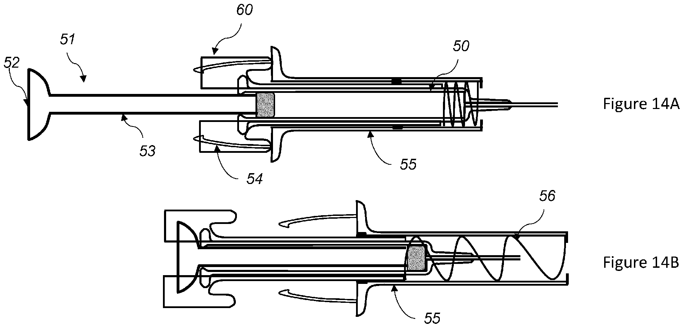

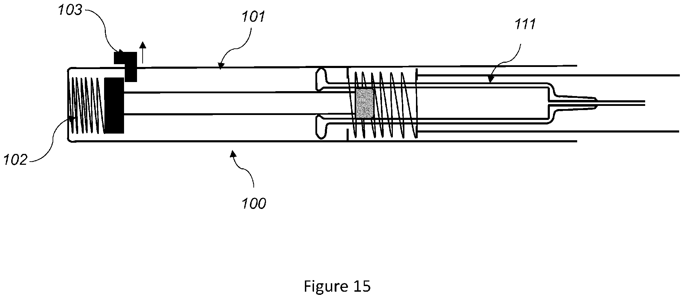

The syringes may be provided alone, in an autoinjector such as the autoinjector described with reference to Figure 15 and/or in a needle shield device such as the needle shield device described with reference to Figures 14A and 14B in this way, the present invention improves patient convenience and risk of injection site reaction and so improves patient compliance.

In addition, it is indicated that larger volumes of highly viscous drugs are more likely to be deposited deeper in the subcutaneous layer than smaller volumes and so are less likely to leak into the intradermal layer, resulting in site leakage and injection site reaction. Therefore, the provision of a method of treatment comprising administering 2mL 150mg/mL omalizumab formulation according to the invention will result less site leakage and reduced risk of injection site reaction than the currently methods of treatment comprising administering 0.5mL and 1mL 150mg/mL omalizumab.

Furthermore, as described previously, the syringes of the present invention filled with 0.5mL 150mg/mL omalizumab and 1mL 150mg/mL omalizumab formulation reduce the injection forces and/or time required as compared to current syringes, improving ease of use and making it more likely that a complete dose will be administered. Hence, the present invention improves ease of use, patient experience, patient adherence and outcomes using omalizumab.

Accordingly, there are also provided new dosing regimens which are similar to those illustrated in Figure 2 but which use the syringes of the present invention filled with 0.5mL 150mg/mL omalizumab and/or 1mL 150mg/mL omalizumab formulation. If a patient requires a dose of 75mg, the dose can be administered using one syringe of the present invention filled with 0.5ml 150mg/mL omalizumab formulation. If a patient requires a dose of 150mg, the dose can be administered using one syringe of the present invention filled with 1ml 150mg/mL omalizumab formulation. If a patient requires a dose of 225mg, the dose can be administered using one syringe filled with 0.5ml 150mg/mL omalizumab and one syringe filled with 1ml 150mg/mL omalizumab formulation; wherein one or both of the syringes is a syringe of the present invention. If a patient requires a dose of 300mg, the dose can be administered using two syringes filled with 1ml 150mg/mL omalizumab formulation; wherein one or both of the syringes is a syringe of the present invention. If a patient requires a dose of 375mg, the dose can be administered using three syringes - one syringe filled with 0.5ml 150mg/mL omalizumab and two syringes filled with 1ml 150mg/mL omalizumab formulation; wherein at least one of the syringes is a syringe of the present invention. If a patient requires a dose of 450mg, the dose can be administered using three syringes, all three syringes filled with 1ml 150mg/mL omalizumab formulation; wherein at least one of the syringes is a syringe of the present invention. If a patient requires a dose of 525mg, the dose can be administered using four syringes - one syringe filled with 0.5ml 150mg/mL omalizumab and three syringes filled with 1ml 150mg/mL omalizumab formulation; wherein at least one of the syringes is a syringe of the present invention. If a patient requires a dose of 600mg, the dose can be administered using four syringes, all four syringes filled with 1ml 150mg/mL omalizumab formulation; wherein at least one of the syringes is a syringe of the present invention.

In the new dosing regiments of the present invention, the syringes may be provided alone, in an autoinjector such as the autoinjector described with reference to Figure 15 and/or in a needle shield device such as the needle shield device described with reference to Figures 14A & 14B.

Accordingly, the invention provides the following new methods of treatment.

A method of treating a patient with one or more of: allergic asthma; food allergy; chronic rhinosinusitis with nasal polyps; chronic spontaneous urticaria; nasal polyps; moderate to severe persistent asthma in patients with a positive skin test or *in vitro* reactivity to a perennial aeroallergen and symptoms that are inadequately controlled with inhaled corticosteroids, the method comprising administering 225mg omalizumab formulation to the patient; wherein the method comprises one of:
a) performing a method of the invention wherein X is 0.5; and performing a method of the invention wherein X is 1;
b) performing a method of the invention wherein X is 0.5; and administering 1mL 150mg/mL omalizumab formulation subcutaneously by a syringe; and
c) performing a method of the invention wherein X is 1; and administering 0.5mL 150mg/mL omalizumab formulation subcutaneously by a syringe.

A method of treating a patient with one or more of: allergic asthma; food allergy; chronic rhinosinusitis with nasal polyps; chronic spontaneous urticaria; nasal polyps; moderate to severe persistent asthma in patients with a positive skin test or *in vitro* reactivity to a perennial aeroallergen and symptoms that are inadequately controlled with inhaled corticosteroids, the method comprising administering 300mg omalizumab formulation to the patient; wherein the method comprises administering 1mL 150mg/mL omalizumab formulation subcutaneously by a syringe and performing a method of the invention wherein X is 1.

A method of treating a patient with one or more of: allergic asthma; food allergy; chronic rhinosinusitis with nasal polyps; chronic spontaneous urticaria; nasal polyps; moderate to severe persistent asthma in patients with a positive skin test or *in vitro* reactivity to a perennial aeroallergen and symptoms that are inadequately controlled with inhaled corticosteroids, the method comprising administering 375mg omalizumab formulation to the patient; wherein the method comprises administering 0.5mL 150mg/mL omalizumab formulation subcutaneously by a syringe; and one of:
a) performing a method of the invention comprising administering 2 mL of 150mg/mL omalizumab formulation to the patient; and
b) performing a method of the invention wherein X is 1 and administering 1mL 150mg/mL omalizumab formulation subcutaneously by a syringe.

A method of treating a patient with one or more of: allergic asthma; food allergy; chronic rhinosinusitis with nasal polyps; chronic spontaneous urticaria; nasal polyps; moderate to severe persistent asthma in patients with a positive skin test or *in vitro* reactivity to a perennial aeroallergen and symptoms that are inadequately controlled with inhaled corticosteroids, the method comprising administering 450mg omalizumab formulation to the patient; wherein the method comprises administering 1mL 150mg/mL omalizumab formulation subcutaneously by a syringe; and one of:
a) performing a method of the invention comprising administering 2 mL of 150mg/mL omalizumab formulation to the patient; and
b) performing a method of the invention wherein X is 1 and administering 1mL 150mg/mL omalizumab formulation subcutaneously by a syringe.

A method of treating a patient with one or more of: allergic asthma; food allergy; chronic rhinosinusitis with nasal polyps; chronic spontaneous urticaria; nasal polyps; moderate to severe persistent asthma in patients with a positive skin test or *in vitro* reactivity to a perennial aeroallergen and symptoms that are inadequately controlled with inhaled corticosteroids, the method comprising administering 525mg omalizumab formulation to the patient; wherein the method comprises administering 1mL 150mg/mL omalizumab formulation subcutaneously by a syringe and administering 0.5mL 150mg/mL omalizumab formulation subcutaneously by a syringe; and one of:
a) performing a method of the invention comprising administering 2 mL of 150mg/mL omalizumab formulation to the patient; and
b) performing a method of the invention wherein X is 1 and administering 1mL 150mg/mL omalizumab formulation subcutaneously by a syringe.

A method of treating a patient with one or more of: allergic asthma; food allergy; chronic rhinosinusitis with nasal polyps; chronic spontaneous urticaria; nasal polyps; moderate to severe persistent asthma in patients with a positive skin test or *in vitro* reactivity to a perennial aeroallergen and symptoms that are inadequately controlled with inhaled corticosteroids, the method comprising administering 600mg omalizumab formulation to the patient; wherein the method comprises:
a) performing a method of the invention comprising administering 2 mL of 150mg/mL omalizumab formulation to the patient and performing a method of the invention comprising administering 2 mL of 150mg/mL omalizumab formulation to the patient; or
b) performing a method of the invention wherein X is 1, administering 1mL 150mg/mL omalizumab formulation subcutaneously by a syringe, administering 1mL 150mg/mL omalizumab formulation subcutaneously by a syringe and administering 1mL 150mg/mL omalizumab formulation subcutaneously by a syringe.

In any of these methods: (A) one of the steps requiring administering 0.5mL 150mg/mL omalizumab formulation subcutaneously by a syringe may comprise performing a method of the invention wherein X is 0.5; and/or (B) one of the steps requiring administering 1mL 150mg/mL omalizumab formulation subcutaneously by a syringe may comprise performing a method of the invention wherein X is 1.

### Further embodiments of the invention

In some embodiments of the present invention wherein X is 2, the syringe may be configured to expel the omalizumab formulation contained in the reservoir through the needle in one or more of:
a) less than 8.5 seconds when a constant force of 17N is applied to the stopper; and
b) less than 5 seconds when a constant force of 30N is applied to the stopper.

Furthermore, in some embodiments of the present invention wherein X is 2, the syringe may be configured to expel X mL of the omalizumab formulation contained in the reservoir through the needle in one or more of:
a) about 16 seconds to about 14 seconds when a constant force of 10N is applied to the stopper;
b) about 8.5 seconds to about 6 seconds when a constant force of 17N is applied to the stopper; and
c) about 5 seconds to about 4 seconds when a constant force of 30N is applied to the stopper.

In some embodiments of the present invention wherein X is 1, the syringe may be configured to expel X mL of 150mg/ml omalizumab formulation through the needle in one or more of:
a) less than 3 seconds when a constant force of 17N is applied to the stopper; and
b) less than 2 seconds when a constant force of 30N is applied to the stopper.

In some embodiments of the present invention wherein X is 1, the syringe may be configured to expel X mL of 150mg/ml omalizumab formulation through the needle in one or more of:
a) less than 7.5 seconds to about 4 seconds when a constant force of 10N is applied to the stopper;
b) less than 3.5 seconds to about 2 seconds when a constant force of 17N is applied to the stopper; and
c) less than 2 seconds to about 1 second when a constant force of 30N is applied to the stopper.

In some embodiments of the present invention wherein X is 1, the syringe may be configured to expel X mL of 150mg/ml omalizumab formulation through the needle in less than 5 seconds, less than 4.75 seconds, less than 4.5 seconds, less than 4.25 seconds, or in about 4 seconds when a constant force of 10N is applied to the stopper.

In some embodiments of the present invention wherein X is 1, the syringe may be configured to expel X mL of 150mg/ml omalizumab formulation through the needle in less than 5 seconds to about 4 seconds, less than 4.75 seconds to about 4 seconds, in less than 4.5 seconds to about 4 seconds, in less than 4.25 seconds to about 4 seconds or in about 4 seconds when a constant force of 10N is applied to the stopper.

In some embodiments of the present invention wherein X is 0.5, the syringe may be configured to expel X mL of 150mg/ml omalizumab formulation through the needle in one or more of:
a) less than 2 seconds when a constant force of 17N is applied to the stopper; and
b) less than 1 second when a constant force of 30N is applied to the stopper.

In some embodiments of the present invention wherein X is 0.5, the syringe may be configured to expel X mL of 150mg/ml omalizumab formulation through the needle in one or more of:
a) less than 4 seconds to about 2 seconds when a constant force of 10N is applied to the stopper;
b) less than 2 seconds to about 1 second when a constant force of 17N is applied to the stopper; and
c) less than 1 second to about 0.5 seconds when a constant force of 30N is applied to the stopper.

In some embodiments of the invention, the needle has a gauge of gauge 25 to gauge 29, preferably gauge 26 to gauge 28, most preferably gauge 27.

In some embodiments where the needle has a gauge of 25, the needle has one of:
a) a minimum internal diameter of 0.292mm, and
b) a minimum internal diameter of 0.232mm.

In some embodiments where the needle has a gauge of 26, the needle has one of:
a) a minimum internal diameter of 0.292mm; and
b) a minimum internal diameter of 0.232mm.

In some embodiments where the needle has a gauge of 27, the needle has one of:
a) a minimum internal diameter of 0.277mm;
b) a minimum internal diameter of 0.191 mm;
c) a minimum internal diameter of 0.184mm; and
d) a minimum internal diameter of 0.241mm.

In some embodiments where the needle has a gauge of 28, the needle has one of:
a) a minimum internal diameter of 0.133mm; and
b) a minimum internal diameter of 0.190mm.

In some embodiments where the needle has a gauge of 29, the needle has one of:
a) a minimum internal diameter of 0.265mm;
b) a minimum internal diameter of 0.240mm;
c) a minimum internal diameter of 0.190mm; and
d) a minimum internal diameter of 0.133mm, preferably wherein the needle has a minimum internal diameter of 0.240mm or 0.265mm.

In some embodiments, the needle can comprise 3 bevels on its distal surface or 5 bevels on its distal surface.

In some embodiments, the stopper may a fluoro-resin on the product contacting side or an ethylene tetrafluoroethylene (ETFE) barrier film lamination.

In some embodiments, the stopper may have a B2-40 UV cured lubrication coating or is lubricated with 1000cSt silicone oil.

In some embodiments, the syringe may have an internal coating of 0.4 ± 0.2mg or 0.7 ± 0.2mg of polydimethylsiloxane.

In some embodiments, the syringe may have a latex-free needle shield.

In some embodiments, the syringe may have round flange at a proximal end of the reservoir; in further embodiments, the round flange has a maximum diameter of 11 ± 0.25mm or 14.7 ± 0.25mm.

In embodiments where X is 1 or 0.5, the syringe may have a length of 54.0 ± 0.5mm and an internal diameter of 6.35mm ± 0.05mm.

In embodiments where X is 1 or 0.5, when the stopper is not inserted into the reservoir, it may have has one of:
a) a maximum external diameter of 6.67mm to 7.10mm and/or a length of 7.85 ± 0.4 mm; and
b) a maximum external diameter of 6.70 ± 0.15mm and/or a length of 7.85 ± 0.4 mm.

In some embodiments when X is 1 or 0.5, the stopper has at least one circumferential rib of outer diameter 6.60 ± 0.15 mm.

In some embodiments where X is 2, the syringe may have a length of 54.0 ± 0.5mm and an internal diameter of 8.65mm ± 0.05mm.

In some embodiments where X is 2, when the stopper is not inserted into the reservoir, the stopper may have a maximum external diameter 9.05 ± 0.15mm and/or length 7.70 ± 0.4 mm when not inserted into the reservoir.

In some embodiments where X is 2, the stopper may have least one circumferential rib of outer diameter 9.00 ± 0.15 mm.

In some embodiments, the apparent viscosity of the 150mg/mL omalizumab formulation at a shear rate of 200s⁻¹ is: 24.7 ± 0.5 mPa·s at 5.0 ± 0.2 °C, 15.3 ± 0.5 mPa·s at 15.0 ± 0.2 °C, 11.4 ± 0.5 mPa·s at 25.0 ± 0.2 °C, or 7.2 ± 1.2 mPa·s at 40.0 ± 0.2 °C.

While the above aspects related to a method of treatment comprising administering omalizumab formulation to the patient, wherein the omalizumab formulation is administered subcutaneously by a syringe, it will be understood that syringes of the present invention can be provided alone or in combination with a needle shield device or autoinjector. Accordingly, the present invention provides syringes containing 150mg/mL omalizumab formulation which are suitable to be used with autoinjectors in that the syringes of the present invention expel 150mg/mL omalizumab formulation in less time than prior art syringes when forces are applied to the stopper.

The present invention provides a syringe comprising:
a reservoir filled with 1mL of 150mg/mL omalizumab formulation,
a stopper, and
a needle; and
which is configured to expel 1mL of 150mg/ml omalizumab formulation through the needle in one or more of:
less than 8 seconds when a constant force of 10N is applied to the stopper;
less than 4 seconds when a constant force of 17N is applied to the stopper; and
less than 2 seconds when a constant force of 30N is applied to the stopper.

In some embodiments, the syringe is configured to expel 1mL of 150mg/ml omalizumab formulation through the needle in one or more of:
less than 8 seconds to about 4 seconds when a constant force of 10N is applied to the stopper;
less than 4 seconds to about 2 seconds when a constant force of 17N is applied to the stopper; and
less than 2 seconds to about 1 second when a constant force of 30N is applied to the stopper.

In some embodiments, the syringe is configured to expel 1mL of 150mg/ml omalizumab formulation through the needle in less than 5 seconds, less than 4.75 seconds, less than 4.5 seconds, less than 4.25 seconds, or in about 4 seconds when a constant force of 10N is applied to the stopper.

In some embodiments, the syringe is configured to expel 1mL of 150mg/ml omalizumab formulation through the needle in less than 7.5 seconds to about 4 seconds, in less than 5 seconds to about 4 seconds, in less than 4.75 seconds to about 4 seconds, in less than 4.5 seconds to about 4 seconds, in less than 4.25 seconds to about 4 seconds or in about 4 seconds when a constant force of 10N is applied to the stopper.

The present invention also provides a syringe for subcutaneous injection, the syringe comprising:
a reservoir filled with 0.5mL of 150mg/mL omalizumab formulation,
a stopper, and
a needle; and

the syringe being configured to expel the omalizumab formulation contained in the reservoir through the needle when a force is applied to the stopper,
the syringe being configured to expel 0.5mL of 150mg/ml omalizumab formulation through the needle in one or more of:
   less than 4 seconds when a constant force of 10N is applied to the stopper;
   less than 2 seconds when a constant force of 17N is applied to the stopper; and
   less than 1 second when a constant force of 30N is applied to the stopper.

In some embodiments, the syringe is configured to expel 0.5mL of 150mg/ml omalizumab formulation through the needle in one or more of:
less than 4 seconds to about 2 seconds when a constant force of 10N is applied to the stopper;
less than 2 seconds to about 1 second when a constant force of 17N is applied to the stopper; and
less than 1 seconds to about 0.5 seconds when a constant force of 30N is applied to the stopper.

The present invention also provides a syringe for subcutaneous injection, the syringe comprising a reservoir filled with 2mL of 150mg/mL omalizumab formulation,
a stopper, and
a needle,
the syringe being configured to expel 2mL of the omalizumab formulation contained in the reservoir through the needle when a force is applied to the stopper.

In some embodiments, the syringe is configured to expel 2mL of 150mg/mL omalizumab formulation out of the reservoir and through the needle in one or more of:
less than 16 seconds when a constant force of 10N is applied to the stopper;
less than 9 seconds when a constant force of 17N is applied to the stopper; and
less than 4 seconds when a constant force of 30N is applied to the stopper.

In some embodiments, the syringe is configured to expel 2mL of 150mg/mL omalizumab formulation out of the reservoir through the needle in one or more of:
less than 16 seconds to about 8 seconds when a constant force of 10N is applied to the stopper;
less than 9 seconds to about 4 seconds when a constant force of 17N is applied to the stopper; and
less than 4 seconds to about 2 seconds when a constant force of 30N is applied to the stopper.

### EXPERIMENTAL RESULTS

Figures 7 to 13 show experimental results carried out on the prior art syringe (described with reference to Figure 1) and five syringes of the present invention (described with reference to Figures 3 to 6). Table 1 summarises the syringes used in the experiments.

| **Syringe** | **A** | **B** | **C** | **D** | **E** | **F** |
|---|---|---|---|---|---|---|
| Described with reference to | Figure 3 | Figure 5 | Figure 5 | Figure 6 | Figure 4 | Figure 1 |
| Fill volume (omalizumab 150mg/m L) | 1mL | 0.5mL | 1mL | 2mL | 1mL | 1mL |

| | | | | | | |
|---|---|---|---|---|---|---|
| *Table 1 - Syringes used in the experiments.* | | | | | | |

### TIME TO EXPEL 150MGlML FORMULATION AT CONSTANT FORCE (Figure 7)

According to the present invention, the 150mg/mL omalizumab formulation is expelled from a syringe in less time than from prior art syringes when forces are applied to the stopper. Figure 7 shows the measured time to expel the 150mg/ml omalizumab formulation from syringes which form part of the present invention (Syringes A, C, D and E) and the prior art syringe (Syringe F) when a constant force of 10N, 17N or 30N is applied to the stopper.

A motorized test stand was used to apply a constant force to each syringe filled with the relevant volume of 150mg/ml omalizumab formulation and the time taken to move the stopper from its proximal position to its distal position, expelling the contents of the syringe into air was measured. The constant force was applied to a plunger which, in turn, applied force to the stopper. Ten syringe samples were tested for each constant force measurement and the mean was calculated. These tests were performed at room temperature. This test method can be used for determining the performance of syringes according to the invention.

The syringe samples were tested immediately after filling (known as time point 0 in the art).

Under the conditions in the experiment, the prior art syringe (Syringe F) is configured to expel 1mL of 150mg/mL omalizumab formulation through the needle in 8.53 ± 0.45 seconds when a constant force of 10N is applied to the stopper, 4.32 ± 0.20 seconds when a constant force of 17N is applied to the stopper and 2.14 ± 0.09 seconds when a constant force of 30N is applied to the stopper. In other words, the prior art syringe is configured to expel 150mg/mL omalizumab formulation through the needle in 0.85 ± 0.4 seconds per mL, per N of constant force applied.

Syringe A is configured to expel 1mL of 150mg/mL omalizumab formulation through the needle in 6.73 ± 0.51 seconds when a constant force of 10N is applied to the stopper, 3.27 ± 0.11 seconds when a constant force of 17N is applied to the stopper and 1.57 ± 0.10 seconds when a constant force of 30N is applied to the stopper. In other words, syringe A is configured to expel 150mg/mL omalizumab formulation through the needle in less than 0.67 ± 0.5 seconds per mL, per N of constant force applied.

Syringe E is configured to expel 1mL of 150mg/mL omalizumab formulation through the needle in 5.40 ± 0.23 seconds when a constant force of 10N is applied to the stopper, 2.84 ± 0.12 seconds when a constant force of 17N is applied to the stopper and 1.67 ± 0.15 seconds when a constant force of 30N is applied to the stopper. In other words, syringe E is configured to expel 150mg/mL omalizumab formulation through the needle in less than 0.54 ± 0.2 seconds per mL, per N of constant force applied.

Syringe C is configured to expel 1mL of 150mg/mL omalizumab formulation through the needle in 4.64 ± 0.22 seconds when a constant force of 10N is applied to the stopper, 2.54 ± 0.11 seconds when a constant force of 17N is applied to the stopper and 1.39 ± 0.04 seconds when a constant force of 30N is applied to the stopper. In other words, syringe C is configured to expel 150mg/mL omalizumab formulation through the needle in less than 0.4 ± 0.5 seconds per mL, per N of constant force applied.

Syringe D is configured to expel 2mL of 150mg/mL omalizumab formulation through the needle in 15.14 ± 0.51 seconds when a constant force of 10N is applied to the stopper, 8.30 ± 0.20 seconds when a constant force of 17N is applied to the stopper and 4.75 ± 0.23 seconds when a constant force of 30N is applied to the stopper. In other words, syringe D is configured to expel 150mg/mL omalizumab formulation through the needle in less than 0.75 ± 0.5 seconds per mL, per N of constant force applied.

Figure 7 demonstrates that syringes of the present invention (Syringes A, C and E) are configured to expel 1mL 150ml/mL omalizumab formation out of the reservoir and through the needle in less time than the syringe of the prior art (Syringe F).

Accordingly, the present invention provides X mL of 150mg/mL omalizumab formulation for use in a method of treatment of one or more of: allergic asthma; food allergy; chronic rhinosinusitis with nasal polyps; chronic spontaneous urticaria; nasal polyps; moderate to severe persistent asthma in patients with a positive skin test or in vitro reactivity to a perennial aeroallergen and symptoms that are inadequately controlled with inhaled corticosteroids, wherein X is 2, 1 or 0.5 and wherein the X mL 150mg/mL omalizumab formulation is administered subcutaneously by a syringe, the syringe comprising:
a reservoir filled with X mL of 150mg/mL omalizumab formulation, wherein the X mL 150mg/mL omalizumab formulation has 6000 or fewer particles of diameter ≥ 10µm and/or 600 or fewer particles of diameter ≥ 25µm; and the syringe further comprising:
a stopper,
a needle; and
the syringe being configured to expel the omalizumab formulation contained in the reservoir through the needle when a force is applied to the stopper,
wherein one or more of the following options is fulfilled when a constant force of 10N is applied to the stopper,
   a) when X is 2, the syringe is configured to expel the omalizumab formulation contained in the reservoir through the needle in less than 16 seconds;
   b) when X is 1, the syringe is configured to expel the omalizumab formulation contained in the reservoir through the needle in less than 7.5 seconds; and
   c) when X is 0.5, the syringe is configured to expel the omalizumab formulation contained in the reservoir through the needle in less than 4 seconds.

In addition, Figure 7 demonstrates that different components of the syringes of the present invention each contribute to the quicker injection time of the syringes of the present invention. The stopper 5 of Syringe F (prior art syringe) is the same as the stopper in Syringe A but the reservoir 2 and needle 3 of Syringe F are different to the reservoir 12 and needle 13 of Syringe A. Therefore, since Figure 7 shows that Syringe A is configured to expel 1ml 150ml/mL omalizumab formation out of the reservoir and through the needle in less time than Syringe F, the characteristics of the needle 13 and reservoir 12 of Syringe A both contribute to the quicker injection time of Syringe A. Similarly, the reservoir 12 and needle 13 of Syringe A are the same as the reservoir 32 and needle 33 of Syringe C but the stopper 15 of Syringe A is different to the stopper 35 of Syringe C. Therefore, since Figure 7 shows that Syringe C is configured to expel 1 ml 150ml/mL omalizumab formation out of the reservoir and through the needle in less time than Syringe A, the characteristics of the stopper 35 of Syringe C contribute to the faster injection time of Syringe C. Finally, the stopper 35 of Syringe C is the same as the stopper 25 in Syringe E but the reservoir 32 and needle 33 of Syringe C are different to the reservoir 22 and needle 23 of Syringe E. Since Figure 7 shows that Syringe E is configured to expel 1 ml 150ml/mL omalizumab formation out of the reservoir and through the needle in less time than Syringe C, the characteristics of the reservoir 22 and needle 23 of Syringe E contribute to the faster injection time of Syringe E.

In view of this, it will be understood that the syringes of the present invention (Syringes A, B, C, D and E) have been described by way of example only and modifications may be made whilst remaining within the scope of the invention. For example, it will be understood that stopper 15 can be combined with syringe reservoir 22 and needle 33, 23 or 13 to provide a syringe which forms part of the invention. In addition, any of the characteristics of the syringes, needles and stoppers of the present invention can be combined to provide a syringe according to the invention, including but not limited to needle gauges suitable for subcutaneous injection (i.e. gauge 23 to 32), needle minimum internal diameter, stopper lamination, stopper coating number of bevels on the needle distal surface, syringe flange, stopper maximum internal diameter and diameter of outer circumferential rib on the stopper.

Accordingly, the present invention provides XmL of 150mg/mL omalizumab formulation for use in a method of treatment of one or more of: allergic asthma; food allergy; chronic rhinosinusitis with nasal polyps; chronic spontaneous urticaria; nasal polyps; moderate to severe persistent asthma in patients with a positive skin test or in vitro reactivity to a perennial aeroallergen and symptoms that are inadequately controlled with inhaled corticosteroids, wherein X is 1 and wherein the X mL 150mg/mL omalizumab formulation is administered subcutaneously by a syringe, the syringe comprising:
a reservoir filled with X mL of 150mg/mL omalizumab formulation, wherein the X mL 150mg/mL omalizumab formulation has 6000 or fewer particles of diameter ≥ 10µm and/or 600 or fewer particles of diameter ≥ 25µm; and the syringe further comprising:
a stopper,
a needle; and
the syringe being configured to expel the omalizumab formulation contained in the reservoir through the needle when a force is applied to the stopper, wherein when a constant force of 10N is applied to the stopper, the syringe is configured to expel the omalizumab formulation contained in the reservoir through the needle in less than 7.5 seconds.

When X is 1, the needle can have a gauge of gauge 25 to gauge 29. When X is 1, the syringe can be configured to to expel the omalizumab formulation contained in the reservoir through the needle in less than 5 seconds, less than 4.75 seconds, less than 4.5 seconds, less than 4.25 seconds or in about 4 seconds when a constant force of 10N is applied to the stopper.

Furthermore, Figure 7 demonstrates that needles of gauge 27 are particularly advantageous in contributing to the quicker injection time of the syringes of the present invention as compared to the prior art because each of Syringes A, C and E comprise a needle of gauge 27 whereas Syringe F comprises a needle of gauge 26.

Accordingly, the present invention provides XmL of 150mg/mL omalizumab formulation for use in a method of treatment one or more of: allergic asthma; food allergy; chronic rhinosinusitis with nasal polyps; chronic spontaneous urticaria; nasal polyps; moderate to severe persistent asthma in patients with a positive skin test or in vitro reactivity to a perennial aeroallergen and symptoms that are inadequately controlled with inhaled corticosteroids, wherein X is 2, wherein the omalizumab formulation is administered subcutaneously by a syringe, the syringe comprising:
a reservoir filled with X mL of 150mg/mL omalizumab formulation, wherein the X mL 150mg/mL omalizumab formulation has 6000 or fewer particles of diameter ≥ 10µm and/or 600 or fewer particles of diameter ≥ 25µm; and the syringe further comprising:
a stopper, and
a needle having a gauge of 27; and
the syringe being configured to expel the omalizumab formulation contained in the reservoir through the needle when a force is applied to the stopper.

In some embodiments, the needle has one of:
a) a minimum internal diameter of 0.277mm;
b) a minimum internal diameter of 0.191mm;
c) a minimum internal diameter of 0.184mm; and
d) a minimum internal diameter of 0.241mm.

Finally, the results of Syringe D in Figure 7 demonstrates that the syringes of the present invention can be used to administer larger volumes of omalizumab than prior art syringes, i.e. larger than 0.5mL and 1mL omalizumab formulation.

### BREAK LOOSE FORCE (Figure 8)

As demonstrated with respect to Figure 8, the faster injection times of syringes of the present invention are, in part, because the syringes of the present invention require a lower break loose force than the prior art syringe. Figure 8 shows the measured break loose force required to move the stopper of the syringes of the present invention (Syringes A, C, D and E) and the prior art syringe (Syringe F) from an initial stationary point to a velocity of 190mm/min.

Syringes A, C, D, E and F were filled with 150mg/ml omalizumab formulation as required and placed into storage at 5°C ± 3 °C, 25°C ± 3 °C or 40°C ± 3 °C. The break loose force of syringe samples stored at 5°C ± 3 °C was tested at 2.5 and 6 months after filling. The break loose force of syringe samples stored at 25°C ± 3 °C was tested at time points after filling, and 1.5, 2.5 and 6 months after filling. The break loose force of syringe samples stored at 40°C ± 3 °C was tested 1.5 and 2.5 months after filling.

A motorized test stand was used to apply force to the stopper and measure the applied force and the instrument was adjusted to move the stopper at a velocity of 190mm/min.

Under the conditions in the experiment, the break loose force required to move the stopper of the prior art syringe (Syringe F) from an initial stationary point to a velocity of 190mm/min is one or more of:
about 4N to about 6N after storage at 5°C ± 3 °C for 2.5 months after filling;
about 4.5N to about 6N after storage at 5°C ± 3 °C for 6 months after filling.

Under the conditions in the experiment, the break loose force required to move the stopper of the prior art syringe (Syringe F) from an initial stationary point to a velocity of 190mm/min is one or more of:
about 4N to about 4.5N at 25°C ± 3 °C after filling;
about 5.5N to about 7N after storage at 25°C ± 3 °C for 1.5 months after filling;
about 4.5N to about 7.5N after storage at 25°C ± 3 °C for 2.5 months after filling; and/or
about 7N to about 10N after storage at 25°C ± 3 °C for 6 months after filling.

Under the conditions in the experiment, the break loose force required to move the stopper of the prior art syringe (Syringe F) from an initial stationary point to a velocity of 190mm/min is one or more of:
about 6N to about 10N after storage at 40°C ± 3 °C for 1.5 months after filling; and
about 8N to about 10N after storage at 25°C ± 3 °C for 2.5 months after filling.

Figure 8 demonstrates that the break loose force required to move the stopper of the syringes of the present invention from an initial stationary point to a velocity of 190mm/min is much lower and much less variable as compared to the prior art.

The break loose force required to move the stopper of the syringes of the present invention from an initial stationary point to a velocity of 190mm/min is one or more of:
About 2N to about 4N after storage at 5°C ± 3 °C for 2.5 months after filling;
about 2N to about 4N after storage at 5°C ± 3 °C for 6 months after filling.

The break loose force required to move the stopper of the syringes of the present invention from an initial stationary point to a velocity of 190mm/min is one or more of:
about 2N to about 3.5N at 25°C ± 3 °C after filling;
about 2N to about 4.5N after storage at 25°C ± 3 °C for 1.5 months after filling;
about 2.5N to about 5N after storage at 25°C ± 3 °C for 2.5 months after filling; and/or
about 2.5N to about 5.5N after storage at 25°C ± 3 °C for 6 months after filling.

The break loose force required to move the stopper of the syringes of the present invention from an initial stationary point to a velocity of 190mm/min is:
about 3N to about 4.5N after storage at 40°C ± 3 °C for 1.5 months after filling; and
about 3N to about 5N after storage at 40°C ± 3 °C for 2.5 months after filling.

### PURITY & STABILITY MEASURED BY ION EXCHANGE CHROMATOGRAPHY (IEC) (Fig 9)

As demonstrated with respect to Figure 9, the syringes of the present invention preserve the purity and stability of omalizumab comparably with the current syringes.

Figure 9 shows the percentage of the main omalizumab charge variant measured by ion exchange chromatography for the prior art syringe (Syringe F) and syringes of the present invention (Syringes A, B, C, D and E).

Syringe samples were filled with 150mg/mL omalizumab formulation and placed into storage at 5°C ± 3 °C or 25°C ± 3 °C. The percentage of the main omalizumab charge variant after storage at 5°C ± 3 °C was measured by IEC after filling, after 2.5 months in storage and after 6 months in storage. The percentage of the main variant after storage at 25°C ± 3 °C was measured by IEC after 1.5 months in storage and after 2.5 months in storage. Table 1 shows the test results.

**Table 2 - By and degradation products by IEC, Main variant %**

| Storage temperature | Storage duration (months) | Syringe A | Syringe B | Syringe C | Syringe D | Syringe E | Syringe F |
|---|---|---|---|---|---|---|---|
| 5°C ± 3 °C | 0 | 76.43 | 76.25 | 76.26 | 76.04 | 76.3 | 75.08 |
| | 2.5 | 77.45 | 77.74 | 77.59 | 77.32 | 77.13 | 76.26 |
| | 6 | 77.47 | 77.97 | 78.12 | 77.57 | 77.96 | 77.27 |
| 25°C ± 3 °C | 1.5 | 69.28 | 68.99 | 69.18 | 69.26 | 69.28 | 68.87 |
| | 2.5 | 68.18 | 68.14 | 68.12 | 68.13 | 68.14 | 68.23 |

As demonstrated in Table 2 and Figure 9, the syringes of the present invention (syringes A, B, C, D and E) preserve the purity and stability of omalizumab similarly to the prior art syringe (syringe F).

The syringes of the present invention contain at least 76% main omalizumab charge variant measured by ion exchange chromatography after filling with 150mg/mL omalizumab solution stored at 5°C ± 3°C. This demonstrates that syringes of the present invention preserve purity of omalizumab comparably with the prior art syringe (syringe F), which only contained 75.08% main omalizumab charge variant measured by ion exchange chromatography after filling with 1 mL 150mg/mL omalizumab solution stored at 5°C ± 3°C.

The syringes of the present invention also preserve the stability of omalizumab similarly to the prior art syringe. The syringes of the present invention contain at least 77% main omalizumab charge variant measured by ion exchange chromatography after being filled with 150mg/mL omalizumab solution and then stored at 5°C ± 3°C for 2.5 months, compared to 76% for the prior art syringe under the same conditions. Furthermore, the syringes of the present invention contain at least 69% main omalizumab charge variant after being filled with 150mg/mL omalizumab solution and then stored at 25°C ± 3°C for 1.5 months.

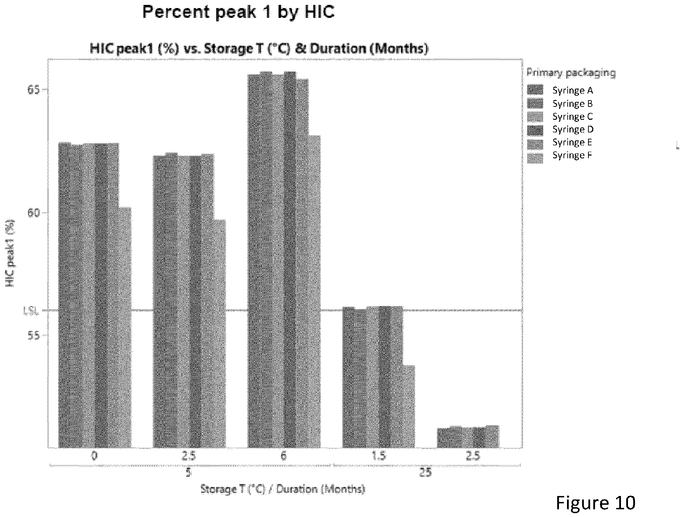

### PURITY & STABILITY MEASURED BY HYDROPHOBIC INTERACTION CHROMATOGRAPHY (HIC) (Figures 10 to 12)

As demonstrated with respect to Figure 10, the syringes of the present invention preserve the purity and stability of omalizumab similarly to the prior art syringe similarly to the current syringe.

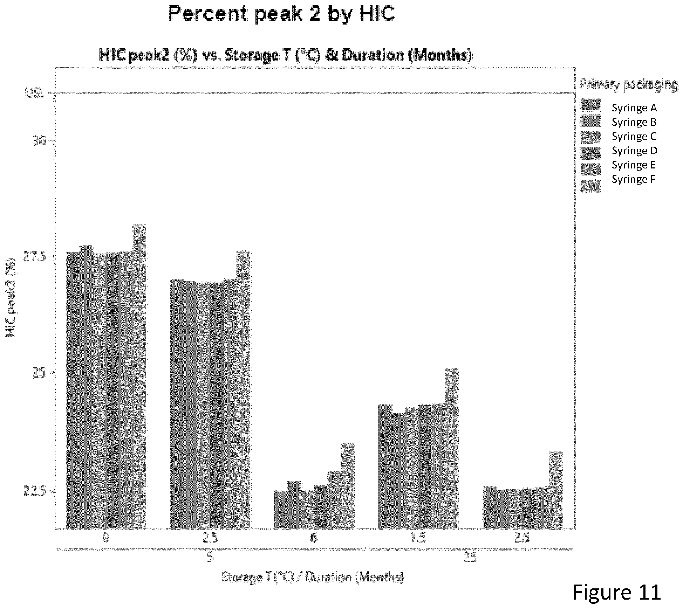

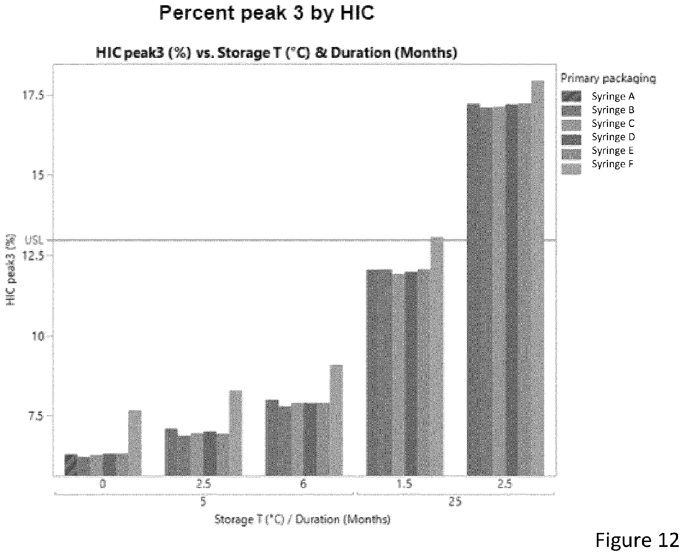

Figure 10 shows the proportion of the first HIC peak for the prior art syringe (Syringe F) and syringes of the present invention (Syringes A, B, C, D and E). Figure 11 shows the proportion of the second HIC peak for the prior art syringe and syringes present invention. Figure 12 shows the proportion of the third HIC peak for the prior art syringe and syringes of the present invention.

Syringe samples were filled with 150mg/mL omalizumab formulation and placed into storage at 5°C ± 3 °C or 25°C ± 3 °C. The proportion of the first, second and third HIC peaks of the syringes stored at 5°C ± 3 °C were measured after filling, after 2.5 months in storage and after 6 months in storage. The proportion of the first, second and third HIC peaks of the syringes stored at 25°C ± 3 °C were measured after 1.5 months in storage and after 2.5 months in storage. Tables 3, 4 and 5 shows the test results.

**Table 3 - By and degradation products by HIC, Peak 1 %**

| Storage temperature | Storage duration (months) | Syringe A | Syring e B | Syringe C | Syringe D | Syringe E | Syringe F |
|---|---|---|---|---|---|---|---|
| 5°C ± 3 °C | 0 | 62.8 | 62.7 | 62.8 | 62.8 | 62.8 | 60.2 |
| | 2.5 | 62.3 | 62.4 | 62.3 | 62.3 | 62.4 | 59.7 |
| | 6 | 65.6 | 65.7 | 65.6 | 65.7 | 65.4 | 63.1 |
| 25°C ± 3 °C | 1.5 | 56.1 | 56.0 | 56.2 | 56.2 | 56.2 | 53.8 |
| | 2.5 | 51.2 | 51.3 | 51.3 | 51.3 | 51.3 | 49.9 |

**Table 4 - By and degradation products by HIC, Peak 2 %**

| Storage temperature | Storage duration (months) | Syringe A | Syringe B | Syringe C | Syringe D | Syringe E | Syringe F |
|---|---|---|---|---|---|---|---|
| 5°C ± 3 °C | 0 | 27.6 | 27.7 | 27.6 | 27.6 | 27.6 | 28.2 |
| | 2.5 | 27.0 | 27.0 | 26.9 | 26.9 | 27.0 | 27.6 |
| | 6 | 22.5 | 22.7 | 22.5 | 22.6 | 22.9 | 23.5 |
| 25°C ± 3 °C | 1.5 | 24.3 | 24.2 | 24.3 | 24.3 | 24.3 | 25.1 |
| | 2.5 | 22.6 | 22.5 | 22.5 | 22.5 | 22.6 | 23.3 |

**Table 5 - By and degradation products by HIC, Peak 3 %**

| Storage temperature | Storage duration (months) | Syringe A | Syringe B | Syringe C | Syringe D | Syringe E | Syringe F |
|---|---|---|---|---|---|---|---|
| 5°C ± 3 °C | 0 | 6.3 | 6.2 | 6.3 | 6.3 | 6.3 | 7.7 |
| | 2.5 | 7.1 | 6.9 | 7.0 | 7.0 | 6.9 | 8.3 |
| | 6 | 8.0 | 7.8 | 7.9 | 7.9 | 7.9 | 9.1 |
| 25°C ± 3 °C | 1.5 | 12.1 | 12.1 | 11.9 | 12.0 | 12.1 | 13.1 |
| | 2.5 | 17.2 | 17.1 | 17.1 | 17.2 | 17.2 | 17.9 |

As demonstrated in Table 3 and Figure 10, the syringes of the present invention (Syringes A, B, C, D and E) preserve the purity and stability of omalizumab comparably with the prior art syringe (Syringe F).

The proportion of the first HIC peak is at least 62% for the syringes of the present invention after filling with 150mg/mL omalizumab solution stored at 5°C ± 3°C. This demonstrates that syringes of the present invention preserve purity of omalizumab comparably with the prior art syringe (syringe F), which has first HIC peak of 60.2% after filling with 150mg/mL omalizumab solution stored at 5°C ± 3°C.

The syringes of the present invention also preserve the stability of omalizumab. The proportion of the first HIC peak is at least 62% for the syringes of the present invention after being filled with 150mg/mL omalizumab solution and then stored at 5°C ± 3°C for 2.5 months, compared to 59% for the prior art syringe under the same conditions. The proportion of the first HIC peak is at least 56% for the syringes of the present invention after being filled with 150mg/mL omalizumab solution and then stored at 25°C ± 3°C for 1.5 months, compared to 53.8% for the prior art syringe under the same conditions. The proportion of the first HIC peak is at least 51% for the syringes of the present invention after being filled with 150mg/mL omalizumab solution and then stored at 25°C ± 3°C for 2.5 months, compared to 49.9% for the prior art syringe under the same conditions.

Accordingly, in some embodiments of the invention, the proportion of the first peak of the syringe measured by hydrophobic interaction chromatography is at least:
a) 62% after filling with 150mg/mL omalizumab solution stored at 5°C ± 3°C; and/or
b) 62% after being filled with 150mg/mL omalizumab solution and then stored at 5°C ± 3°C for 2.5 months.

In some embodiments of the invention, the proportion of the first peak of the syringe measured by hydrophobic interaction chromatography reduces by 0.4% or less after the syringe is stored at 5°C ± 3°C for 2.5 months.

In some embodiments of the invention, the proportion of the first peak of the syringe measured by hydrophobic interaction chromatography is at least:
a) 56% for the syringe after being filled with 150mg/mL omalizumab solution and then stored at 25°C ± 3°C for 1.5 months; and/or
b) 51% for the syringe after being filled with 150mg/mL omalizumab solution and then stored at 25°C ± 3°C for 2.5 months.

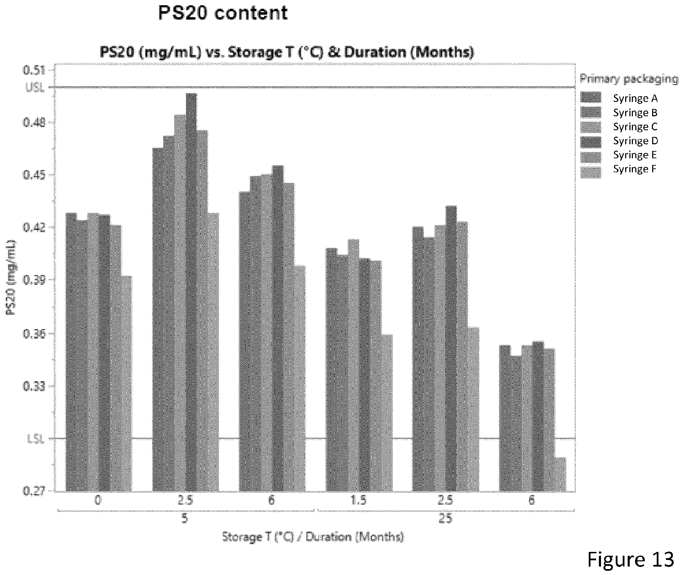

### POLYSORBATE 20 CONTENT (Figure 13)

As demonstrated with respect to Figure 13, the syringes of the present invention preserve the purity and stability of omalizumab similarly to current syringes.

Figure 13 shows the polysorbate 20 (PS20) content of the prior art syringe and syringes of the present invention.

Syringe samples were filled with 150mg/ml omalizumab formulation and placed into storage at 5°C ± 3 °C or 25°C ± 3 °C. The polysorbate 20 content was measured for each sample after filling, after 2.5 months in storage and after 6 months in storage. Table 6 shows the test results.

**Table 6 - excipient assay by HPLC PS20 (mg/mL)**

| Storage temperature | Storage duration (months) | Syringe A | Syringe B | Syringe C | Syringe D | Syringe E | Syringe F |
|---|---|---|---|---|---|---|---|
| 5°C ± 3 °C | 0 | 0.429 | 0.425 | 0.428 | 0.427 | 0.422 | 0.392 |
| | 2.5 | 0.462 | 0.472 | 0.484 | 0.496 | 0.475 | 0.428 |
| | 6 | 0.440 | 0.449 | 0.450 | 0.455 | 0.445 | 0.398 |
| 25°C ± 3 °C | 1.5 | 0.40- | 0.404 | 0.413 | 0.402 | 0.401 | 0.360 |
| | 2.5 | 0.420 | 0.414 | 0.421 | 0.432 | 0.423 | 0.363 |
| | 6 | 0.353 | 0.347 | 0.353 | 0.355 | 0.351 | 0.289 |

As demonstrated in Table 6 and Figure 13, the syringes of the present invention (syringes A, B, C, D and E preserve the purity and stability of omalizumab comparably with the prior art syringe (Syringe F).

The polysorbate 20 content of the syringes of the present invention is at least 0.4mg/mL after filling with 150mg/mL omalizumab solution stored at 5°C ± 3°C. This demonstrates that the syringes of the present invention preserve purity of the 150mg/mL omalizumab solution comparably with the prior art syringe (syringe F), which contains 0.392mg/mL polysorbate 20 content under the same conditions.

The syringes of the present invention also preserve the stability of omalizumab. The polysorbate 20 content of the syringes of the present invention is at least 0.4mg/mL for the syringes of the present invention after being filled with 150mg/mL omalizumab solution and then stored at 25°C ± 3°C for 1.5 months, compared to 0.360mg/mL for the prior art syringe under the same conditions. The polysorbate 20 content of the syringes of the present invention is at least 0.3mg/mL for the syringes of the present invention after being filled with 150mg/mL omalizumab solution and then stored at 25°C ± 3°C for 6 months, compared to 0.289mg/mL for the prior art syringe under the same conditions.

Accordingly, in some embodiments of the invention, the syringe contains at least 0.4 mg/mL polysorbate 20 after filling with 150mg/mL omalizumab solution stored at 5°C ± 3°C.

In some embodiments of the invention, the syringe contains:
a) at least 0.4mg/mL polysorbate 20 after being filled with 150mg/mL omalizumab solution and then stored at 25°C ± 3°C for 1.5 months; and/or
b) at least 0.3mg/mL polysorbate 20 after being filled with 150mg/mL omalizumab solution and then stored at 25°C ± 3°C for 6 months.

### NEEDLE SHIELD DEVICES

While the present application describes 150mg/mL omalizumab formulation for use in a method of treatment, wherein 150mg/mL omalizumab formulation is administered subcutaneously by a syringe, it will be understood that the syringes of the present invention can be provided alone, or in combination with a needle shield device.

Figures 14A and 14B show schematic diagrams of a prefilled syringe 50 in accordance with the present invention with a needle shield device 60. Figure 14A shows a pre-use configuration and Figure 14B shows a used configuration.

A plunger 51 is provided comprising a plunger rod 53 and thumb pad 52. A user can apply manual force to the thumb pad 22 in a distal direction to manually expel the fluid contained in the reservoir through the needle. As the plunger reaches the end of its distal movement, it can unlatch needle shield retaining arms 54 provided within the needle shield device, releasing a needle sleeve 55. When the needle shield device is removed from an injection site, the needle sleeve 15 can extend under the bias of the needle shield spring 56 to cover the needle, as shown in Figure 14B.

Accordingly, in some embodiments of the present invention, the syringe is provided in needle shield device comprising a needle sleeve and a plunger; and wherein a manual force can be applied to the plunger to expel the 150mg/mL omalizumab formulation and unlatch needle sleeve so that the needle sleeve can cover the needle after injection.

### AUTOINJECTORS

While the present application describes 150mg/mL omalizumab formulation for use in a method of treatment, wherein 150mg/mL omalizumab formulation is administered subcutaneously by a syringe, it will be understood that the syringes of the present invention can be provided alone, or in combination with an autoinjector. Accordingly, in some embodiments of the invention, the syringe is provided in an autoinjector.

Figure 15 is a schematic diagram of a prefilled syringe 111 within an autoinjector 100. The autoinjector comprises a housing 101 configured to contain the syringe 111 and a drive mechanism 102 configured to apply force to the stopper in a distal direction and expel fluid from the syringe through the needle. A trigger mechanism 103 is provided to actuate the drive mechanism. In the example shown in Figure 15, the trigger 103 is moved in the direction of the arrow, away from the prefilled syringe.

The syringe comprises a reservoir filled with 150mg/mL omalizumab formulation, a stopper and a needle. As will be appreciated, the autoinjector 100 can also be combined with any of the syringes of the invention - further details of the syringes of the invention and the fill volume are described with reference to Figures 3 to 6.

The drive mechanism 102 is configured to apply a force to the stopper to drive the stopper through the reservoir towards the needle to expel the 150mg/mL omalizumab formulation out of the reservoir and through the needle.

A trigger mechanism 103 is provided which is movable from a hold position, in which the drive mechanism is held in a rest position, to a release position, in which the drive mechanism is released, causing the drive mechanism to drive the stopper through the reservoir towards the needle to expel the 150mg/mL omalizumab formulation out of the reservoir and through the needle in about 1 second to about 17 seconds.

In the embodiment shown in Figure 15, the drive mechanism is configured to expel the 150mg/mL omalizumab formulation out of the reservoir and through the needle in about 1 to 17 seconds by applying a mean average force of at least 10N to the stopper over the time period in which the 150mg/mL omalizumab formulation is expelled. In some embodiments, the drive mechanism is configured to apply a mean average force of at least 11N to the stopper, at least 12N, at least 13N, at least 14N, at least 15N, at least 16N, at least 17N, at least 18N, at least 19N, at least 20N, at least 21N, at least 22N, at least 23N, at least 24N, at least 25N, at least 26N, at least 27N, at least 28N, at least 29N, at least 30N, at least 31N, at least 32N, at least 33N, at least 34N, at least 35N, at least 36N, at least 37N, at least 38N, at least 39N or at least 40N.

In the embodiment shown in Figure 15, the drive mechanism comprises a helical spring configured to apply a mean force of 25N to the stopper. The mean force can be calculated by determining the force applied to the stopper at the beginning of the injection (when the drive mechanism is released) and the force applied to the stopper at the end of the injection (when the stopper has been driven through the reservoir until it is engaged with the distal end of reservoir and cannot be driven any further) and calculating the mean of the two forces. As the skilled person will understand, Hooke's law can be used to calculate the force applied by a given spring at a given extension.

While the drive mechanism of the autoinjector shown in Figure 15 comprises a helical drive spring, the invention is not limited to such a drive mechanism. Other types of drive mechanism can be used to apply a mean average force of at least 10N to the stopper to expel the 150mg/mL omalizumab formulation out of the reservoir and through the needle in a time period of less than 17s in accordance with the present invention. For example, a drive mechanism comprising a torsion spring or an electromechanical drive can be used.

The autoinjector further comprises an autoinjector cap (not shown). The autoinjector cap is configured to permanently engage with the syringe needle shield (described in more detail with reference to Figures 3 to 6) and removably engage with the autoinjector housing. When the autoinjector cap is disengaged from the housing, it removes the needle shield from the syringe, thus allowing access to the needle.

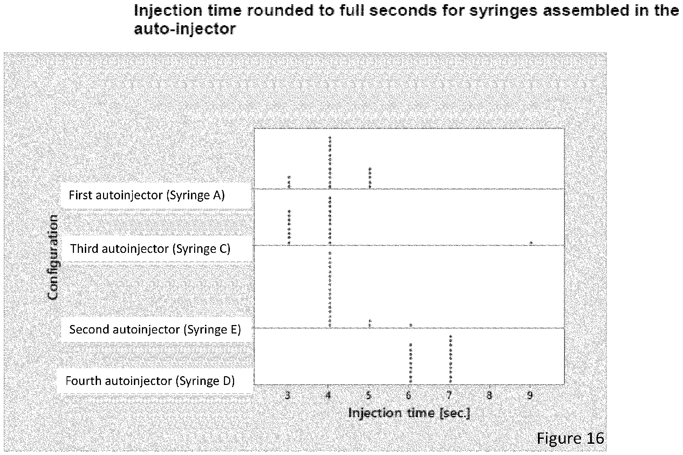

### TIME TO EXPEL OMALIZUMAB FORMULATION USING AN AUTOINJECTOR COMBINED WITH SYRINGES OF THE PRESENT INVENTION (Figure 16)

Figure 16 shows the measured time to expel the 150mg/mL omalizumab formulation out of the reservoir and through the needle using an autoinjector when combined with syringes of the invention.

Four autoinjectors as shown and described with reference to Figure 15 were tested. The first autoinjector was fitted with Syringe A. The second autoinjector was fitted with Syringe E. The third autoinjector was fitted with Syringe C. The fourth autoinjector was fitted Syringe D. The trigger mechanism of each autoinjector was moved from the hold position to the release position and the time for the stopper to be driven through the reservoir until it engaged with the distal end of reservoir was measured. The test was repeated 20 times for each autoinjector embodiment.

Each autoinjector tested expelled the 150mg/mL omalizumab formulation out of the reservoir and through the needle in about 1 second to about 17 seconds. The drive mechanism of each autoinjector that was tested included a helical spring configured to apply a mean average force of 25N to the stopper.

The first autoinjector expelled 1 ml of 150mg/mL omalizumab solution out of the reservoir and through the needle in about 3 to 5 seconds.

The second autoinjector expelled 1 ml of 150mg/mL omalizumab solution out of the reservoir and through the needle in about 4 to 6 seconds.

The third autoinjector expelled 1 ml of 150mg/mL omalizumab solution out of the reservoir and through the needle in about 3 to 4 seconds. In one test, the third autoinjector expelled 1 ml of 150mg/mL omalizumab solution out of the reservoir and through the needle in about 9 seconds - this test result appears to be an error.

The fourth autoinjector embodiment expelled 2 ml of 150mg/mL omalizumab solution out of the reservoir and through the needle in about 6 to 7 seconds.

As demonstrated in Figure 16, the present invention further provides an autoinjector filled with 150mg/mL omalizumab formulation which is configured to needle to expel the 150mg/mL omalizumab formulation out of the reservoir and through the needle in a time period of less than 17s. This time period of less than 17s is acceptable to patients.

While Figure 16 only shows test results of autoinjectors with syringes filled with 1mL or 2mL 150mg/mL omalizumab formulation, it will be understood that autoinjectors with syringes filled with 0.5mL 150mg/mL omalizumab formulation can also be provided in accordance with the invention. In addition, Figure 16 does not show test results of an autoinjector fitted with the prior art syringe (syringe F) because the prior art syringe is not suitable for use with autoinjectors. The prior art syringe is not suitable for use with autoinjectors because it cannot reliably withstand the high injection forces required to administer the omalizumab within an acceptable time period.

Accordingly, in one aspect, there is described an autoinjector comprising:
a syringe according to the invention, e.g. a syringe comprising a reservoir filled with 0.5mL, 1mL or 2mL of 150mg/mL omalizumab formulation, a stopper, and a needle;
a drive mechanism configured to apply force to the stopper to drive the stopper through the reservoir towards the needle to expel the 150mg/mL omalizumab formulation out of the reservoir and through the needle;
a housing configured to contain the syringe and the drive mechanism; and
a trigger mechanism movable from a hold position, in which the drive mechanism is held in a rest position in which it does not apply a force to the stopper, to a release position, in which the drive mechanism is released, causing the drive mechanism to apply a force to the stopper to drive the stopper through the reservoir towards the needle to expel the 150mg/mL omalizumab formulation out of the reservoir and through the needle in a time period of less than 17s.

The syringe can be filled with 0.5mL of 150mg/mL omalizumab formulation and wherein the drive mechanism is configured to expel the 0.5mL of 150mg/mL out of the reservoir and through the needle in a time period of about 1 second to about 17 seconds, about 1 second to about 16 seconds, about 1 second to about 15 seconds, about 1 second to about 14 seconds, about 1 second to about 13 seconds, about 1 second to about 12 seconds, about 1 second to about 11 seconds, about 1 second to about 10 seconds, about 1 second to about 9 seconds, about 1 second to about 8 seconds, about 1 second to about 7 seconds, about 1 seconds to about 6 seconds, about 1 second to about 5 seconds, about 1 second to about 4 seconds, about 1 second to about 2 seconds; or about 1 second.

The syringe can be filled with 1mL of 150mg/mL omalizumab formulation, and the drive mechanism is configured to expel the 1mL of 150mg/mL out of the reservoir and through the needle in a time period of about 3 seconds to about 17 seconds, about 3 seconds to about 16 seconds, about 3 seconds to about 15 seconds, about 3 seconds to about 14 seconds, about 3 seconds to about 13 seconds, about 3 seconds to about 12 seconds, about 3 seconds to about 11 seconds, about 3 seconds to about 10 seconds, about 3 seconds to about 9 seconds, about 3 seconds to about 8 seconds, about 3 seconds to about 7 seconds, about 3 seconds to about 6 seconds, about 3 seconds to about 5 seconds, about 3 seconds to about 4 seconds; or about 3 seconds.

The syringe be filled with 2mL of 150mg/mL omalizumab formulation, and the drive is configured to expel the 2mL of 150mg/mL omalizumab formulation out of the reservoir and through the needle in a time period of about 6 seconds to 17 seconds, about 6 seconds to about 16 seconds, about 6 seconds to about 15 seconds, about 6 seconds to about 14 seconds, about 6 seconds to about 13 seconds, about 6 seconds to about 12 seconds, about 6 seconds to about 11 seconds, about 6 seconds to about 10 seconds, about 6 seconds to about 9 seconds, about 6 seconds to about 8 seconds, about 6 seconds to about 7 seconds, or about 6 seconds

The drive mechanism can be configured to apply a mean average force of at least 10N to the stopper over the time period in which the 150mg/mL omalizumab formulation is expelled. It can be configured to apply a mean average force to the stopper over the time period in which the 150mg/mL omalizumab formulation is expelled of at least 11N, at least 12N, at least 13N, at least 14N, at least 15N, at least 16N, at least 17N, at least 18N, at least 19N, at least 20N, at least 21N, at least 22N, at least 23N, at least 24N, at least 25N, at least 26N, at least 27N, at least 28N, at least 29N, at least 30N, at least 31N, at least 32N, at least 33N, at least 34N, at least 35N, at least 36N, at least 37N, at least 38N, at least 39N or at least 40N.

The drive mechanism can comprise a helical spring, a torsion spring and/or an electromechanical drive.

In some aspects, the syringe is filled with 0.5mL of 150mg/mL omalizumab formulation and the drive is configured to expel the 0.5mL of 150mg/mL out of the reservoir and through the needle in a time period of about 1 second to about 17 seconds, about 1 second to about 16 seconds, about 1 second to about 15 seconds, about 1 second to about 14 seconds, about 1 second to about 13 seconds, about 1 second to about 12 seconds, about 1 second to about 11 seconds, about 1 second to about 10 seconds, about 1 second to about 9 seconds, about 1 second to about 8 seconds, about 1 second to about 7 seconds, about 1 seconds to about 6 seconds, about 1 second to about 5 seconds, about 1 second to about 4 seconds, about 1 second to about 2 seconds; or about 1 second.

In some aspects, the syringe is filled with 1mL of 150mg/mL omalizumab formulation, and the drive is configured to expel the 1mL of 150mg/mL out of the reservoir and through the needle in a time period of about 3 seconds to about 17 seconds, about 3 seconds to about 16 seconds, about 3 seconds to about 15 seconds, about 3 seconds to about 14 seconds, about 3 seconds to about 13 seconds, about 3 seconds to about 12 seconds, about 3 seconds to about 11 seconds, about 3 seconds to about 10 seconds, about 3 seconds to about 9 seconds, about 3 seconds to about 8 seconds, about 3 seconds to about 7 seconds, about 3 seconds to about 6 seconds, about 3 seconds to about 5 seconds, about 3 seconds to about 4 seconds; or about 3 seconds. In some aspects, the syringe is filled with 2mL of 150mg/mL omalizumab formulation and the drive is configured to expel the 2mL of 150mg/mL omalizumab formulation out of the reservoir and through the needle in a time period of about 6 seconds to 17 seconds, about 6 seconds to about 16 seconds; optionally wherein the time period is one of: about 6 seconds to about 15 seconds, about 6 seconds to about 14 seconds, about 6 seconds to about 13 seconds, about 6 seconds to about 12 seconds, about 6 seconds to about 11 seconds, about 6 seconds to about 10 seconds, about 6 seconds to about 9 seconds, about 6 seconds to about 8 seconds, about 6 seconds to about 7 seconds, or about 6 seconds.

There is also provided a kit comprising a syringe according to the present invention and optionally: an autoinjector, a needle shield device, and/or instructions for administration.

In one aspect of the invention, there is provided, for the first time, injection of highly viscous 150mg/mL omalizumab formulation by an autoinjector. In particular, one aspect provides for injection of highly viscous 150mg/mL omalizumab formulation in a time period of 1 second to 17 seconds - this time period is acceptable to patients and so improves ease of use and patient compliance.

Furthermore, as shown in Figure 16, the autoinjectors comprise a syringe filled with 1mL of 150mg/mL omalizumab formulation and comprising a drive mechanism configured to expel the 1mL of 150mg/mL out of the reservoir and through the needle in a time period of about 3 seconds to 6 seconds. This injection time is much faster than the injection time of comparable self-administered antibody formulations such as the 30mg/1mL Fasenra (benralizumab) autoinjector (injection time period of up to 15 seconds) and the 100mg/1mL Nucala (mepolizumab) autoinjector (injection time period of up to 15 seconds).

This faster injection time facilitates the injection of high doses of omalizumab which have not yet been available to patients, such as 2mL of 300mg/2mL omalizumab formulation in a single syringe. As shown in Figure 16, the present invention provides an autoinjector comprising a syringe filled with 2mL of 150mg/mL omalizumab formulation and comprising a drive mechanism configured to expel the 2mL of 150mg/mL out of the reservoir and through the needle in a time period of about 6 seconds to 7 seconds. This injection time is much faster than the injection time of comparable antibody formulations of similar volume; for example the 300mg/mL Dupixent (dupilumab) autoinjector (injection time of up to 20 seconds).

As demonstrated with respect to Figure 7, the faster injection times of autoinjectors of the present invention are, in part, because the syringes of the present invention expel 150mg/mL omalizumab formulation at faster times than prior art syringes when constant forces are applied are applied to the stopper. Furthermore, as demonstrated with respect to Figure 8, the faster injection times of the autoinjectors of the present invention are, in part, because the syringes of the present invention require a lower break loose force than the prior art syringe.

Surprisingly, as demonstrated with respect to Figures 9 to 13, the syringes of the present invention better preserve the purity and stability of omalizumab. The better preserved purity and stability of omalizumab also contributes to the faster injection time of the syringes and autoinjectors of the present invention because it reduces aggregation and so limits increases in fluid viscosity.

### BIOEQUIVALENCE DATA

It has been demonstrated that new methods of treatment comprising administering omalizumab described in this application are bioequivalent to the previously approved methods of treatment comprising administering omalizumab, see Sangana et al. Bioequialvence between a new omalizumab prefilled syringe with an autoinjector or with a needle safety device compared with the current prefilled syringe: a randomized controlled trial in healthy volunteers, Clinical Pharmacology. 2024, 0(0) 1-10.

Sangana et al. demonstrated the bioequivalence of omalizumab between:
(1) the new 300mg/2mL prefilled syringe in an autoinjector (new PFS-AI), i.e. autoinjector 100 described with reference to Figure 15 fitted with syringe D described with reference to Figure 6 and two of the current 150mg/1mL prefilled syringe in a needle shield device (current PFS-NSD), i.e. needle shield device 60 described with reference to Figures 14A and 14B fitted with syringe F described with reference to Figure 1;
(2) the new 300mg/2mL prefilled syringe in a needle shield device (new PFS-NSD), i.e. needle shield device 60 described with reference to Figures 14A and 14B fitted with syringe D described with reference to Figure 6 and two of the current 150mg/1mL prefilled syringes in a needle shield device (current PFS-NSD), i.e. needle shield device 60 described with reference to Figures 14A and 14B fitted with syringe F described with reference to Figure 1; and
(3) the new 300mg/2mL prefilled syringe in an autoinjector (new PFS-AI), i.e. autoinjector 100 described with reference to Figure 15 fitted with syringe D described with reference to Figure 6 and the new 300mg/2mL prefilled syringe in a needle shield device (new PFS-NSD), i.e. needle shield device 60 described with reference to Figures 14A and 14B fitted with syringe D described with reference to Figure 6.

For the bioequivalence comparison, natural log-transformed pharmacokinetic parameters (Cₘₐₓ, AUCₗₐₛₜ, AUC_{inf}) were analyzed by an analysis of covariance (ANCOVA) model with treatment group, body area of injection and body weight strata as fixed effects and baseline IgE as a covariate. Cₘₐₓ is the maximum observed serum concentration, AUCₗₐₛₜ is the area under the serum concentration-time curve calculated to last quantifiable concentration point and AUC_{inf}. is the area under the serum concentration-time curve extrapolated to infinity. Based on the ANCOVA model, point estimates and the corresponding 95% and 90% confidence limits were calculated for the difference between each new omalizumab configuration (new PFS-AI, new PFS-NSD) and the control intervention (current PFS-NSD). These point estimates and confidence limits were exponentiated to obtain the point estimates with 95% and 90% confidence intervals (Cis) for the ratios of geometric means on the original scale. Bioequiavalence claims were made for each comparison if and only if Cₘₐₓ, AUCₗₐₛₜ, and AUC_{inf} were declared equivalent (ie, the Cis for all ratios were contained between 80% and 125%) based on the stepwise Bonferroni-Holm method for multiple comparisons.

The statistical comparisons of omalizumab serum pharmacokinetic parameter values for the new PFS-AI, new PFS-NSD and current PFS-NSD are summarised below.

| Parameter | (1) New PFS-AI vs. Current PFS-NSD | (2) New PFS-NSD vs. Current PFS-NSD | (3) New PFS-AI vs. New PFS-NSD |
|---|---|---|---|
| | GMR (%) | GMR (%) | GMR (%) |
| | [95% Cl]^{a} | [95% Cl]^{b} | [90% Cl]^{c} |
| Cₘₐₓ (µg/mL) | 108.5 [99.6-118.1] | 100.6 [92.3-109.6] | 107.8 [100.4-115.8] |
| AUCₗₐₛₗ (µg*h/mL) | 109.3 [99.7-119.9] | 101.6 [92.5-111.6] | 107.6 [99.6-116.3] |
| AUC_{inf} (µg*h/mL) | 110.0 [100.1-120.9] | 102.7 [93.3-113.0] | 107.2 [99.0-116.0] |

| | | | |
|---|---|---|---|
| ^{a}In the comparison of each new configuration versus the current configuration bioequivalence was established based on the 95% Cis according to the Bonferroni-Holm procedure ^{b}In the comparison of the new configurations to each other bioequivalence was established based on the 90% Cis. | | | |

AUC_{inf}, area under curve from time 0 to infinity; AUCₗₐₛₜ, area under curve from time 0 to last measurable concentration sampling time; Cl, confidence interval; Cₘₐₓ, maximum (peak) observed drug concentration after single dose administration; GMR, geometric mean ratio; PFS-Al, prefilled syringe assembled with an autoinjector; PFS-NSD, prefilled syringe with a needle safety device.

Formal bioequivalence was established between: (1) new PFS-AI and current PFS-NSD (95% CI or 90% Cl, based on Bonferroni-Holm procedure); (2) new PFS-NSD and current PFS-NSD (95% CI or 90% Cl, based on Bonferroni-Holm procedure); and (3) new PFS-AI and new PFS-NSD (90% CI); with the CI for each treatment contrast contained within the 80%-125% range for Cₘₐₓ, AUCₗₐₛₜ, and AUC_{inf} following a single dose of omalizumab.

### OTHER CLAUSES OF THE INVENTION

1. X mL of 150mg/mL omalizumab formulation for use in a method of treatment of one or more of: allergic asthma; food allergy; chronic rhinosinusitis with nasal polyps; chronic spontaneous urticaria; nasal polyps; moderate to severe persistent asthma in patients with a positive skin test or *in vitro* reactivity to a perennial aeroallergen and symptoms that are inadequately controlled with inhaled corticosteroids, wherein X is 2, and wherein the X mL 150mg/mL omalizumab formulation is administered subcutaneously by a syringe, the syringe comprising:
   a reservoir filled with X mL of 150mg/mL omalizumab formulation, wherein the X mL of 150mg/mL omalizumab formulation has 6000 or fewer particles of diameter ≥ 10µm and/or 600 or fewer particles of diameter ≥ 25µm; and the syringe further comprising:
   a stopper, and
   a needle,
   the syringe being configured to expel X mL of the omalizumab formulation contained in the reservoir through the needle when a force is applied to the stopper.
2. X mL of 150mg/mL omalizumab formulation for use in a method of treatment of one or more of: allergic asthma; food allergy; chronic rhinosinusitis with nasal polyps; chronic spontaneous urticaria; nasal polyps; moderate to severe persistent asthma in patients with a positive skin test or *in vitro* reactivity to a perennial aeroallergen and symptoms that are inadequately controlled with inhaled corticosteroids, wherein X is 2, 1 or 0.5 and wherein the X mL 150mg/mL omalizumab formulation is administered subcutaneously by a syringe, the syringe comprising:
   a reservoir filled with X mL of 150mg/mL omalizumab formulation, wherein the XmL 150mg/mL omalizumab formulation has 6000 or fewer particles of diameter≥ 10µm and/or 600 or fewer particles of diameter ≥ 25µm; and the syringe further comprising:
   a stopper, and
   a needle having a gauge of 27; and
   the syringe being configured to expel the omalizumab formulation contained in the reservoir through the needle when a force is applied to the stopper.
3. X mL of 150mg/mL omalizumab formulation for use according to clause 2, wherein the needle has one of:
   a) a minimum internal diameter of 0.277mm;
   b) a minimum internal diameter of 0.191mm;
   c) a minimum internal diameter of 0.184mm; and
   d) a minimum internal diameter of 0.241mm.
4. X mL of 150mg/mL omalizumab formulation for use in a method of treatment of one or more of: allergic asthma; food allergy; chronic rhinosinusitis with nasal polyps; chronic spontaneous urticaria; nasal polyps; moderate to severe persistent asthma in patients with a positive skin test or *in vitro* reactivity to a perennial aeroallergen and symptoms that are inadequately controlled with inhaled corticosteroids, wherein X is 2, 1 or 0.5 and wherein the X mL 150mg/mL omalizumab formulation is administered subcutaneously by a syringe, the syringe comprising:
   a reservoir filled with X mL of 150mg/mL omalizumab formulation, wherein the XmL 150mg/mL omalizumab formulation has 6000 or fewer particles of diameter ≥ 10µm and/or 600 or fewer particles of diameter ≥ 25µm; and the syringe further comprising:
   a stopper, and
   a needle; and
   the syringe being configured to expel the omalizumab formulation contained in the reservoir through the needle when a force is applied to the stopper,
   the syringe being configured to expel 150mg/mL omalizumab formulation through the needle in less than 0.8 seconds per mL per N of constant force applied to the stopper.
5. X mL of 150mg/mL omalizumab formulation for use according to any one of claims 1 to 4 wherein X is 2, wherein the syringe is configured to expel the omalizumab formulation contained in the reservoir through the needle in less than 0.75 ± 0.5 seconds per mL, per N of constant force applied.
6. X mL of 150mg/mL omalizumab formulation for use according to any one of clauses 1 to 5 wherein X is 2, wherein the syringe is configured to expel the omalizumab formulation contained in the reservoir through the needle in one or more of:
   a) less than 16 seconds when a constant force of 10N is applied to the stopper;
   b) less than 8.5 seconds when a constant force of 17N is applied to the stopper; and
   c) less than 5 seconds when a constant force of 30N is applied to the stopper.
7. XmL of 150mg/mL omalizumab formulation for use according to clause 6, wherein the syringe is configured to expel X mL of the omalizumab formulation contained in the reservoir through the needle in one or more of:
   a) about 16 seconds to about 14 seconds when a constant force of 10N is applied to the stopper;
   b) about 8.5 seconds to about 6 seconds when a constant force of 17N is applied to the stopper; and
   c) about 5 seconds to about 4 seconds when a constant force of 30N is applied to the stopper.
8. X mL of 150mg/mL omalizumab formulation for use according to any one of clauses 2 to 4, wherein X is 1 or 0.5, wherein the syringe is configured to expel X mL of 150mg/ml omalizumab formulation through the needle in less than 0.67 ± 0.5 seconds per mL, per N of constant force applied, optionally less than 0.54 ± 0.2 seconds per mL, per N of constant force applied, further optionally less than 0.4 ± 0.5 seconds per mL, per N of constant force applied.
9. X mL of 150mg/mL omalizumab formulation for use according to any one of clauses 2 to 4 and 8, wherein X is 1, and wherein the syringe is to expel X mL of 150mg/ml omalizumab formulation through the needle in one or more of:
   a) less than 7.5 seconds when a constant force of 10N is applied to the stopper;
   b) less than 3 seconds when a constant force of 17N is applied to the stopper; and
   c) less than 2 seconds when a constant force of 30N is applied to the stopper.
10. X mL of 150mg/mL omalizumab formulation for use according to clause 9, wherein the syringe is configured to expel X mL of 150mg/ml omalizumab formulation through the needle in one or more of:
   a) less than 7.5 seconds to about 4 seconds when a constant force of 10N is applied to the stopper;
   b) less than 3.5 seconds to about 2 seconds when a constant force of 17N is applied to the stopper; and
   c) less than 2 seconds to about 1 second when a constant force of 30N is applied to the stopper.
11. X mL of 150mg/mL omalizumab formulation for use according to any one of clauses 2 to 4 and 8, wherein X is 0.5, wherein the syringe is to expel XmL of 150mg/ml omalizumab formulation through the needle in one or more of:
   a) less than 4 seconds when a constant force of 10N is applied to the stopper;
   b) less than 2 seconds when a constant force of 17N is applied to the stopper; and
   c) less than 1 second when a constant force of 30N is applied to the stopper.
12. X mL of 150mg/mL omalizumab formulation for use according to clause 11, wherein the syringe is to expel X mL of 150mg/ml omalizumab formulation through the needle in one or more of:
   a) less than 4 seconds to about 2 seconds when a constant force of 10N is applied to the stopper;
   b) less than 2 seconds to about 1 second when a constant force of 17N is applied to the stopper; and
   c) less than 1 second to about 0.5 seconds when a constant force of 30N is applied to the stopper.
13. X mL of 150mg/mL omalizumab formulation for use according to any one of clauses 1 to 12, wherein the syringe contains:
   a) at least 76% main omalizumab charge variant measured by ion exchange chromatography after being filled with 150mg/mL omalizumab solution stored at 5°C ± 1°C; and/or
   b) at least 77% main omalizumab charge variant measured by ion exchange chromatography after being filled with 150mg/mL omalizumab solution and then stored at 5°C ± 1°C for at 2.5 months.
14. X mL of 150mg/mL omalizumab formulation for use according to any one of clauses 1 to 13, wherein the percentage of main omalizumab charge variant in the syringe measured by ion exchange chromatography reduces by 2% or less after the syringe is stored at 5°C ± 1°C for at 2.5 months.
15. X mL of 150mg/mL omalizumab formulation for use according to any one of clauses 1 to 14, wherein the proportion of the first peak of the syringe measured by hydrophobic interaction chromatography is at least:
   a) 62% after filling with 150mg/mL omalizumab solution stored at 5°C ± 1°C; and/or
   b) 62% after being filled with 150mg/mL omalizumab solution and then stored at 5°C ± 1°C for 2.5 months.
16. X mL of 150mg/mL omalizumab formulation for use according to any one of clauses 1 to 15, wherein the proportion of the first peak of the syringe measured by hydrophobic interaction chromatography reduces by 0.4% or less after the syringe is stored at 5°C ± 1°C for at 2.5 months.
17. X mL of 150mg/mL omalizumab formulation for use according to any one of clauses 1 to 16, wherein the syringe contains at least 0.4 mg/mL polysorbate 20 after filling with 150mg/mL omalizumab solution stored at 5°C ± 1°C.
18. X mL of 150mg/mL omalizumab formulation for use according to any one of clauses 1 to 12, wherein the syringe contains at least 69% main omalizumab charge variant measured by ion exchange chromatography after being filled with 150mg/mL omalizumab solution and then stored at 25°C ± 1°C for 1.5 months.
19. X mL of 150mg/mL omalizumab formulation for use according to any one of clauses 1 to 12 and 18, wherein the proportion of the first peak of the syringe measured by hydrophobic interaction chromatography is at least:
   a) 56% for the syringe after being filled with 150mg/mL omalizumab solution and then stored at 25°C ± 1°C for 1.5 months; and/or
   b) 51 % for the syringe after being filled with 150mg/mL omalizumab solution and then stored at 25°C ± 1°C for 2.5 months.
20. X mL of 150mg/mL omalizumab formulation for use according to any one of clauses 1 to 12, 18 and 19, wherein the syringe contains:
   a) at least 0.4mg/mL polysorbate 20 after being filled with 150mg/mL omalizumab solution and then stored at 25°C ± 1°C for 1.5 months; and/or
   b) at least 0.3mg/mL polysorbate 20 after being filled with 150mg/mL omalizumab solution and then stored at 25°C ± 1°C for 6 months.
21. X mL of 150mg/mL omalizumab formulation for use according to any one of clauses 1 and 4 to 20 when not dependent on clause 2 or clause 3, wherein the needle has one of: gauge 23, gauge 24, gauge 25, gauge 26, gauge 27, gauge 28, gauge 29, gauge 30, gauge 31 and gauge 32.
22. X mL of 150mg/mL omalizumab formulation for use according to any one of clauses 1 and 4 to 20 when not dependent on clause 2 or clause 3, wherein the needle has one of:
   a) gauge 23 defining a minimum internal diameter of 0.370mm,
   b) gauge 23 defining a minimum internal diameter of 0.317mm,
   c) gauge 24 defining a minimum internal diameter of 0.343mm
   d) gauge 24 defining a minimum internal diameter of 0.280mm
   e) gauge 25 defining a minimum internal diameter of 0.292mm,
   f) gauge 25 defining a minimum internal diameter of 0.232mm,
   g) gauge 26 defining a minimum internal diameter of 0.292mm,
   h) gauge 26 defining a minimum internal diameter of 0.232mm,
   i) gauge 27 defining a minimum internal diameter of 0.277mm;
   j) gauge 27 defining a minimum internal diameter of 0.191 mm;
   k) gauge 27 defining a minimum internal diameter of 0.184mm;
   l) gauge 27 defining a minimum internal diameter of 0.241 mm;
   m) gauge 28 defining a minimum internal diameter of 0.133mm,
   n) gauge 28 defining a minimum internal diameter of 0.190mm,
   o) gauge 29 defining a minimum internal diameter of 0.265mm
   p) gauge 29 defining a minimum internal diameter of 0.240mm,
   q) gauge 29 defining a minimum internal diameter of 0.190mm,
   r) gauge 29 defining a minimum internal diameter of 0.133mm,
   s) gauge 30 defining a minimum internal diameter of 0.240mm,
   t) gauge 30 defining a minimum internal diameter of 0.190mm,
   u) gauge 30 defining a minimum internal diameter of 0.165mm,
   v) gauge 30 defining a minimum internal diameter of 0.133mm,
   w) gauge 31 defining a minimum internal diameter of 0.176mm,
   x) gauge 31 defining a minimum internal diameter of 0.146mm,
   y) gauge 31 defining a minimum internal diameter of 0.125mm,
   z) gauge 31 defining a minimum internal diameter of 0.114mm,
   aa) gauge 32 defining a minimum internal diameter of 0.146mm,
   bb) gauge 32 defining a minimum internal diameter of 0.125mm,
   cc) gauge 32 defining a minimum internal diameter of 0.105mm, and
   dd) gauge 32 defining a minimum internal diameter of 0.089mm.
23. X mL of 150mg/mL omalizumab formulation for use according to any one of clauses 1 to 22, wherein the stopper has a fluoro-resin on the product contacting side or an ethylene tetrafluoroethylene (ETFE) barrier film lamination.
24. X mL of 150mg/mL omalizumab formulation for use according to any one of clauses 1 to 23, wherein the stopper has a B2-40 UV cured lubrication coating or is lubricated with 1000cSt silicone oil..
25. X mL of 150mg/mL omalizumab formulation for use according to any one of clauses 1 to 24, wherein the syringe has an internal coating of 0.4 ± 0.2mg of polydimethylsiloxane.
26. X mL of 150mg/mL omalizumab formulation for use according to any one of clauses 1 to 25, wherein the needle has 3 bevels on its distal surface or 5 bevels on its distal surface.
27. X mL of 150mg/mL omalizumab formulation for use according to any one of clauses 1 to 26, wherein the apparent viscosity of the 150mg/mL omalizumab formulation at a shear rate of 200s⁻¹ is: 24.7 ± 0.5 mPa·s at 5.0 ± 0.2 °C, 15.3 ± 0.5 mPa·s at 15.0 ± 0.2 °C, 11.4 ± 0.5 mPa·s at 25.0 ± 0.2 °C, or 7.2 ± 1.2 mPa·s at 40.0 ± 0.2 °C.
28. X mL of 150mg/mL omalizumab formulation for use according to any one of clauses 1 to 27, wherein the syringe has a latex-free needle shield.
29. X mL of 150mg/mL omalizumab formulation for use according to any one of clauses 1 to 28, wherein the syringe has a round flange; optionally wherein the round flange has a maximum diameter of 11 ± 0.25mm or 14.7 ± 0.25mm.
30. X mL of 150mg/mL omalizumab formulation for use according to any one of clauses 2 to 4 and 8 to 29 when X is 1 or 0.5, wherein the syringe has a length of 54.0 ± 0.5mm and an internal diameter of 6.35mm ± 0.05mm.
31. X mL of 150mg/mL omalizumab formulation for use according to any one of clauses 2 to 4 and 8 to 30 when X is 1 or 0.5, wherein when the stopper is not inserted into the reservoir, it has one of:
   a) a maximum external diameter of 6.67mm to 7.10mm and/or a length of 7.85 ± 0.4 mm; and
   b) a maximum external diameter of 6.70 ± 0.15mm and/or a length of 7.85 ± 0.4 mm.
32. X mL of 150mg/mL omalizumab formulation for use according to any one of clauses 2 to 4 and 8 to 31 when X is 1 or 0.5, wherein the stopper has at least one rib of outer diameter 6.60 ± 0.15 mm.
33. X mL of 150mg/mL omalizumab formulation for use according to any one of clauses 1 to 7 and 13 to 29 when X is 2, wherein the syringe has a length of 54.0 ± 0.5mm and an internal diameter of 8.65mm ± 0.05mm.
34. X mL of 150mg/mL omalizumab formulation for use according to any one of clauses 1 to 7, 13 to 29 and 33, when X is 2, wherein the stopper has a maximum external diameter 9.05 ± 0.15mm and/or length 7.70 ± 0.4 mm when not inserted into the reservoir.
35. X mL of 150mg/mL omalizumab formulation for use according to any one of clauses 1 to 7, 13 to 29 and 33 to 34, when X is 2, wherein the stopper has at least one rib of outer diameter 9.00 ± 0.15 mm.
36. X mL of 150mg/mL omalizumab formulation for use according to any one of clauses 1 to 17 and 21 to 35 when not dependent on clauses 18 to 20, wherein a break loose force configured to move the stopper of the present invention from an initial stationary point to a velocity of 190mm/min is one or more of:
   a) about 2N to about 4N after storage at 5°C ± 1 °C for 2.5 months;
   b) about 2N to about 4N after storage at 5°C ± 1 °C for 6 months.
37. X mL of 150mg/mL omalizumab formulation for use according to any one of clauses 1 to 12 and 18 to 35 when not dependent on clauses 13 to 17, wherein a break loose force configured to move the stopper of the present invention from an initial stationary point to a velocity of 190mm/min is one or more of:
   a) about 2N to about 3.5N at 25°C ± 1 °C after filling;
   b) about 2N to about 4.5N after storage at 25°C ± 1 °C for 1.5 months;
   c) about 2.5N to about 5N after storage at 25°C ± 1 °C for 2.5 months; and/or
   d) about 2.5N to about 5.5N after storage at 25°C ± 1 °C for 6 months.
38. X mL of 150mg/mL omalizumab formulation for use according to any one of clauses 1 to 12 and 21 to 35 when not dependent on clauses 13 to 20, wherein a break loose force configured to move the stopper of the present invention from an initial stationary point to a velocity of 190mm/min is one or more of:
   a) about 3N to about 4.5N after storage at 40°C ± 1 °C for 1.5 months; and
   b) about 3N to about 5N after storage at 40°C ± 1 °C for 2.5 months.
39. X mL of 150mg/mL omalizumab formulation for use according to any one of clauses 1 to 38, wherein the syringe is provided in needle shield device comprising a needle sleeve and a plunger; and wherein
   a manual force can be applied to the plunger to expel the 150mg/mL omalizumab formulation and unlatch needle sleeve so that the needle sleeve can cover the needle after injection.
40. X mL of 150mg/mL omalizumab formulation for use according to any one of clauses 1 to 39, wherein the syringe is manufactured by a method comprising:
   aseptic filling of the reservoir with the 150mg/mL omalizumab formulation;
   and stoppering the reservoir with the stopper.
41. X mL of 150mg/mL omalizumab formulation for use according to clause 40, wherein the aseptic filling is performed by a peristaltic pump or stainless steel piston pump.
42. X mL of 150mg/mL omalizumab formulation for use according to clause 40 or 41 wherein, prior to the aseptic filling, the reservoir and needle are sterilised by ethylene oxide gas treatment, e-beam and/or steam sterilisation by autoclave.
43. A syringe comprising:
   a reservoir filled with X mL of 150mg/mL omalizumab formulation, wherein the X mL of 150mg/mL omalizumab formulation has 6000 or fewer particles of diameter≥ 10µm and/or 600 or fewer particles of diameter ≥ 25µm; and the syringe further comprising:
   a stopper, and
   a needle,
   the syringe being configured to expel X mL of the omalizumab formulation contained in the reservoir through the needle when a force is applied to the stopper.
44. A syringe comprising:
   a reservoir filled with X mL of 150mg/mL omalizumab formulation, wherein the XmL 150mg/mL omalizumab formulation has 6000 or fewer particles of diameter≥ 10µm and/or 600 or fewer particles of diameter ≥ 25µm; and the syringe further comprising:
   a stopper, and
   a needle having a gauge of 27; and
   the syringe being configured to expel the omalizumab formulation contained in the reservoir through the needle when a force is applied to the stopper.
45. A syringe comprising:
   a reservoir filled with X mL of 150mg/mL omalizumab formulation, wherein the X mL of 150mg/mL omalizumab formulation has 6000 or fewer particles of diameter ≥ 10µm and/or 600 or fewer particles of diameter ≥ 25µm, wherein X is 2; and the syringe further comprising:
   a stopper, and
   a needle,
   the syringe being configured to expel X mL of the omalizumab formulation contained in the reservoir through the needle when a force is applied to the stopper.
46. A syringe comprising:
   a reservoir filled with X mL of 150mg/mL omalizumab formulation, wherein the XmL 150mg/mL omalizumab formulation has 6000 or fewer particles of diameter ≥ 10µm and/or 600 or fewer particles of diameter ≥ 25µm, wherein X is 0.5, 1 or 2; and the syringe further comprising:
   a stopper, and
   a needle having a gauge of 27; and
   the syringe being configured to expel the omalizumab formulation contained in the reservoir through the needle when a force is applied to the stopper.
47. The syringe of clause 46 the needle has one of:
   a) a minimum internal diameter of 0.277mm;
   b) a minimum internal diameter of 0.191mm;
   c) a minimum internal diameter of 0.184mm; and
   d) a minimum internal diameter of 0.241mm.
48. A syringe comprising:
   a reservoir filled with X mL of 150mg/mL omalizumab formulation, wherein the XmL 150mg/mL omalizumab formulation has 6000 or fewer particles of diameter≥ 10µm and/or 600 or fewer particles of diameter ≥ 25µm, wherein X is 0.5, 1 or 2; and the syringe further comprising:
   a stopper, and
   a needle; and
   the syringe being configured to expel the omalizumab formulation contained in the reservoir through the needle when a force is applied to the stopper,
   the syringe being configured to expel 150mg/mL omalizumab formulation through the needle in less than 0.8 seconds per mL per N of constant force applied to the stopper.
49. The syringe according to any one of clauses 45 to 48 wherein X is 2, wherein the syringe is configured to expel the omalizumab formulation contained in the reservoir through the needle in less than 0.75 ± 0.5 seconds per mL, per N of constant force applied.
50. The syringe according to any one of clauses 45 to 49 wherein X is 2, wherein the syringe is configured to expel the omalizumab formulation contained in the reservoir through the needle in one or more of:
   a) less than 16 seconds when a constant force of 10N is applied to the stopper;
   b) less than 8.5 seconds when a constant force of 17N is applied to the stopper; and
   c) less than 5 seconds when a constant force of 30N is applied to the stopper.
51. The syringe according to clause 50, wherein the syringe is configured to expel X mL of the omalizumab formulation contained in the reservoir through the needle in one or more of:
   a) about 16 seconds to about 14 seconds when a constant force of 10N is applied to the stopper;
   b) about 8.5 seconds to about 6 seconds when a constant force of 17N is applied to the stopper; and
   c) about 5 seconds to about 4 seconds when a constant force of 30N is applied to the stopper.
52. The syringe according to any one of clauses 46 to 48, wherein X is 1 or 0.5, wherein the syringe is configured to expel X mL of 150mg/ml omalizumab formulation through the needle in less than 0.67 ± 0.5 seconds per mL, per N of constant force applied, optionally less than 0.54 ± 0.2 seconds per mL, per N of constant force applied, further optionally less than 0.4 ± 0.5 seconds per mL, per N of constant force applied.
53. The syringe according to any one of clauses 46 to 48 and 52, wherein X is 1, and wherein the syringe is to expel X mL of 150mg/ml omalizumab formulation through the needle in one or more of:
   a) less than 7.5 seconds when a constant force of 10N is applied to the stopper;
   b) less than 3 seconds when a constant force of 17N is applied to the stopper; and
   c) less than 2 seconds when a constant force of 30N is applied to the stopper.
54. The syringe according to clause 53, wherein the syringe is configured to expel X mL of 150mg/ml omalizumab formulation through the needle in one or more of:
   a) less than 7.5 seconds to about 4 seconds when a constant force of 10N is applied to the stopper;
   b) less than 3.5 seconds to about 2 seconds when a constant force of 17N is applied to the stopper; and
   c) less than 2 seconds to about 1 second when a constant force of 30N is applied to the stopper.
55. The syringe according to any one of clauses 46 to 48 and 52, wherein X is 0.5, wherein the syringe is to expel X mL of 150mg/ml omalizumab formulation through the needle in one or more of:
   a) less than 4 seconds when a constant force of 10N is applied to the stopper;
   b) less than 2 seconds when a constant force of 17N is applied to the stopper; and
   c) less than 1 second when a constant force of 30N is applied to the stopper.
56. The syringe according to clause 55, wherein the syringe is to expel XmL of 150mg/ml omalizumab formulation through the needle in one or more of:
   a) less than 4 seconds to about 2 seconds when a constant force of 10N is applied to the stopper;
   b) less than 2 seconds to about 1 second when a constant force of 17N is applied to the stopper; and
   c) less than 1 second to about 0.5 seconds when a constant force of 30N is applied to the stopper.
57. The syringe according to any one of clauses 45 to 56, wherein the syringe contains:
   a) at least 76% main omalizumab charge variant measured by ion exchange chromatography after being filled with 150mg/mL omalizumab solution stored at 5°C ± 1°C; and/or
   b) at least 77% main omalizumab charge variant measured by ion exchange chromatography after being filled with 150mg/mL omalizumab solution and then stored at 5°C ± 1°C for at 2.5 months.
58. The syringe according to any one of clauses 45 to 56, wherein the percentage of main omalizumab charge variant in the syringe measured by ion exchange chromatography reduces by 2% or less after the syringe is stored at 5°C ± 1°C for at 2.5 months.
59. The syringe according to any one of clauses 45 to 58, wherein the proportion of the first peak of the syringe measured by hydrophobic interaction chromatography is at least:
   a) 62% after filling with 150mg/mL omalizumab solution stored at 5°C ± 1°C; and/or
   b) 62% after being filled with 150mg/mL omalizumab solution and then stored at 5°C ± 1°C for 2.5 months.
60. The syringe according to any one of clauses 45 to 59, wherein the proportion of the first peak of the syringe measured by hydrophobic interaction chromatography reduces by 0.4% or less after the syringe is stored at 5°C ± 1°C for at 2.5 months.
61. The syringe according to any one of clauses 45 to 60, wherein the syringe contains at least 0.4 mg/mL polysorbate 20 after filling with 150mg/mL omalizumab solution stored at 5°C ± 1°C.
62. The syringe according to any one of clauses 45 to 56, wherein the syringe contains at least 69% main omalizumab charge variant measured by ion exchange chromatography after being filled with 150mg/mL omalizumab solution and then stored at 25°C ± 1°C for 1.5 months.
63. The syringe according to any one of clauses 45 to 56 and 62, wherein the proportion of the first peak of the syringe measured by hydrophobic interaction chromatography is at least:
   a) 56% for the syringe after being filled with 150mg/mL omalizumab solution and then stored at 25°C ± 1°C for 1.5 months; and/or
   b) 51% for the syringe after being filled with 150mg/mL omalizumab solution and then stored at 25°C ± 1°C for 2.5 months.
64. The syringe according to any one of clauses 45 to 56, 62 and 63, wherein the syringe contains:
   a) at least 0.4mg/mL polysorbate 20 after being filled with 150mg/mL omalizumab solution and then stored at 25°C ± 1°C for 1.5 months; and/or
   b) at least 0.3mg/mL polysorbate 20 after being filled with 150mg/mL omalizumab solution and then stored at 25°C ± 1°C for 6 months.
65. The syringe according to any one of clauses 45 and 48 to 64 when not dependent on clause 2 or clause 3, wherein the needle has one of: gauge 23, gauge 24, gauge 25, gauge 26, gauge 27, gauge 28, gauge 29, gauge 30, gauge 31 and gauge 32.
66. The syringe according to any one of clauses 45 and 48 to 64 when not dependent on clause 2 or clause 3, wherein the needle has one of:
   a) gauge 23 defining a minimum internal diameter of 0.370mm,
   b) gauge 23 defining a minimum internal diameter of 0.317mm,
   c) gauge 24 defining a minimum internal diameter of 0.343mm
   d) gauge 24 defining a minimum internal diameter of 0.280mm
   e) gauge 25 defining a minimum internal diameter of 0.292mm,
   f) gauge 25 defining a minimum internal diameter of 0.232mm,
   g) gauge 26 defining a minimum internal diameter of 0.292mm,
   h) gauge 26 defining a minimum internal diameter of 0.232mm,
   i) gauge 27 defining a minimum internal diameter of 0.277mm;
   j) gauge 27 defining a minimum internal diameter of 0.191mm;
   k) gauge 27 defining a minimum internal diameter of 0.184mm;
   l) gauge 27 defining a minimum internal diameter of 0.241mm;
   m) gauge 28 defining a minimum internal diameter of 0.133mm,
   n) gauge 28 defining a minimum internal diameter of 0.190mm,
   o) gauge 29 defining a minimum internal diameter of 0.265mm
   p) gauge 29 defining a minimum internal diameter of 0.240mm,
   q) gauge 29 defining a minimum internal diameter of 0.190mm,
   r) gauge 29 defining a minimum internal diameter of 0.133mm,
   s) gauge 30 defining a minimum internal diameter of 0.240mm,
   t) gauge 30 defining a minimum internal diameter of 0.190mm,
   u) gauge 30 defining a minimum internal diameter of 0.165mm,
   v) gauge 30 defining a minimum internal diameter of 0.133mm,
   w) gauge 31 defining a minimum internal diameter of 0.176mm,
   x) gauge 31 defining a minimum internal diameter of 0.146mm,
   y) gauge 31 defining a minimum internal diameter of 0.125mm,
   z) gauge 31 defining a minimum internal diameter of 0.114mm,
   aa) gauge 32 defining a minimum internal diameter of 0.146mm,
   bb) gauge 32 defining a minimum internal diameter of 0.125mm,
   cc) gauge 32 defining a minimum internal diameter of 0.105mm, and
   dd) gauge 32 defining a minimum internal diameter of 0.089mm.
67. The syringe according to any one of clauses 45 to 66, wherein the stopper has a fluoro-resin on the product contacting side or an ethylene tetrafluoroethylene (ETFE) barrier film lamination.
68. The syringe according to any one of clauses 45 to 67, wherein the stopper has a B2-40 UV cured lubrication coating or is lubricated with 1000cSt silicone oil.
69. The syringe according to any one of clauses 45 to 68, wherein the syringe has an internal coating of 0.4 ± 0.2mg of polydimethylsiloxane.
70. The syringe according to any one of clauses 45 to 69, wherein the needle has 3 bevels on its distal surface or 5 bevels on its distal surface.
71. The syringe according to any one of clauses 45 to 70, wherein the apparent viscosity of the 150mg/mL omalizumab formulation at a shear rate of 200s⁻¹ is: 24.7 ± 0.5 mPa·s at 5.0 ± 0.2 °C, 15.3 ± 0.5 mPa·s at 15.0 ± 0.2 °C, 11.4 ± 0.5 mPa·s at 25.0 ± 0.2 °C, or 7.2 ± 1.2 mPa·s at 40.0 ± 0.2 °C.
72. The syringe according to any one of clauses 45 to 71, wherein the syringe has a latex-free needle shield.
73. The syringe according to any one of clauses 45 to 72, wherein the syringe has a round flange; optionally wherein the round flange has a maximum diameter of 11 ± 0.25mm or 14.7 ± 0.25mm.
74. The syringe according to any one of clauses 46 to 48 and 52 to 73 when X is 1 or 0.5, wherein the syringe has a length of 54.0 ± 0.5mm and an internal diameter of 6.35mm ± 0.05mm.
75. The syringe according to any one of clauses 46 to 48 and 52 to 74 when X is 1 or 0.5, wherein when the stopper is not inserted into the reservoir, it has one of:
   a) a maximum external diameter of 6.67mm to 7.10mm and/or a length of 7.85 ± 0.4 mm; and
   b) a maximum external diameter of 6.70 ± 0.15mm and/or a length of 7.85 ± 0.4 mm.
76. The syringe according to any one of clauses 46 to 48 and 52 to 74 when X is 1 or 0.5, wherein the stopper has at least one rib of outer diameter 6.60 ± 0.15 mm.
77. The syringe according to any one of clauses 45 to 51 and 57 to 73 when X is 2, wherein the syringe has a length of 54.0 ± 0.5mm and an internal diameter of 8.65mm ± 0.05mm.
78. The syringe according to any one of clauses 45 to 51, 57 to 73 and 77, when X is 2, wherein the stopper has a maximum external diameter 9.05 ± 0.15mm and/or length 7.70 ± 0.4 mm when not inserted into the reservoir.
79. The syringe according to any one of clauses 45 to 51, 57 to 73 and 77 to 78, when X is 2, wherein the stopper has at least one rib of outer diameter 9.00 ± 0.15 mm.
80. The syringe according to any one of clauses 45 to 61 and 65 to 79 when not dependent on clauses 62 to 64, wherein a break loose force configured to move the stopper of the present invention from an initial stationary point to a velocity of 190mm/min is one or more of:
   a) about 2N to about 4N after storage at 5°C ± 1 °C for 2.5 months;
   b) about 2N to about 4N after storage at 5°C ± 1 °C for 6 months.
81. The syringe according to any one of clauses 45 to 56 and 62 to 79 when not dependent on clauses 57 to 61, wherein a break loose force configured to move the stopper of the present invention from an initial stationary point to a velocity of 190mm/min is one or more of:
   a) about 2N to about 3.5N at 25°C ± 1 °C after filling;
   b) about 2N to about 4.5N after storage at 25°C ± 1 °C for 1.5 months;
   c) about 2.5N to about 5N after storage at 25°C ± 1 °C for 2.5 months; and/or
   d) about 2.5N to about 5.5N after storage at 25°C ± 1 °C for 6 months.
82. The syringe according to any one of clauses 45 to 56 and 65 to 79 when not dependent on clauses 57 to 64, wherein a break loose force configured to move the stopper of the present invention from an initial stationary point to a velocity of 190mm/min is one or more of:
   a) about 3N to about 4.5N after storage at 40°C ± 1 °C for 1.5 months; and
   b) about 3N to about 5N after storage at 40°C ± 1 °C for 2.5 months.
83. The syringe according to any one of clauses 45 to 82, wherein the syringe is provided in needle shield device comprising a needle sleeve and a plunger; and wherein
   a manual force can be applied to the plunger to expel the 150mg/mL omalizumab formulation and unlatch needle sleeve so that the needle sleeve can cover the needle after injection.
84. The syringe according to any one of clauses 45 to 83, wherein the syringe is manufactured by a method comprising:
   aseptic filling of the reservoir with the 150mg/mL omalizumab formulation;
   and stoppering the reservoir with the stopper.
85. The syringe according to clause 84, wherein the aseptic filling is performed by a peristaltic pump or stainless steel piston pump.
86. The syringe according to clause 84 or 85 wherein, prior to the aseptic filling, the reservoir and needle are sterilised by ethylene oxide gas treatment, e-beam and/or steam sterilisation by autoclave.
87. The syringe according to clauses 45 to 86, which is suitable for use with an autoinjector.
88. A method of treating a patient with one or more of: allergic asthma; food allergy; chronic rhinosinusitis with nasal polyps; chronic spontaneous urticaria; nasal polyps; moderate to severe persistent asthma in patients with a positive skin test or *in vitro* reactivity to a perennial aeroallergen and symptoms that are inadequately controlled with inhaled corticosteroids, the method comprising administering 2 mL of 150mg/mL omalizumab formulation to the patient, wherein the omalizumab formulation is administered subcutaneously by a syringe, the syringe comprising:
   a reservoir filled with 2 mL of 150mg/mL omalizumab formulation, wherein the 2 mL 150mg/mL omalizumab formulation has 6000 or fewer particles of diameter ≥ 10µm and/or 600 or fewer particles of diameter ≥ 25µm; and the syringe further comprising:
   a stopper, and
   a needle having a gauge of 27; and
   the syringe being configured to expel the omalizumab formulation contained in the reservoir through the needle when a force is applied to the stopper.
89. The method of clause 88, wherein the needle has one of:
   a) a minimum internal diameter of 0.277mm;
   b) a minimum internal diameter of 0.191mm;
   c) a minimum internal diameter of 0.184mm; and
   d) a minimum internal diameter of 0.241mm.
90. A method of treating a patient with of one or more of: allergic asthma; food allergy; chronic rhinosinusitis with nasal polyps; chronic spontaneous urticaria; nasal polyps; moderate to severe persistent asthma in patients with a positive skin test or *in vitro* reactivity to a perennial aeroallergen and symptoms that are inadequately controlled with inhaled corticosteroids, the method comprising administering X mL of 150mg/mL omalizumab formulation to the patient, wherein X is 2, 1 or 0.5 and wherein the X mL 150mg/mL omalizumab formulation is administered subcutaneously by a syringe, the syringe comprising:
   a reservoir filled with X mL of 150mg/mL omalizumab formulation, wherein the XmL 150mg/mL omalizumab formulation has 6000 or fewer particles of diameter ≥ 10µm and/or 600 or fewer particles of diameter ≥ 25µm; and the syringe further comprising:
   a stopper, and
   a needle having a gauge of gauge 25 to gauge 29; and
   the syringe being configured to expel the omalizumab formulation contained in the reservoir through the needle when a force is applied to the stopper, wherein one of the following options is fulfilled when a constant force of 10N is applied to the stopper,
      a) when X is 2, the syringe is configured to expel the omalizumab formulation contained in the reservoir through the needle in less than 16 seconds;
      b) when X is 1, the syringe is configured to expel the omalizumab formulation contained in the reservoir through the needle in less than 5 seconds; and
      c) when X is 0.5, the syringe is configured to expel the omalizumab formulation contained in the reservoir through the needle in less than 4 seconds.
91. The method of clause 88 or 90 wherein X is 2, wherein the syringe is configured to expel the omalizumab formulation contained in the reservoir through the needle in one or more of:
   a) less than 8.5 seconds when a constant force of 17N is applied to the stopper; and
   b) less than 5 seconds when a constant force of 30N is applied to the stopper.
92. The method of clause 91, wherein the syringe is configured to expel X mL of the omalizumab formulation contained in the reservoir through the needle in one or more of:
   a) about 16 seconds to about 14 seconds when a constant force of 10N is applied to the stopper;
   b) about 8.5 seconds to about 6 seconds when a constant force of 17N is applied to the stopper; and
   c) about 5 seconds to about 4 seconds when a constant force of 30N is applied to the stopper.
93. The method of clause 90, wherein X is 1, and wherein the syringe is to expel X mL of 150mg/ml omalizumab formulation through the needle in one or more of:
   a) less than 5 seconds, less than 4.75 seconds, less than 4.5 seconds, less than 4.25 seconds, or in about 4 seconds when a constant force of 10N is applied to the stopper;
   b) less than 3 seconds when a constant force of 17N is applied to the stopper; and
   c) less than 2 seconds when a constant force of 30N is applied to the stopper.
94. The method of clause 93, wherein the syringe is configured to expel XmL of 150mg/ml omalizumab formulation through the needle in one or more of:
   a) less than 5 seconds to about 4 seconds, less than 4.75 seconds to about 4 seconds, less than 4.5 seconds to about 4 seconds, less than 4.25 seconds to about 4 seconds, or in about 4 seconds when a constant force of 10N is applied to the stopper;
   b) less than 3.5 seconds to about 2 seconds when a constant force of 17N is applied to the stopper; and
   c) less than 2 seconds to about 1 second when a constant force of 30N is applied to the stopper.
95. The method of clause 90, wherein X is 0.5, wherein the syringe is to expel X mL of 150mg/ml omalizumab formulation through the needle in one or more of:
   a) less than 2 seconds when a constant force of 17N is applied to the stopper; and
   b) less than 1 second when a constant force of 30N is applied to the stopper.
96. The method of clause 95, wherein the syringe is to expel XmL of 150mg/ml omalizumab formulation through the needle in one or more of:
   a) less than 4 seconds to about 2 seconds when a constant force of 10N is applied to the stopper;
   b) less than 2 seconds to about 1 second when a constant force of 17N is applied to the stopper; and
   c) less than 1 second to about 0.5 seconds when a constant force of 30N is applied to the stopper.
97. The method of clause 88 or clause 90, wherein the syringe contains:
   a) at least 76% main omalizumab charge variant measured by ion exchange chromatography after being filled with 150mg/mL omalizumab solution stored at 5°C ± 1°C; and/or
   b) at least 77% main omalizumab charge variant measured by ion exchange chromatography after being filled with 150mg/mL omalizumab solution and then stored at 5°C ± 1°C for 2.5 months.
98. The method of clause 88 or clause 90, wherein the percentage of main omalizumab charge variant in the syringe measured by ion exchange chromatography reduces by 2% or less after the syringe is stored at 5°C ± 1°C for 2.5 months measured from filling.
99. The method of clause 88 or clause 90, wherein the proportion of the first peak of the syringe measured by hydrophobic interaction chromatography is at least:
   a) 62% after filling with 150mg/mL omalizumab solution stored at 5°C ± 1°C; and/or
   b) 62% after being filled with 150mg/mL omalizumab solution and then stored at 5°C ± 1°C for 2.5 months.
100. The method of clause 88 or clause 90, wherein the proportion of the first peak of the syringe measured by hydrophobic interaction chromatography reduces by 0.4% or less after the syringe is stored at 5°C ± 1°C for 2.5 months.
101. The method of clause 88 or clause 90 wherein the syringe contains at least 0.4 mg/mL polysorbate 20 after filling with 150mg/mL omalizumab solution stored at 5°C ± 1°C.
102. The method of clause 88 or clause 90, wherein the syringe contains at least 69% main omalizumab charge variant measured by ion exchange chromatography after being filled with 150mg/mL omalizumab solution and then stored at 25°C ± 1°C for 1.5 months.
103. The method of clause 88 or clause 90, wherein the proportion of the first peak of the syringe measured by hydrophobic interaction chromatography is at least:
   a) 56% for the syringe after being filled with 150mg/mL omalizumab solution and then stored at 25°C ± 1°C for 1.5 months; and/or
   b) 51% for the syringe after being filled with 150mg/mL omalizumab solution and then stored at 25°C ± 1°C for 2.5 months.
104. The method of clause 88 or clause 90, wherein the syringe contains:
   a) at least 0.4mg/mL polysorbate 20 after being filled with 150mg/mL omalizumab solution and then stored at 25°C ± 1°C for 1.5 months; and/or
   b) at least 0.3mg/mL polysorbate 20 after being filled with 150mg/mL omalizumab solution and then stored at 25°C ± 1°C for 6 months.
105. The method of clause 90, wherein the needle has a gauge of gauge 26 to gauge 29, most preferably gauge 27.
106. The method of clause 90, wherein when the needle has a gauge of 25, the needle has one of:
   a) a minimum internal diameter of 0.292mm, and
   b) a minimum internal diameter of 0.232mm.
107. The method of clause 105, wherein when the needle has a gauge of 26, the needle has one of:
   a) a minimum internal diameter of 0.292mm; and
   b) a minimum internal diameter of 0.232mm.
108. The method of clause 105, wherein when the needle has a gauge of 27, the needle has one of:
   a) a minimum internal diameter of 0.277mm;
   b) a minimum internal diameter of 0.191mm;
   c) a minimum internal diameter of 0.184mm; and
   d) a minimum internal diameter of 0.241mm.
109. The method of clause 105, wherein when the needle has a gauge of 28, the needle has one of:
   a) a minimum internal diameter of 0.133mm; and
   b) a minimum internal diameter of 0.190mm.
110. The method of clause 105, wherein when the needle has a gauge of 29, the needle has one of:
   a) a minimum internal diameter of 0.265mm;
   b) a minimum internal diameter of 0.240mm;
   c) a minimum internal diameter of 0.190mm; and
   d) a minimum internal diameter of 0.133mm;
   preferably wherein the needle has a minimum internal diameter of 0.240mm or 0.265mm.
111. The method of clause 88 or clause 90 wherein the stopper has a fluoro-resin on the product contacting side or an ethylene tetrafluoroethylene (ETFE) barrier film lamination.
112. The method of clause 88 or clause 90, wherein the stopper has a B2-40 UV cured lubrication coating or is lubricated with 1000cSt silicone oil.
113. The method of clause 88 or clause 90 when X is 2, wherein the syringe has an internal coating of 0.7 ± 0.2mg of polydimethylsiloxane.
114. The method of clause 90, when X is 1 or 0.5, wherein the syringe has an internal coating of 0.4 ± 0.2mg of polydimethylsiloxane.
115. The method of clause 88 or clause 90, wherein the needle has 3 bevels on its distal surface or 5 bevels on its distal surface.
116. The method of clause 88 or clause 90, wherein the apparent viscosity of the 150mg/mL omalizumab formulation is 12 to 14mPa·s or, when measured at a shear rate of 200s⁻¹ is: 24.7 ± 0.5 mPa·s at 5.0 ± 0.2 °C, 15.3 ± 0.5 mPa·s at 15.0 ± 0.2 °C, 11.4 ± 0.5 mPa·s at 25.0 ± 0.2 °C, or 7.2 ± 1.2 mPa·s at 40.0 ± 0.2 °C.
117. The method of clause 88 or clause 90, wherein the syringe has a latex-free needle shield.
118. The method of clause 88 or clause 90, wherein the syringe has a round flange at a proximal end of the reservoir; optionally wherein the round flange has a maximum diameter of 11 ± 0.25mm or 14.7 ± 0.25mm.
119. The method of clause 90 when X is 1 or 0.5, wherein the syringe has a length of 54.0 ± 0.5mm and an internal diameter of 6.35mm ± 0.05mm.
120. The method of clause 90, wherein when the stopper is not inserted into the reservoir, it has one of:
   a) a maximum external diameter of 6.67mm to 7.10mm and/or a length of 7.85 ± 0.4 mm; and
   b) a maximum external diameter of 6.70 ± 0.15mm and/or a length of 7.85 ± 0.4 mm.
121. The method of clause 90 when X is 1 or 0.5, wherein the stopper has at least one circumferential rib of outer diameter 6.60 ± 0.15 mm.
122. The method of clause 88 or clause 90 when X is 2, wherein the syringe has a length of 54.0 ± 0.5mm and an internal diameter of 8.65mm ± 0.05mm.
123. The method of clause 88 or clause 90 when X is 2, wherein the stopper has a maximum external diameter 9.05 ± 0.15mm and/or length 7.70 ± 0.4 mm when not inserted into the reservoir.
124. The method of clause 88 or clause 90 when X is 2, wherein the stopper has at least one circumferential rib of outer diameter 9.00 ± 0.15 mm.
125. The method of clause 88 or clause 90 wherein a break loose force configured to move the stopper of the present invention from an initial stationary point to a velocity of 190mm/min is one or more of:
   a) about 2N to about 4N after storage at 5°C ± 1 °C for 2.5 months after filling;
   b) about 2N to about 4N after storage at 5°C ± 1 °C for 6 months after filling.
126. The method of clause 88 or clause 90 wherein a break loose force configured to move the stopper of the present invention from an initial stationary point to a velocity of 190mm/min is one or more of:
   a) about 2N to about 3.5N at 25°C ± 1 °C after filling;
   b) about 2N to about 4.5N after storage at 25°C ± 1 °C for 1.5 months after filling;
   c) about 2.5N to about 5N after storage at 25°C ± 1 °C for 2.5 months after filling; and/or
   d) about 2.5N to about 5.5N after storage at 25°C ± 1 °C for 6 months after filling.
127. The method of clause 88 or clause 90 wherein a break loose force configured to move the stopper of the present invention from an initial stationary point to a velocity of 190mm/min is one or more of:
   a) about 3N to about 4.5N after storage at 40°C ± 1 °C for 1.5 months after filling; and
   b) about 3N to about 5N after storage at 40°C ± 1 °C for 2.5 months after filling.
128. The method of clause 88 or clause 90 wherein the syringe is provided in a needle shield device comprising a needle sleeve and a plunger; and wherein
   a manual force can be applied to the plunger to expel the 150mg/mL omalizumab formulation and unlatch needle sleeve so that the needle sleeve can cover the needle after injection.
129. The method of clause 88 or clause 90, wherein the syringe is manufactured by a method comprising:
   aseptic filling of the reservoir with the 150mg/mL omalizumab formulation;
   and stoppering the reservoir with the stopper.
130. The method of claim 129, wherein the aseptic filling is performed by a peristaltic pump or stainless steel piston pump.
131. The method of claim 129 wherein, prior to the aseptic filling, the reservoir and needle are sterilised by ethylene oxide gas treatment, electron beam and/or steam sterilisation by autoclave.
132. A syringe comprising:
   a reservoir filled with X mL of 150mg/mL omalizumab formulation, wherein X is 2, 1 or 0.5, wherein the X mL of 150mg/mL omalizumab formulation has 6000 or fewer particles of diameter ≥ 10µm and/or 600 or fewer particles of diameter ≥ 25µm; and the syringe further comprising:
   a stopper, and
   a needle having a gauge of gauge 25 to gauge 29,
   the syringe being configured to expel X mL of the omalizumab formulation contained in the reservoir through the needle when a force is applied to the stopper, wherein one of the following options is fulfilled when a constant force of 10N is applied to the stopper:
      a) when X is 2, the syringe is configured to expel the omalizumab formulation contained in the reservoir through the needle in less than 16 seconds;
      b) when X is 1, the syringe is configured to expel the omalizumab formulation contained in the reservoir through the needle in less than 5 seconds; and
      c) when X is 0.5, the syringe is configured to expel the omalizumab formulation contained in the reservoir through the needle in less than 4 seconds.
133. The syringe of clause 132, wherein X is 1, wherein the syringe is configured to expel X mL of 150mg/ml omalizumab formulation through the needle in less than 5 seconds to about 4 seconds, in less than 4.75 seconds to about 4 seconds, in less than 4.5 seconds to about 4 seconds, in less than 4.25 seconds to about 4 seconds or in about 4 seconds when a constant force of 10N is applied to the stopper.
134. A syringe comprising:
   a reservoir filled with X mL of 150mg/mL omalizumab formulation, wherein X is 2, the X mL 150mg/mL omalizumab formulation has 6000 or fewer particles of diameter ≥ 10µm and/or 600 or fewer particles of diameter ≥ 25µm; and the syringe further comprising:
   a stopper, and
   a needle having a gauge of 27; and
   the syringe being configured to expel the omalizumab formulation contained in the reservoir through the needle when a force is applied to the stopper.
135. The method of clause 88 or clause 90, or the syringe according to clause 132 or 134, wherein the 150mg/ml omalizumab formulation is 150mg/mL antibody with 42.1mg/mL L-arginine hydrochloride, 1.37mg/mL L-histidine, 2.34mg/mL L-histidine hydrochloride monohydrate, 0.4mg/mL polysorbate 20 as an aqueous solution.
136. A method of treating a patient with one or more of: allergic asthma; food allergy; chronic rhinosinusitis with nasal polyps; chronic spontaneous urticaria; nasal polyps; moderate to severe persistent asthma in patients with a positive skin test or *in vitro* reactivity to a perennial aeroallergen and symptoms that are inadequately controlled with inhaled corticosteroids, the method comprising administering 300mg omalizumab formulation to the patient, wherein the method comprises administering 2mL of 150mg/mL omalizumab formulation subcutaneously by one syringe filled with 2mL 150mg/mL omalizumab formulation.
137. A method of treating a patient with one or more of: allergic asthma; food allergy; chronic rhinosinusitis with nasal polyps; chronic spontaneous urticaria; nasal polyps; moderate to severe persistent asthma in patients with a positive skin test or *in vitro* reactivity to a perennial aeroallergen and symptoms that are inadequately controlled with inhaled corticosteroids, the method comprising administering 375mg omalizumab formulation to the patient, wherein the method comprises:
   administering 2mL of 150mg/mL omalizumab formulation subcutaneously by one syringe filled with 2mL 150mg/mL omalizumab formulation; and
   administering 0.5mL of 150mg/mL omalizumab formulation subcutaneously by one syringe filled with 0.5mL 150mg/mL omalizumab formulation.
138. A method of treating a patient with one or more of: allergic asthma; food allergy; chronic rhinosinusitis with nasal polyps; chronic spontaneous urticaria; nasal polyps; moderate to severe persistent asthma in patients with a positive skin test or *in vitro* reactivity to a perennial aeroallergen and symptoms that are inadequately controlled with inhaled corticosteroids, the method comprising administering 450mg omalizumab formulation to the patient, wherein the method comprises:
   administering 2mL of 150mg/mL omalizumab formulation subcutaneously by one syringe filled with 2mL 150mg/mL omalizumab formulation; and
   administering 1mL of 150mg/mL omalizumab formulation subcutaneously by one syringe filled with 1mL 150mg/mL omalizumab formulation.
139. A method of treating a patient with one or more of: allergic asthma; food allergy; chronic rhinosinusitis with nasal polyps; chronic spontaneous urticaria; nasal polyps; moderate to severe persistent asthma in patients with a positive skin test or *in vitro* reactivity to a perennial aeroallergen and symptoms that are inadequately controlled with inhaled corticosteroids, the method comprising administering 525mg omalizumab formulation to the patient, wherein the method comprises:
   administering 2mL of 150mg/mL omalizumab formulation subcutaneously by one syringe filled with 2mL 150mg/mL omalizumab formulation;
   administering 1mL of 150mg/mL omalizumab formulation subcutaneously by one syringe filled with 1mL 150mg/mL omalizumab formulation; and
   administering 0.5mL of 150mg/mL omalizumab formulation subcutaneously by one syringe filled with 0.5mL 150mg/mL omalizumab formulation.
140. A method of treating a patient with one or more of: allergic asthma; food allergy; chronic rhinosinusitis with nasal polyps; chronic spontaneous urticaria; nasal polyps; moderate to severe persistent asthma in patients with a positive skin test or *in vitro* reactivity to a perennial aeroallergen and symptoms that are inadequately controlled with inhaled corticosteroids, the method comprising administering 600mg omalizumab formulation to the patient, wherein the method comprises:
   administering 2mL of 150mg/mL omalizumab formulation subcutaneously by one syringe filled with 2mL 150mg/mL omalizumab formulation; and
   administering 2mL of 150mg/mL omalizumab formulation subcutaneously by one syringe filled with 2mL 150mg/mL omalizumab formulation.
141. The method of any one of clauses 136 to 140, wherein any one of the steps requiring administering 2mL of 150mg/mL omalizumab formulation subcutaneously by one syringe filled with 2mL 150mg/mL omalizumab formulation comprises performing the method of clause 88, or clause 90 wherein X is 2.
142. The method of any one of clauses 137 and 139, wherein any one of the steps requiring administering 0.5mL 150mg/mL omalizumab formulation subcutaneously by one syringe filled with 0.5mL 150mg/mL omalizumab formulation comprises performing the method of clause 90 wherein X is 0.5.
143. The method of any one of clauses 138 and 139, wherein any one of the steps requiring administering 1mL 150mg/mL omalizumab formulation subcutaneously by one syringe filled with 1mL 150mg/mL omalizumab formulation comprises performing the method of clause 90 wherein X is 1.
144. A syringe comprising:
   a reservoir filled with 2 mL of 150mg/mL omalizumab formulation, wherein the 2 mL 150mg/mL omalizumab formulation has 6000 or fewer particles of diameter ≥ 10µm and/or 600 or fewer particles of diameter ≥ 25µm; and the syringe further comprises:
   a stopper, and
   a needle having a gauge of 27; and
   the syringe being configured to expel the omalizumab formulation contained in the reservoir through the needle when a force is applied to the stopper.
145. The syringe of clause 144, wherein the needle has one of:
   a) a minimum internal diameter of 0.277mm;
   b) a minimum internal diameter of 0.191mm;
   c) a minimum internal diameter of 0.184mm; and
   d) a minimum internal diameter of 0.241mm.
146. A syringe comprising:
   a reservoir filled with X mL of 150mg/mL omalizumab formulation, wherein X is 2, 1 or 0.5, wherein the X mL 150mg/mL omalizumab formulation has 6000 or fewer particles of diameter ≥ 10µm and/or 600 or fewer particles of diameter ≥ 25µm; and the syringe further comprises:
   a stopper, and
   a needle having a gauge of gauge 25 to gauge 29; and
   the syringe is configured to expel the omalizumab formulation contained in the reservoir through the needle when a force is applied to the stopper, wherein one of the following options is fulfilled when a constant force of 10N is applied to the stopper,
      a) when X is 2, the syringe is configured to expel the omalizumab formulation contained in the reservoir through the needle in less than 16 seconds;
      b) when X is 1, the syringe is configured to expel the omalizumab formulation contained in the reservoir through the needle in less than 5 seconds; and
      c) when X is 0.5, the syringe is configured to expel the omalizumab formulation contained in the reservoir through the needle in less than 4 seconds.
147. The syringe of clause 144 or 146 wherein X is 2, wherein the syringe is configured to expel the omalizumab formulation contained in the reservoir through the needle in one or more of:
   a) less than 8.5 seconds when a constant force of 17N is applied to the stopper; and
   b) less than 5 seconds when a constant force of 30N is applied to the stopper.
148. The syringe of clause 147, wherein the syringe is configured to expel X mL of the omalizumab formulation contained in the reservoir through the needle in one or more of:
   a) about 16 seconds to about 14 seconds when a constant force of 10N is applied to the stopper;
   b) about 8.5 seconds to about 6 seconds when a constant force of 17N is applied to the stopper; and
   c) about 5 seconds to about 4 seconds when a constant force of 30N is applied to the stopper.
149. The syringe of clause 146, wherein X is 1, and wherein the syringe is to expel X mL of 150mg/ml omalizumab formulation through the needle in one or more of:
   a) less than 5 seconds, less than 4.75 seconds, less than 4.5 seconds, less than 4.25 seconds, or in about 4 seconds when a constant force of 10N is applied to the stopper;
   b) less than 3 seconds when a constant force of 17N is applied to the stopper; and
   c) less than 2 seconds when a constant force of 30N is applied to the stopper.
150. The syringe of clause 149, wherein the syringe is configured to expel X mL of 150mg/ml omalizumab formulation through the needle in one or more of:
   a) less than 5 seconds to about 4 seconds, less than 4.75 seconds to about 4 seconds, less than 4.5 seconds to about 4 seconds, less than 4.25 seconds to about 4 seconds, or in about 4 seconds when a constant force of 10N is applied to the stopper;
   b) less than 3.5 seconds to about 2 seconds when a constant force of 17N is applied to the stopper; and
   c) less than 2 seconds to about 1 second when a constant force of 30N is applied to the stopper.
151. The syringe of clause 146, wherein X is 0.5, wherein the syringe is to expel X mL of 150mg/ml omalizumab formulation through the needle in one or more of:
   a) less than 2 seconds when a constant force of 17N is applied to the stopper; and
   b) less than 1 second when a constant force of 30N is applied to the stopper.
152. The syringe of clause 151, wherein the syringe is to expel X mL of 150mg/ml omalizumab formulation through the needle in one or more of:
   a) less than 4 seconds to about 2 seconds when a constant force of 10N is applied to the stopper;
   b) less than 2 seconds to about 1 second when a constant force of 17N is applied to the stopper; and
   c) less than 1 second to about 0.5 seconds when a constant force of 30N is applied to the stopper.
153. The syringe of clause 144 or clause 146, wherein the syringe contains:
   a) at least 76% main omalizumab charge variant measured by ion exchange chromatography after being filled with 150mg/mL omalizumab solution stored at 5°C ± 1°C; and/or
   b) at least 77% main omalizumab charge variant measured by ion exchange chromatography after being filled with 150mg/mL omalizumab solution and then stored at 5°C ± 1°C for 2.5 months.
154. The syringe of clause 144 or clause 146, wherein the percentage of main omalizumab charge variant in the syringe measured by ion exchange chromatography reduces by 2% or less after the syringe is stored at 5°C ± 1°C for 2.5 months measured from filling.
155. The syringe of clause 144 or clause 146, wherein the proportion of the first peak of the syringe measured by hydrophobic interaction chromatography is at least:
   a) 62% after filling with 150mg/mL omalizumab solution stored at 5°C ± 1°C; and/or
   b) 62% after being filled with 150mg/mL omalizumab solution and then stored at 5°C ± 1°C for 2.5 months.
156. The syringe of clause 144 or clause 146, wherein the proportion of the first peak of the syringe measured by hydrophobic interaction chromatography reduces by 0.4% or less after the syringe is stored at 5°C ± 1°C for 2.5 months.
157. The syringe of clause 144 or clause 146 wherein the syringe contains at least 0.4 mg/mL polysorbate 20 after filling with 150mg/mL omalizumab solution stored at 5°C ± 1°C.
158. The syringe of clause 144 or clause 146, wherein the syringe contains at least 69% main omalizumab charge variant measured by ion exchange chromatography after being filled with 150mg/mL omalizumab solution and then stored at 25°C ± 1°C for 1.5 months.
159. The syringe of clause 144 or clause 146, wherein the proportion of the first peak of the syringe measured by hydrophobic interaction chromatography is at least:
   a) 56% for the syringe after being filled with 150mg/mL omalizumab solution and then stored at 25°C ± 1°C for 1.5 months; and/or
   b) 51% for the syringe after being filled with 150mg/mL omalizumab solution and then stored at 25°C ± 1°C for 2.5 months.
160. The syringe of clause 144 or clause 146, wherein the syringe contains:
   a) at least 0.4mg/mL polysorbate 20 after being filled with 150mg/mL omalizumab solution and then stored at 25°C ± 1°C for 1.5 months; and/or
   b) at least 0.3mg/mL polysorbate 20 after being filled with 150mg/mL omalizumab solution and then stored at 25°C ± 1°C for 6 months.
161. The syringe of clause 146, wherein the needle has a gauge of gauge 26 to gauge 29, most preferably gauge 27.
162. The syringe of clause 146, wherein when the needle has a gauge of 25, the needle has one of:
   a) a minimum internal diameter of 0.292mm, and
   b) a minimum internal diameter of 0.232mm.
163. The syringe of clause 161, wherein when the needle has a gauge of 26, the needle has one of:
   a) a minimum internal diameter of 0.292mm; and
   b) a minimum internal diameter of 0.232mm.
164. The syringe of clause 161, wherein when the needle has a gauge of 27, the needle has one of:
   a) a minimum internal diameter of 0.277mm;
   b) a minimum internal diameter of 0.191mm;
   c) a minimum internal diameter of 0.184mm; and
   d) a minimum internal diameter of 0.241mm.
165. The syringe of clause 161, wherein when the needle has a gauge of 28, the needle has one of:
   a) a minimum internal diameter of 0.133mm; and
   b) a minimum internal diameter of 0.190mm.
166. The syringe of clause 161, wherein when the needle has a gauge of 29, the needle has one of:
   a) a minimum internal diameter of 0.265mm;
   b) a minimum internal diameter of 0.240mm;
   c) a minimum internal diameter of 0.190mm; and
   d) a minimum internal diameter of 0.133mm;
   preferably wherein the needle has a minimum internal diameter of 0.240mm or 0.265mm.
167. The syringe of clause 144 or clause 146 wherein the stopper has a fluoro-resin on the product contacting side or an ethylene tetrafluoroethylene (ETFE) barrier film lamination.
168. The syringe of clause 144 or clause 146, wherein the stopper has a B2-40 UV cured lubrication coating or is lubricated with 1000cSt silicone oil.
169. The syringe of clause 144 or clause 146 when X is 2, wherein the syringe has an internal coating of 0.7 ± 0.2mg of polydimethylsiloxane.
170. The syringe of clause 146, when X is 1 or 0.5, wherein the syringe has an internal coating of 0.4 ± 0.2mg of polydimethylsiloxane.
171. The syringe of clause 144 or clause 146, wherein the needle has 3 bevels on its distal surface or 5 bevels on its distal surface.
172. The syringe of clause 144 or clause 146, wherein the apparent viscosity of the 150mg/mL omalizumab formulation is 12 to 14mPa·s or, when measured at a shear rate of 200s⁻¹ is: 24.7 ± 0.5 mPa·s at 5.0 ± 0.2 °C, 15.3 ± 0.5 mPa·s at 15.0 ± 0.2 °C, 11.4 ± 0.5 mPa·s at 25.0 ± 0.2 °C, or 7.2 ± 1.2 mPa·s at 40.0 ± 0.2 °C.
173. The syringe of clause 144 or clause 146, wherein the syringe has a latex-free needle shield.
174. The syringe of clause 144 or clause 146, wherein the syringe has a round flange at a proximal end of the reservoir; optionally wherein the round flange has a maximum diameter of 11 ± 0.25mm or 14.7 ± 0.25mm.
175. The syringe of clause 146 when X is 1 or 0.5, wherein the syringe has a length of 54.0 ± 0.5mm and an internal diameter of 6.35mm ± 0.05mm.
176. The syringe of clause 146, wherein when the stopper is not inserted into the reservoir, it has one of:
   a) a maximum external diameter of 6.67mm to 7.10mm and/or a length of 7.85 ± 0.4 mm; and
   b) a maximum external diameter of 6.70 ± 0.15mm and/or a length of 7.85 ± 0.4 mm.
177. The syringe of clause 146 when X is 1 or 0.5, wherein the stopper has at least one circumferential rib of outer diameter 6.60 ± 0.15 mm.
178. The syringe of clause 144 or clause 146 when X is 2, wherein the syringe has a length of 54.0 ± 0.5mm and an internal diameter of 8.65mm ± 0.05mm.
179. The syringe of clause 144 or clause 146 when X is 2, wherein the stopper has a maximum external diameter 9.05 ± 0.15mm and/or length 7.70 ± 0.4 mm when not inserted into the reservoir.
180. The syringe of clause 144 or clause 146 when X is 2, wherein the stopper has at least one circumferential rib of outer diameter 9.00 ± 0.15 mm.
181. The syringe of clause 144 or clause 146 wherein a break loose force configured to move the stopper of the present invention from an initial stationary point to a velocity of 190mm/min is one or more of:
   a) about 2N to about 4N after storage at 5°C ± 1 °C for 2.5 months after filling;
   b) about 2N to about 4N after storage at 5°C ± 1 °C for 6 months after filling.
182. The syringe of clause 144 or clause 146 wherein a break loose force configured to move the stopper of the present invention from an initial stationary point to a velocity of 190mm/min is one or more of:
   a) about 2N to about 3.5N at 25°C ± 1 °C after filling;
   b) about 2N to about 4.5N after storage at 25°C ± 1 °C for 1.5 months after filling;
   c) about 2.5N to about 5N after storage at 25°C ± 1 °C for 2.5 months after filling; and/or
   d) about 2.5N to about 5.5N after storage at 25°C ± 1 °C for 6 months after filling.
183. The syringe of clause 144 or clause 146 wherein a break loose force configured to move the stopper of the present invention from an initial stationary point to a velocity of 190mm/min is one or more of:
   a) about 3N to about 4.5N after storage at 40°C ± 1 °C for 1.5 months after filling; and
   b) about 3N to about 5N after storage at 40°C ± 1 °C for 2.5 months after filling.
184. The syringe of clause 144 or clause 146 wherein the syringe is provided in a needle shield device comprising a needle sleeve and a plunger; and wherein
   a manual force can be applied to the plunger to expel the 150mg/mL omalizumab formulation and unlatch needle sleeve so that the needle sleeve can cover the needle after injection.
185. The syringe of clause 144 or clause 146, wherein the syringe is manufactured by a method comprising:
   aseptic filling of the reservoir with the 150mg/mL omalizumab formulation;
   and stoppering the reservoir with the stopper.
186. The syringe of clause 185, wherein the aseptic filling is performed by a peristaltic pump or stainless steel piston pump.
187. The syringe of clause 185 wherein, prior to the aseptic filling, the reservoir and needle are sterilised by ethylene oxide gas treatment, electron beam and/or steam sterilisation by autoclave.
188. The syringe of clause 146, wherein X is 1, wherein the syringe is configured to expel X mL of 150mg/ml omalizumab formulation through the needle in less than 5 seconds to about 4 seconds, in less than 4.75 seconds to about 4 seconds, in less than 4.5 seconds to about 4 seconds, in less than 4.25 seconds to about 4 seconds or in about 4 seconds when a constant force of 10N is applied to the stopper.
189. The syringe of clause 144 or clause 146, wherein the 150mg/ml omalizumab formulation is 150mg/mL antibody with 42.1mg/mL L-arginine hydrochloride, 1.37mg/mL L-histidine, 2.34mg/mL L-histidine hydrochloride monohydrate, 0.4mg/mL polysorbate 20 as an aqueous solution.
190. A method of treating a patient with one or more of: allergic asthma; food allergy; chronic rhinosinusitis with nasal polyps; chronic spontaneous urticaria; nasal polyps; moderate to severe persistent asthma in patients with a positive skin test or *in vitro* reactivity to a perennial aeroallergen and symptoms that are inadequately controlled with inhaled corticosteroids, the method comprising administering 225mg omalizumab formulation to the patient; wherein the method comprises one of:
   a) performing the method of clause 90 wherein X is 0.5; and performing the method of any one of claim 3 wherein X is 1;
   b) performing the method of clause 90 wherein X is 0.5; and administering 1mL 150mg/mL omalizumab formulation subcutaneously by a syringe; and
   c) performing the method of clause 90 wherein X is 1; and administering 0.5mL 150mg/mL omalizumab formulation subcutaneously by a syringe.
191. A method of treating a patient with one or more of: allergic asthma; food allergy; chronic rhinosinusitis with nasal polyps; chronic spontaneous urticaria; nasal polyps; moderate to severe persistent asthma in patients with a positive skin test or *in vitro* reactivity to a perennial aeroallergen and symptoms that are inadequately controlled with inhaled corticosteroids, the method comprising administering 300mg omalizumab formulation to the patient; wherein the method comprises administering 1mL 150mg/mL omalizumab formulation subcutaneously by a syringe and performing the method of clause 90 wherein X is 1.
192. A method of treating a patient with one or more of: allergic asthma; food allergy; chronic rhinosinusitis with nasal polyps; chronic spontaneous urticaria; nasal polyps; moderate to severe persistent asthma in patients with a positive skin test or *in vitro* reactivity to a perennial aeroallergen and symptoms that are inadequately controlled with inhaled corticosteroids, the method comprising administering 375mg omalizumab formulation to the patient; wherein the method comprises administering 0.5mL 150mg/mL omalizumab formulation subcutaneously by a syringe; and one of:
   a) performing the method of clause 88 or clause 90 wherein X is 2; and
   b) performing the method of clause 90 wherein X is 1 and administering 1mL 150mg/mL omalizumab formulation subcutaneously by a syringe.
193. A method of treating a patient with one or more of: allergic asthma; food allergy; chronic rhinosinusitis with nasal polyps; chronic spontaneous urticaria; nasal polyps; moderate to severe persistent asthma in patients with a positive skin test or *in vitro* reactivity to a perennial aeroallergen and symptoms that are inadequately controlled with inhaled corticosteroids, the method comprising administering 450mg omalizumab formulation to the patient; wherein the method comprises administering 1mL 150mg/mL omalizumab formulation subcutaneously by a syringe; and one of:
   a) performing the method of clause 88 or clause 90 wherein X is 2; and
   b) performing the method of clause 90 wherein X is 1 and administering 1mL 150mg/mL omalizumab formulation subcutaneously by a syringe.
194. A method of treating a patient with one or more of: allergic asthma; food allergy; chronic rhinosinusitis with nasal polyps; chronic spontaneous urticaria; nasal polyps; moderate to severe persistent asthma in patients with a positive skin test or *in vitro* reactivity to a perennial aeroallergen and symptoms that are inadequately controlled with inhaled corticosteroids, the method comprising administering 525mg omalizumab formulation to the patient; wherein the method comprises administering 1mL 150mg/mL omalizumab formulation subcutaneously by a syringe and administering 0.5mL 150mg/mL omalizumab formulation subcutaneously by a syringe; and one of:
   a) performing the method of clause 88 or clause 90 wherein X is 2;
   b) performing the method of clause 90 wherein X is 1 and administering 1mL 150mg/mL omalizumab formulation subcutaneously by a syringe.
195. A method of treating a patient with one or more of: allergic asthma; food allergy; chronic rhinosinusitis with nasal polyps; chronic spontaneous urticaria; nasal polyps; moderate to severe persistent asthma in patients with a positive skin test or *in vitro* reactivity to a perennial aeroallergen and symptoms that are inadequately controlled with inhaled corticosteroids, the method comprising administering 600mg omalizumab formulation to the patient; wherein the method comprises performing the method of clause 88 or clause 90 wherein X is 2 and then performing the method of clause 88 or clause 90 wherein X is 2.
196. A method of treating a patient with one or more of: allergic asthma; food allergy; chronic rhinosinusitis with nasal polyps; chronic spontaneous urticaria; nasal polyps; moderate to severe persistent asthma in patients with a positive skin test or *in vitro* reactivity to a perennial aeroallergen and symptoms that are inadequately controlled with inhaled corticosteroids, the method comprising administering 600mg omalizumab formulation to the patient; wherein the method comprises administering 1mL 150mg/mL omalizumab formulation subcutaneously by a syringe, administering 1mL 150mg/mL omalizumab formulation subcutaneously by a syringe administering 1mL 150mg/mL omalizumab formulation subcutaneously by a syringe; and performing the method of clause 90 wherein X is 1.
197. The method of any one of clauses 190, 192 and 194, wherein any one of the steps requiring administering 0.5mL 150mg/mL omalizumab formulation subcutaneously by a syringe comprises performing the method of clause 90 wherein X is 0.5.
198. The method of any one of claims 190 to 194 and 196, wherein any one of the steps requiring administering 1mL 150mg/mL omalizumab formulation subcutaneously by a syringe comprises performing the method of clause wherein X is 1.
199. X mL of 150mg/mL omalizumab formulation for use according to any one of clauses 1 to 42, or a syringe according to any one of clauses 43 to 87, 132 to 134 and 144 to 189, or a method according to any one of clauses 88 to 131, 136 to 143 and 190 to 198, wherein the 150mg/ml omalizumab formulation is 150mg/mL antibody with 42.1mg/mL L-arginine hydrochloride, 1.37mg/mL L-histidine, 2.34mg/mL L-histidine hydrochloride monohydrate, 0.4mg/L polysorbate 20 as an aqueous solution.

## Claims

1. X mL of 150mg/mL omalizumab formulation for use in a method of treatment of one or more of: allergic asthma; food allergy; chronic rhinosinusitis with nasal polyps; chronic spontaneous urticaria; nasal polyps; moderate to severe persistent asthma in patients with a positive skin test or *in vitro* reactivity to a perennial aeroallergen and symptoms that are inadequately controlled with inhaled corticosteroids, wherein X is 2 and wherein the XmL 150mg/mL omalizumab formulation is administered subcutaneously by a syringe, the syringe comprising:
a reservoir filled with X mL of 150mg/mL omalizumab formulation, wherein the XmL 150mg/mL omalizumab formulation has 6000 or fewer particles of diameter ≥ 10µm and/or 600 or fewer particles of diameter ≥ 25µm; and the syringe further comprising:
a stopper, and
a needle having a gauge of 27; and
the syringe being configured to expel the omalizumab formulation contained in the reservoir through the needle when a force is applied to the stopper.

2. X mL of 150mg/mL omalizumab formulation for use according to claim 1, wherein the needle has one of:
a) a minimum internal diameter of 0.277mm;
b) a minimum internal diameter of 0.191mm;
c) a minimum internal diameter of 0.184mm; and
d) a minimum internal diameter of 0.241mm.

3. X mL of 150mg/mL omalizumab formulation for use in a method of treatment of one or more of: allergic asthma; food allergy; chronic rhinosinusitis with nasal polyps; chronic spontaneous urticaria; nasal polyps; moderate to severe persistent asthma in patients with a positive skin test or *in vitro* reactivity to a perennial aeroallergen and symptoms that are inadequately controlled with inhaled corticosteroids, wherein X is 2, 1 or 0.5 and wherein the X mL 150mg/mL omalizumab formulation is administered subcutaneously by a syringe, the syringe comprising:
a reservoir filled with X mL of 150mg/mL omalizumab formulation, wherein the XmL 150mg/mL omalizumab formulation has 6000 or fewer particles of diameter≥ 10µm and/or 600 or fewer particles of diameter ≥ 25µm; and the syringe further comprising:
a stopper, and
a needle; and
the syringe being configured to expel the omalizumab formulation contained in the reservoir through the needle when a force is applied to the stopper, wherein one of the following options is fulfilled when a constant force of 10N is applied to the stopper,
a) when X is 2, the syringe is configured to expel the omalizumab formulation contained in the reservoir through the needle in less than 16 seconds;
b) when X is 1, the syringe is configured to expel the omalizumab formulation contained in the reservoir through the needle in less than 7.5 seconds; and
c) when X is 0.5, the syringe is configured to expel the omalizumab formulation contained in the reservoir through the needle in less than 4 seconds.

4. X mL of 150mg/mL omalizumab formulation for use according to any one of claims 1 to 3 wherein X is 2, wherein the syringe is configured to expel the omalizumab formulation contained in the reservoir through the needle in one or more of:
a) less than 8.5 seconds when a constant force of 17N is applied to the stopper; and
b) less than 5 seconds when a constant force of 30N is applied to the stopper.

5. X mL of 150mg/mL omalizumab formulation for use according to claim 4, wherein the syringe is configured to expel X mL of the omalizumab formulation contained in the reservoir through the needle in one or more of:
a) about 16 seconds to about 14 seconds when a constant force of 10N is applied to the stopper;
b) about 8.5 seconds to about 6 seconds when a constant force of 17N is applied to the stopper; and
c) about 5 seconds to about 4 seconds when a constant force of 30N is applied to the stopper.

6. X mL of 150mg/mL omalizumab formulation for use according to claim 3, wherein X is 1, and wherein the syringe is to expel X mL of 150mg/ml omalizumab formulation through the needle in one or more of:
a) less than 3 seconds when a constant force of 17N is applied to the stopper; and
b) less than 2 seconds when a constant force of 30N is applied to the stopper.

7. X mL of 150mg/mL omalizumab formulation for use according to claim 6, wherein the syringe is configured to expel X mL of 150mg/ml omalizumab formulation through the needle in one or more of:
a) less than 7.5 seconds to about 4 seconds when a constant force of 10N is applied to the stopper;
b) less than 3.5 seconds to about 2 seconds when a constant force of 17N is applied to the stopper; and
c) less than 2 seconds to about 1 second when a constant force of 30N is applied to the stopper.

8. X mL of 150mg/mL omalizumab formulation for use according to any one of claims 3, 6 and 7, wherein X is 1, and wherein the syringe is to expel X mL of 150mg/ml omalizumab formulation through the needle in less than 5 seconds, less than 4.75 seconds, less than 4.5 seconds, less than 4.25 seconds, or in about 4 seconds when a constant force of 10N is applied to the stopper.

9. X mL of 150mg/mL omalizumab formulation for use according to any one of claims 3, 6 and 7, wherein X is 1, and wherein the syringe is to expel X mL of 150mg/ml omalizumab formulation through the needle in less than 5 seconds to about 4 seconds, less than 4.75 seconds to about 4 seconds, less than 4.5 seconds to about 4 seconds, less than 4.25 seconds to about 4 seconds, or in about 4 seconds when a constant force of 10N is applied to the stopper.

10. X mL of 150mg/mL omalizumab formulation for use according to claim 3, wherein X is 0.5, wherein the syringe is to expel X mL of 150mg/ml omalizumab formulation through the needle in one or more of:
a) less than 2 seconds when a constant force of 17N is applied to the stopper; and
b) less than 1 second when a constant force of 30N is applied to the stopper.

11. X mL of 150mg/mL omalizumab formulation for use according to claim 10, wherein the syringe is to expel X mL of 150mg/ml omalizumab formulation through the needle in one or more of:
a) less than 4 seconds to about 2 seconds when a constant force of 10N is applied to the stopper;
b) less than 2 seconds to about 1 second when a constant force of 17N is applied to the stopper; and
c) less than 1 second to about 0.5 seconds when a constant force of 30N is applied to the stopper.

12. X mL of 150mg/mL omalizumab formulation for use according to any one of claims 1 to 11, wherein the syringe contains:
a) at least 76% main omalizumab charge variant measured by ion exchange chromatography after being filled with 150mg/mL omalizumab solution stored at 5°C ± 1°C; and/or
b) at least 77% main omalizumab charge variant measured by ion exchange chromatography after being filled with 150mg/mL omalizumab solution and then stored at 5°C ± 1°C for at 2.5 months.

13. X mL of 150mg/mL omalizumab formulation for use according to any one of claims 1 to 11, wherein the percentage of main omalizumab charge variant in the syringe measured by ion exchange chromatography reduces by 2% or less after the syringe is stored at 5°C ± 1°C for at 2.5 months.

14. X mL of 150mg/mL omalizumab formulation for use according to any one of claims 1 to 13, wherein the proportion of the first peak of the syringe measured by hydrophobic interaction chromatography is at least:
a) 62% after filling with 150mg/mL omalizumab solution stored at 5°C ± 1°C; and/or
b) 62% after being filled with 150mg/mL omalizumab solution and then stored at 5°C ± 1°C for 2.5 months.

15. X mL of 150mg/mL omalizumab formulation for use according to any one of claims 1 to 14, wherein the proportion of the first peak of the syringe measured by hydrophobic interaction chromatography reduces by 0.4% or less after the syringe is stored at 5°C ± 1°C for at 2.5 months.

16. X mL of 150mg/mL omalizumab formulation for use according to any one of claims 1 to 15, wherein the syringe contains at least 0.4 mg/mL polysorbate 20 after filling with 150mg/mL omalizumab solution stored at 5°C ± 1°C.

17. X mL of 150mg/mL omalizumab formulation for use according to any one of claims 1 to 12, wherein the syringe contains at least 69% main omalizumab charge variant measured by ion exchange chromatography after being filled with 150mg/mL omalizumab solution and then stored at 25°C ± 1°C for 1.5 months.

18. X mL of 150mg/mL omalizumab formulation for use according to any one of claims 1 to 12 and 17, wherein the proportion of the first peak of the syringe measured by hydrophobic interaction chromatography is at least:
a) 56% for the syringe after being filled with 150mg/mL omalizumab solution and then stored at 25°C ± 1°C for 1.5 months; and/or
b) 51% for the syringe after being filled with 150mg/mL omalizumab solution and then stored at 25°C ± 1°C for 2.5 months.

19. X mL of 150mg/mL omalizumab formulation for use according to any one of claims 1 to 12, 17 and 18, wherein the syringe contains:
a) at least 0.4mg/mL polysorbate 20 after being filled with 150mg/mL omalizumab solution and then stored at 25°C ± 1°C for 1.5 months; and/or
b) at least 0.3mg/mL polysorbate 20 after being filled with 150mg/mL omalizumab solution and then stored at 25°C ± 1°C for 6 months.

20. X mL of 150mg/mL omalizumab formulation for use according to any one of claims 3 to 19 when not dependent on claim 1 or claim 2, wherein the needle has one of: gauge 23, gauge 24, gauge 25, gauge 26, gauge 27, gauge 28, gauge 29, gauge 30, gauge 31 and gauge 32.

21. X mL of 150mg/mL omalizumab formulation for use according to any one of claims 3 to 19 when not dependent on claim 1 or claim 2, wherein the needle has one of:
a) gauge 23 defining a minimum internal diameter of 0.370mm,
b) gauge 23 defining a minimum internal diameter of 0.317mm,
c) gauge 24 defining a minimum internal diameter of 0.343mm
d) gauge 24 defining a minimum internal diameter of 0.280mm
e) gauge 25 defining a minimum internal diameter of 0.292mm,
f) gauge 25 defining a minimum internal diameter of 0.232mm,
g) gauge 26 defining a minimum internal diameter of 0.292mm,
h) gauge 26 defining a minimum internal diameter of 0.232mm,
i) gauge 27 defining a minimum internal diameter of 0.277mm;
j) gauge 27 defining a minimum internal diameter of 0.191 mm;
k) gauge 27 defining a minimum internal diameter of 0.184mm;
l) gauge 27 defining a minimum internal diameter of 0.241mm;
m) gauge 28 defining a minimum internal diameter of 0.133mm,
n) gauge 28 defining a minimum internal diameter of 0.190mm,
o) gauge 29 defining a minimum internal diameter of 0.265mm
p) gauge 29 defining a minimum internal diameter of 0.240mm,
q) gauge 29 defining a minimum internal diameter of 0.190mm,
r) gauge 29 defining a minimum internal diameter of 0.133mm,
s) gauge 30 defining a minimum internal diameter of 0.240mm,
t) gauge 30 defining a minimum internal diameter of 0.190mm,
u) gauge 30 defining a minimum internal diameter of 0.165mm,
v) gauge 30 defining a minimum internal diameter of 0.133mm,
w) gauge 31 defining a minimum internal diameter of 0.176mm,
x) gauge 31 defining a minimum internal diameter of 0.146mm,
y) gauge 31 defining a minimum internal diameter of 0.125mm,
z) gauge 31 defining a minimum internal diameter of 0.114mm,
aa) gauge 32 defining a minimum internal diameter of 0.146mm,
bb) gauge 32 defining a minimum internal diameter of 0.125mm,
cc) gauge 32 defining a minimum internal diameter of 0.105mm, and
dd) gauge 32 defining a minimum internal diameter of 0.089mm.

22. X mL of 150mg/mL omalizumab formulation for use according to any one of claims 3 to 19 when not dependent on claim 1 or claim 2, wherein the needle has a gauge of gauge 25 to gauge 29.

23. X mL of 150mg/mL omalizumab formulation for use according to claim 22, wherein the needle has a gauge of gauge 26 to gauge 29, most preferably gauge 27.

24. X mL of 150mg/mL omalizumab formulation for use according to claim 22, wherein when the needle has a gauge of 25, the needle has one of:
a) a minimum internal diameter of 0.292mm, and
b) a minimum internal diameter of 0.232mm.

25. X mL of 150mg/mL omalizumab formulation for use according to claim 23, wherein when the needle has a gauge of 26, the needle has one of:
a) a minimum internal diameter of 0.292mm; and
b) a minimum internal diameter of 0.232mm.

26. X mL of 150mg/mL omalizumab formulation for use according to claim 23, wherein when the needle has a gauge of 27, the needle has one of:
a) a minimum internal diameter of 0.277mm;
b) a minimum internal diameter of 0.191mm;
c) a minimum internal diameter of 0.184mm; and
d) a minimum internal diameter of 0.241mm.

27. X mL of 150mg/mL omalizumab formulation for use according to claim 23, wherein when the needle has a gauge of 28, the needle has one of:
a) a minimum internal diameter of 0.133mm; and
b) a minimum internal diameter of 0.190mm.

28. X mL of 150mg/mL omalizumab formulation for use according to claim 23, wherein when the needle has a gauge of 29, the needle has one of:
a) a minimum internal diameter of 0.265mm;
b) a minimum internal diameter of 0.240mm;
c) a minimum internal diameter of 0.190mm; and
d) a minimum internal diameter of 0.133mm;
preferably wherein the needle has a minimum internal diameter of 0.240mm or 0.265mm.

29. X mL of 150mg/mL omalizumab formulation for use according to any one of claims 1 to 28, wherein the stopper has a fluoro-resin on the product contacting side or an ethylene tetrafluoroethylene (ETFE) barrier film lamination.

30. X mL of 150mg/mL omalizumab formulation for use according to any one of claims 1 to 29, wherein the stopper has a B2-40 UV cured lubrication coating or is lubricated with 1000cSt silicone oil.

31. X mL of 150mg/mL omalizumab formulation for use according to any one of claims 1 to 5 and 12 to 30 wherein X is 2, wherein the syringe has an internal coating of 0.7 ± 0.2mg of polydimethylsiloxane.

32. X mL of 150mg/mL omalizumab formulation for use according to any one of claims 1 to 30, wherein the syringe has an internal coating of 0.4 ± 0.2mg of polydimethylsiloxane.

33. X mL of 150mg/mL omalizumab formulation for use according to any one of claims 1 to 32, wherein the needle has 3 bevels on its distal surface or 5 bevels on its distal surface.

34. X mL of 150mg/mL omalizumab formulation for use according to any one of claims 1 to 33, wherein the apparent viscosity of the 150mg/mL omalizumab formulation at a shear rate of 200s⁻¹ is: 24.7 ± 0.5 mPa·s at 5.0 ± 0.2 °C, 15.3 ± 0.5 mPa·s at 15.0 ± 0.2 °C, 11.4 ± 0.5 mPa·s at 25.0 ± 0.2 °C, or 7.2 ± 1.2 mPa·s at 40.0 ± 0.2 °C.

35. X mL of 150mg/mL omalizumab formulation for use according to any one of claims 1 to 34, wherein the syringe has a latex-free needle shield.

36. X mL of 150mg/mL omalizumab formulation for use according to any one of claims 1 to 35, wherein the syringe has a round flange; optionally wherein the round flange has a maximum diameter of 11 ± 0.25mm or 14.7 ± 0.25mm.

37. X mL of 150mg/mL omalizumab formulation for use according to any one of claims 3, 6 to 30 and 32 to 36 when X is 1 or 0.5, wherein the syringe has a length of 54.0 ± 0.5mm and an internal diameter of 6.35mm ± 0.05mm.

38. X mL of 150mg/mL omalizumab formulation for use according to any one of claims 3, 6 to 30 and 32 to 37 when X is 1 or 0.5, wherein when the stopper is not inserted into the reservoir, it has one of:
a) a maximum external diameter of 6.67mm to 7.10mm and/or a length of 7.85 ± 0.4 mm; and
b) a maximum external diameter of 6.70 ± 0.15mm and/or a length of 7.85 ± 0.4 mm.

39. X mL of 150mg/mL omalizumab formulation for use according to any one of claims 3, 6 to 30 and 32 to 37, when X is 1 or 0.5, wherein the stopper has at least one rib of outer diameter 6.60 ± 0.15 mm.

40. X mL of 150mg/mL omalizumab formulation for use according to any one of claims 1 to 5 and 12 to 36 when X is 2, wherein the syringe has a length of 54.0 ± 0.5mm and an internal diameter of 8.65mm ± 0.05mm.

41. X mL of 150mg/mL omalizumab formulation for use according to any one of claims 1 to 5, 12 to 36 and 40, when X is 2, wherein the stopper has a maximum external diameter 9.05 ± 0.15mm and/or length 7.70 ± 0.4 mm when not inserted into the reservoir.

42. X mL of 150mg/mL omalizumab formulation for use according to any one of claims 1 to 5, 12 to 36 and 40 to 41, when X is 2, wherein the stopper has at least one rib of outer diameter 9.00 ± 0.15 mm.

43. X mL of 150mg/mL omalizumab formulation for use according to any one of claims 1 to 16 and 20 to 42 when not dependent on claims 17 to 20, wherein a break loose force configured to move the stopper of the present invention from an initial stationary point to a velocity of 190mm/min is one or more of:
a) about 2N to about 4N after storage at 5°C ± 1 °C for 2.5 months;
b) about 2N to about 4N after storage at 5°C ± 1 °C for 6 months.

44. X mL of 150mg/mL omalizumab formulation for use according to any one of claims 1 to 11 and 17 to 42 when not dependent on claims 12 to 16, wherein a break loose force configured to move the stopper of the present invention from an initial stationary point to a velocity of 190mm/min is one or more of:
a) about 2N to about 3.5N at 25°C ± 1 °C after filling;
b) about 2N to about 4.5N after storage at 25°C ± 1 °C for 1.5 months;
c) about 2.5N to about 5N after storage at 25°C ± 1 °C for 2.5 months; and/or
d) about 2.5N to about 5.5N after storage at 25°C ± 1 °C for 6 months.

45. X mL of 150mg/mL omalizumab formulation for use according to any one of claims 1 to 11 and 20 to 42 when not dependent on claims 12 to 19, wherein a break loose force configured to move the stopper of the present invention from an initial stationary point to a velocity of 190mm/min is one or more of:
a) about 3N to about 4.5N after storage at 40°C ± 1 °C for 1.5 months; and
b) about 3N to about 5N after storage at 40°C ± 1 °C for 2.5 months.

46. X mL of 150mg/mL omalizumab formulation for use according to any one of claims 1 to 45, wherein the syringe is provided in needle shield device comprising a needle sleeve and a plunger; and wherein
a manual force can be applied to the plunger to expel the 150mg/mL omalizumab formulation and unlatch needle sleeve so that the needle sleeve can cover the needle after injection.

47. X mL of 150mg/mL omalizumab formulation for use according to any one of claims 1 to 46, wherein the syringe is manufactured by a method comprising:
aseptic filling of the reservoir with the 150mg/mL omalizumab formulation;
and stoppering the reservoir with the stopper.

48. X mL of 150mg/mL omalizumab formulation for use according to claim 47, wherein the aseptic filling is performed by a peristaltic pump or stainless steel piston pump.

49. X mL of 150mg/mL omalizumab formulation for use according to claim 47 or 48 wherein, prior to the aseptic filling, the reservoir and needle are sterilised by ethylene oxide gas treatment, e-beam and/or steam sterilisation by autoclave.

50. A syringe comprising:
a reservoir filled with X mL of 150mg/mL omalizumab formulation, wherein X is 2, 1 or 0.5, wherein the X mL of 150mg/mL omalizumab formulation has 6000 or fewer particles of diameter ≥ 10µm and/or 600 or fewer particles of diameter ≥ 25µm; and the syringe further comprising:
a stopper, and
a needle,
the syringe being configured to expel X mL of the omalizumab formulation contained in the reservoir through the needle when a force is applied to the stopper, wherein one of the following options is fulfilled when a constant force of 10N is applied to the stopper,
a) when X is 2, the syringe is configured to expel the omalizumab formulation contained in the reservoir through the needle in less than 16 seconds;
b) when X is 1, the syringe is configured to expel the omalizumab formulation contained in the reservoir through the needle in less than 7.5 seconds; and
c) when X is 0.5, the syringe is configured to expel the omalizumab formulation contained in the reservoir through the needle in less than 4 seconds.

51. The syringe of claim 50, wherein the needle has a gauge of gauge 25 to 29.

52. The syringe of claim 50 or 51, wherein the syringe is configured to expel X mL of 150mg/ml omalizumab formulation through the needle in less than 5 seconds, less than 4.75 seconds, less than 4.5 seconds, less than 4.25 seconds, or in about 4 seconds when a constant force of 10N is applied to the stopper.

53. The syringe of claim 50 or 51, wherein X is 1, wherein the syringe is configured to expel X mL of 150mg/ml omalizumab formulation through the needle in less than 5 seconds to about 4 seconds, in less than 4.75 seconds to about 4 seconds, in less than 4.5 seconds to about 4 seconds, in less than 4.25 seconds to about 4 seconds or in about 4 seconds when a constant force of 10N is applied to the stopper.

54. A syringe comprising:
a reservoir filled with X mL of 150mg/mL omalizumab formulation, wherein X is 2, the X mL 150mg/mL omalizumab formulation has 6000 or fewer particles of diameter ≥ 10µm and/or 600 or fewer particles of diameter ≥ 25µm; and the syringe further comprising:
a stopper, and
a needle having a gauge of 27; and
the syringe being configured to expel the omalizumab formulation contained in the reservoir through the needle when a force is applied to the stopper.

55. X mL of 150mg/mL omalizumab formulation for use according to any one of claims 1 to 49, or the syringe according to any one of claims 50 to 54, wherein the 150mg/ml omalizumab formulation is 150mg/mL antibody with 42.1mg/mL L-arginine hydrochloride, 1.37mg/mL L-histidine, 2.34mg/mL L-histidine hydrochloride monohydrate, 0.4mg/mL polysorbate 20 as an aqueous solution.

56. 150mg/mL omalizumab formulation for use in a method of treatment of one or more of: allergic asthma; food allergy; chronic rhinosinusitis with nasal polyps; chronic spontaneous urticaria; nasal polyps; moderate to severe persistent asthma in patients with a positive skin test or *in vitro* reactivity to a perennial aeroallergen and symptoms that are inadequately controlled with inhaled corticosteroids, wherein 300mg 150mg/mL omalizumab formulation is administered subcutaneously by one syringe filled with 2mL 150mg/mL omalizumab formulation.

57. 150mg/mL omalizumab formulation for use in a method of treatment of one or more of: allergic asthma; food allergy; chronic rhinosinusitis with nasal polyps; chronic spontaneous urticaria; nasal polyps; moderate to severe persistent asthma in patients with a positive skin test or *in vitro* reactivity to a perennial aeroallergen and symptoms that are inadequately controlled with inhaled corticosteroids, wherein 375mg 150mg/mL omalizumab formulation is administered subcutaneously by one syringe filled with 2mL 150mg/mL omalizumab formulation and one syringe filled with 0.5mL 150mg/mL omalizumab formulation.

58. 150mg/mL omalizumab formulation for use in a method of treatment of one or more of: allergic asthma; food allergy; chronic rhinosinusitis with nasal polyps; chronic spontaneous urticaria; nasal polyps; moderate to severe persistent asthma in patients with a positive skin test or *in vitro* reactivity to a perennial aeroallergen and symptoms that are inadequately controlled with inhaled corticosteroids, wherein 450mg 150mg/mL omalizumab formulation is administered subcutaneously by one syringe filled with 2mL 150mg/mL omalizumab formulation and one syringe filled with 1mL 150mg/mL omalizumab formulation.

59. 150mg/mL omalizumab formulation for use in a method of treatment of one or more of: allergic asthma; food allergy; chronic rhinosinusitis with nasal polyps; chronic spontaneous urticaria; nasal polyps; moderate to severe persistent asthma in patients with a positive skin test or *in vitro* reactivity to a perennial aeroallergen and symptoms that are inadequately controlled with inhaled corticosteroids, wherein 525mg 150mg/mL omalizumab formulation is administered subcutaneously by one syringe filled with 2mL 150mg/mL omalizumab formulation, one syringe filled with 1mL 150mg/mL omalizumab formulation and one syringe filled with 0.5mL 150mg/mL omalizumab formulation.

60. 150mg/mL omalizumab formulation for use in a method of treatment of one or more of: allergic asthma; food allergy; chronic rhinosinusitis with nasal polyps; chronic spontaneous urticaria; nasal polyps; moderate to severe persistent asthma in patients with a positive skin test or *in vitro* reactivity to a perennial aeroallergen and symptoms that are inadequately controlled with inhaled corticosteroids, wherein 600mg 150mg/mL omalizumab formulation is administered subcutaneously by one syringe filled with 2mL 150mg/mL omalizumab formulation and one syringe filled with 2mL 150mg/mL omalizumab formulation.

61. 150mg/mL omalizumab formulation for use according to any one of claims 55 to 60, wherein a syringe filled with 2mL 150mg/mL omalizumab formulation is a syringe according to any one of claims 50 to 54 when X is 2.

62. 150mg/mL omalizumab formulation for use according to any one of claims 57 and 59, wherein the one syringe filled with 0.5mL 150mg/mL omalizumab formulation is a syringe according to any one of claims 50 and 51 when X is 0.5.

63. 150mg/mL omalizumab formulation for use according to any one of claims 58 and 59, wherein the one syringe filled with 1mL 150mg/mL omalizumab formulation is a syringe according to any one of claims 50 and 51 when X is 1.
